# EUROPEAN PATENT APPLICATION

(11) **EP 1 325 959 A1**
(43) Date of publication of application: **09.07.2003**
(21) Application number: 01945681.3
(22) Date of filing: 28.06.2001
(51) Int. Cl.: C12N 15/80, C12N 15/11, C12N 1/19, C12N 9/04, C12N 9/06, C12N 9/14, C12N 9/16, C12N 9/36

(54) **TRANSFORMATION SYSTEM OF FUNGUS BELONGING TO THE GENUS MONASCUS**

(30) Priority: 28.06.2000 JP 2000195142
(71) Applicant: KYOWA HAKKO KOGYO CO., LTD., Chiyoda-ku, Tokyo 100-8185 (JP); Shin Nihon Chemical Co., Ltd., Aichi 446-0063 (JP)
(72) Inventor: ANAZAWA, Hideharu, KYOWA HAKKO KOGYO CO., LTD, Chiyoda-ku, Tokyo 100-8185 (JP); KATO, Yoko, KYOWA HAKKO KOGYOCO., LTD., Machida-shi, Tokyo 194-8533 (JP); NAGASHIMA, Tadashi, Anjyo-shi, Aichi 444-1154 (JP); MIYOSHI, Kenzo, Shin Nihon Chemical Co., Ltd., Anjyo-shi, Aichi 446-0063 (JP); YAMAZUMI, Takao, Shin Nihon Chemical Co., Ltd., Anjyo-shi, Aichi 446-0063 (JP)
(74) Representative: VOSSIUS & PARTNER
(86) International application number: JP0105596
(87) International publication number: WO02000898

(57) **Abstract**

Provided are a novel DNA which encodes a selection marker appropriate for the transformation system of filamentous fungi of the genus *Monascus* and for expression of a recombinant DNA, and a novel DNA which functions as a promoter and a terminator. There is also provided a novel transformation system of filamentous fungi of the genus *Monascus*, in which filamentous fungi of the genus *Monascus* is transformed with a vector comprising the DNA encoding the above selection marker and the DNA encoding a desired protein in addition to promoter and terminator sequences to obtain a transformant; culturing the transformant; and isolating at high levels the expression product of the recombinant DNA.

## Description

### TECHNICAL FIELD

The present invention relates to a reproducible transformation system in filamentous fungi belonging to the genus *Monascus* of the class Hemiascomycetes. More specifically, the present invention relates to transformation of filamentous fungi of the genus *Monascus* with recombinant DNA, and expression of recombinant DNA by the resultant, novel transformant. In addition, the present invention relates to a novel DNA sequence which can be used as a selection marker for transformation, and novel DNA sequences which can be used as a promoter and a terminator for expressing recombinant DNA.

As DNA which encodes the novel selection marker disclosed in the present invention, nitrate reductase gene and acetamidase gene which are derived from *Monascus purpureus* are exemplified. In addition, as the novel DNA sequences which can be used as a promoter and as a terminator for expressing recombinant DNA, glyceraldehyde-3-phosphate dehydrogenase gene, alcohol dehydrogenase gene and acid phosphatase gene which are derived from *Monascus purpureus* are exemplified.

### BACKGROUND ART

Development of genetic recombination techniques has realized mass production of useful protein by microorganisms. A convenient host for this purpose is a prokaryotic system, such as *Escherichia coli* or *Bacillus subtilis*. In particular, *Escherichia coli* is the most frequently used as a host. However, the *Eschericia coli* system cannot fully satisfy various needs, because most proteins produced by *E. coli* are insoluble, and because the *E. coli* system undergoes such constraints that no sugar chain can be added to the proteins when they are secreted, and the like.

In contrast, production of useful proteins in a eukaryotic microorganism system, such as yeast and filamentous fungi, is useful for the following reasons. Yeast of the genus *Saccharomyces* and filamentous fungi, such as *Aspergillus,* have been utilized for a long time for alcoholic beverages production and fermented food production. In addition to use as very safe hosts, they have been commercially used as enzyme-producing and secreting fungi. Therefore, their ability to produce and secrete a useful protein in a soluble form is high. Further, sugar chains can be added to the protein when it is produced by secretion, because they are eukaryotic organisms.

Among filamentous fungi, *Aspergillus nidulans* has been researched best and genetic findings of *Aspergillus nidulans* have been accumulated. Specifically, various selection markers and regulator genes for transformation have been reported for *Aspergillus nidulans*, and a transformation system using *Aspergillus nidulans* host has been developed. Selection marker genes that have been used for transformation of *Aspergillus nidulans* are orotidine-5'-phosphate decarboxylase gene *Pyr4* [Biochem. Biophys. Res. Commun., 112, 284-289 (1983)] of *Neurosora crassa*, acetamidase gene *amdS* [Gene, 26, 205-221 (1983)], and ornithine carbamyl transferase gene *argB* [Enzyme Microb. Technol., 6, 386-389 (1984)] of *Aspergillus nidulans* and the like.

In addition, transformation of *Aspergillus niger* [EMBO J., 4, 475-479 (1985)] using *amdS* gene of *Aspergillus nidulans*, and transformation of *Aspergillus oryzae* [J. Ferment. Bioeng., 74, 389-391 (1992)] using *amdS* gene of *Aspergillus oryzae* have been reported. Further, transformation of *Aspergillus niger* [Gene, 37, 207-214 (1985)] and transformation of *Aspergillus oryzae* [Agric. Biol. Chem., 51, 2549-2555 (1987)] using *argB* gene of *Aspergillus nidulans* have been reported. Furthermore, transformation of *Aspergillus niger* using nitrate reductase gene *niaD* of *Aspergillus niger* [Gene, 78, 157-166 (1989)], and transformation of *Aspergillus oryzae* [Mol. Gen. Genet., 218, 99-104 (1989)] using *niaD* gene of *Aspergillus oryzae* have been reported. Moreover, transformation of *Aspergillus niger* [Mol. Gen. Genet., 206, 71-75 (1987), Curr. Genet., 11, 499-503 (1987)] using orotidine-5'-phosphate decarboxylase gene *pyrG* of *Aspergillus niger* has been reported.

On the other hand, the filamentous fungi belonging to the genus *Monascus* taxonomically belong to the family Hemiascomycetes. About 20 species or 70 different strains of the filamentous fungi belonging to the genus *Monascus* have been isolated and identified to date.

Filamentous fungi belonging to the genus *Monascus* have been used from ancient times in China, Taiwan and the like, mainly as koji for brewing and as fungi for producing a colorant or a flavoring agent. Specifically, they are used in red liquor, Chinese wine, red Chinese style cheese, pickles of flesh and of vegetables, and sautéed food. In addition, filamentous fungi contribute to the gastronomic culture of Okinawa in Japan, which is represented by tofu carbuncle, steamed rice with red beans, red rice-cake sweets and the like.

The filamentous fungus belonging to the genus *Monascus* produces a significant amount of red pigment, and its koji presents dark red so it is generally called red koji mold. The red pigments produced by red koji have been used as a colorant preferentially, because a red food material is scarce and the pigments are safe as they are natural pigments. Red pigments consisting of rubropunctatin, monascorubrin and the like are industrially produced as natural coloring agents which are extracted and isolated by organic solvents. Since synthetic red pigments can no longer be used because of concerns over their possible carcinogenesis, consumption of the natural red pigments is increasing. Red pigments produced by red koji have been reported to have an antiseptic effect and anti-cancerous effect in addition to their application as a coloring agent. Therefore, improvement of their productivity and their application as a pharmaceutical preparation are expected.

Further, red koji has an alcohol production ability higher than that of other koji and has been also used as a flavoring agent which adds a sweet aroma. Further, using its high alcohol production ability, application of red koji to alcohol fermentation using biomass has been attempted. In contrast to the limited assimilability of yeast, *Aspergillus* can be expected to have assimilability for a wider variety of substances. Thus, it is considered that *Aspergillus* has a high utility value. Actually, production of red alcoholic beverages has been attempted by combining red pigment production with alcohol fermentation, of red koji.

Furthermore, red koji is known to have a variety of functionalities that are not seen in other koji, such that it produces physiologically active substances comprising manifold metabolites, for example, various organic acids and peptides, in addition to enzymes, such as protease and amylase. A variety of industrial applications of red koji have been attempted.

Red koji is the sole type of koji used also as a Chinese herbal medicine. Specifically, red koji has been used widely and regularly since ancient times as a Chinese herbal medicine which helps digestion and improves blood circulation. Monacolin K having a strong hypotensive effect and inhibitory effect on cholesterol biosynthesis has been reported as a physiologically active substance produced by red koji [J. Antibiot., 32, 852-854, (1979), J. Antibiot., 33, 334-336, (1980), Japanese Patent Examined Publication No. 60-44914]. In addition to Monacolin K, the presence of a substance having a hypotensive effect has been reported [Food and Development, 28, 47-50 (1993)]. Furthermore, red koji has also been reported to produce a substance, such as Monascidin A, which has antibacterial activity against those of the genera *Bacillus, Streptococcus* and *Pseudomonas* [Fermentation and Industry, 43, 544-552 (1985)].

As described above, similarly to yellow koji mold, *Aspergillus oryzae*, filamentous fungus belonging to the genus *Monascus* (red koji mold) is recognized as a very safe filamentous fungus that has long been consumed as food, and as a fungus producing industrially and pharmaceutically useful substances [Fermentation and Industry, 43, 544-552 (1985); Science and Technology for Miso, 45, 322-328 (1997)].

The above knowledge also suggests that establishing both a transformation system of filamentous fungi belonging to the genus *Monascus* and a recombinant expression system using filamentous fungi of the genus *Monascus* is industrially very useful.

However, there is no known transformation system of the filamentous fungi belonging to the genus *Monascus*.

### DISCLOSURE OF THE INVENTION

An object of the present invention is to establish a transformation system of the filamentous fungus belonging to the genus *Monascus*, red koji mold, which has long been consumed as food similarly to yellow koji mold and which is thus very safe for the human, and provide a method for producing a protein using transformants established by the transformation system.

We have thoroughly studied the host-vector system of filamentous fungi belonging to the genus *Monascus*. Thus, we have completed the present invention by establishing the transformation system of this strain.

The present invention encompasses the following inventions.
(1) In a method for transforming filamentous fungi, the improvement comprising using a filamentous fungus belonging to the genus *Monascus* as a host.
(2) The method according to (1), wherein the filamentous fungus belonging to the genus *Monascus* is *Monascus purpureus.*
(3) The method according to (1) or (2), which comprises introducing into a host a recombinant DNA that is obtainable by incorporating into one vector a DNA encoding a marker for selecting a transformant and a DNA encoding a desired protein.
(4) The method according to (1) or (2), which comprises introducing into a host two types of recombinant DNAs, one of which is obtainable by incorporating a DNA encoding a marker for selecting a transformant into a vector, and the other of which is obtained by incorporating a DNA encoding a desired protein into a vector.
(5) The method according to (3) or (4), wherein the DNA encoding a marker for selecting a transformant is selected from the group consisting of DNA encoding the nitrate reductase of filamentous fungi, DNA encoding acetamidase of filamentous fungi, DNA encoding ornithine carbamyl transferase of filamentous fungi and DNA encoding orotidine -5'- phosphate decarboxylase of filamentous fungi.
(6) The method according to (3) or (4), wherein the DNA encoding a marker for selecting a transformant is a DNA comprising a nucleotide sequence represented by any one of SEQ ID NOS: 1, 2, 3, 5 and 7.
(7) The method according to (3) or (4), wherein the DNA encoding a marker for selecting a transformant is a DNA hybridizing to the DNA comprising the nucleotide sequence represented by SEQ ID NO: 1 or 3 under stringent conditions, and encoding a protein having activity substantially equivalent to nitrate reductase; or a DNA hybridizing to the DNA comprising the nucleotide sequence represented by SEQ ID NO: 5 or 7 under stringent conditions, and encoding a protein having activity substantially equivalent to acetamidase.
(8) The method according to (3) or (4), wherein the recombinant DNA has a promoter which is located upstream of the DNA encoding a desired protein and is derived from a gene selected from the group consisting of an alcohol dehydrogenase gene, an acid phosphatase gene, a glyceraldehyde-3-phosphate dehydrogenase gene, a phosphoglycerate kinase gene, a glucoamylase gene, a phytase gene, a protease gene and a cellulase gene.
(9) The method according to (3) or (4), wherein the recombinant DNA has a terminator which is located downstream of the DNA encoding a desired protein and is derived from a gene selected from the group consisting of an alcohol dehydrogenase gene, an acid phosphatase gene, a glyceraldehyde-3-phosphate dehydrogenase gene, a phosphoglycerate kinase gene, a glucoamylase gene, a phytase gene, a protease gene and a cellulase gene.
(10) The method according to (8) or (9), wherein the alcohol dehydrogenase gene, the acid phosphatase gene or the glyceraldehyde-3-phosphate dehydrogenase gene is derived from the filamentous fungi belonging to the genus *Monascus*.
(11) The method according to (10), wherein the alcohol dehydrogenase gene comprises the nucleotide sequence represented by SEQ ID NO: 9, the acid phosphatase gene comprises the nucleotide sequence represented by SEQ ID NO: 13, and the glyceraldehyde-3-phosphate dehydrogenase gene comprises the nucleotide sequence represented by SEQ ID NO: 17 or 18.
(12) The method according to (8), wherein the promoter is capable of enhancing gene expression in the presence of lower alcohol.
(13) The method according to (12), wherein the lower alcohol is ethanol or methanol.
(14) The method according to (8), wherein the promoter comprises a DNA having a nucleotide sequence of 50 or more consecutive nucleotides between positions 1 and 615 of the nucleotide sequence of SEQ ID NO: 9.
(15) The method according to (8), wherein the promoter comprises a DNA having a nucleotide sequence of 50 or more consecutive nucleotides between positions 1 and 1013 of the nucleotide sequence of SEQ ID NO: 13.
(16) The method according to (8), wherein the promoter comprises a DNA having a nucleotide sequence of 50 or more consecutive nucleotides in the nucleotide sequence of SEQ ID NO: 17.
(17) The method according to (8), wherein the promoter comprises a DNA having a nucleotide sequence of 50 or more consecutive nucleotides between positions 1 and 1078 of the nucleotide sequence of SEQ ID NO: 18.
(18) The method according to (9), wherein the terminator comprises a DNA having a nucleotide sequence of 50 or more consecutive nucleotides between positions 1950 and 4142 of the nucleotide sequence of SEQ ID NO: 9.
(19) The method according to (9), wherein the terminator comprises a DNA having a nucleotide sequence of 50 or more consecutive nucleotides between positions 2633 and 3831 of the nucleotide sequence of SEQ ID NO: 13.
(20) The method according to (9), wherein the terminator comprises a DNA having a nucleotide sequence of 50 or more consecutive nucleotides between positions 2347 and 2456 of the nucleotide sequence of SEQ ID NO: 18.
(21) The method according to (3) or (4), wherein the DNA encoding a desired protein comprises the nucleotide sequence represented by any one of SEQ ID NOS: 1, 3, 5, 7, 9, 11, 13 and 15.
(22) The method according to (3) or (4), wherein the desired protein is selected from the group consisting of nitrate reductase, acetamidase, alcohol dehydrogenase II and acid phosphatase that are derived from filamentous fungi belonging to the genus *Monascus*, and phytase that is derived from *Aspergillus niger.*
(23) The method according to (3) or (4), wherein the desired protein comprises an amino acid sequence represented by any one of SEQ ID NOS: 4, 8, 12 and 16.
(24) The method according to (3) or (4), wherein the DNA encoding a desired protein is a DNA encoding a protein comprising a desired protein and a signal peptide of the secretory protein of a filamentous fungus which peptide has been added to the N-terminus of the desired protein.
(25) The method according to (24), wherein the signal peptide of the secretory protein of the filamentous fungus is a signal peptide of phytase of *Aspergillus niger,* acid phosphatase of *Monascus purpureus,* or Taka-amylase A of *Aspergillus oryzae*.
(26) A transformant of a filamentous fungus belonging to the genus *Monascus*, which is obtainable by any one of the methods according to (1) to (25).
(27) A method of producing a protein, which comprises culturing the transformant according to (26), until a desired protein is produced and accumulated in a culture, and recovering the protein therefrom.
(28) A DNA, which comprises a nucleotide sequence of 50 or more consecutive nucleotides between positions 1 to 615 of the nucleotide sequence of SEQ ID NO: 9.
(29) A DNA, which comprises a nucleotide sequence of 50 or more consecutive nucleotides between positions 1950 and 4142 of the nucleotide sequence of SEQ ID NO: 9.
(30) The DNA according to (28), which is capable of enhancing gene expression in the presence of lower alcohol.
(31) The DNA according to (30), wherein the lower alcohol is ethanol or methanol.
(32) A DNA, which comprises a nucleotide sequence of 50 or more consecutive nucleotides between positions 1 and 1013 of the nucleotide sequence of SEQ ID NO: 13.
(33) A DNA, which comprises a nucleotide sequence of 50 or more consecutive nucleotides between positions 2633 and 3831 of the nucleotide sequence of SEQ ID NO: 13.
(34) A DNA, which comprises the nucleotide sequence of SEQ ID NO: 17.
(35) A recombinant DNA, which comprises as a selection marker a DNA comprising the nucleotide sequence represented by SEQ ID NO: 1 or 3, or a DNA which hybridizes under stringent conditions to a DNA comprising the nucleotide sequence represented by SEQ ID NO: 1 or 3 and encodes a protein having activity substantially equivalent to that of nitrate reductase.
(36) A recombinant DNA, which comprises as a selection marker a DNA comprising the nucleotide sequence represented by SEQ ID NO: 5 or 7, or a DNA which hybridizes under stringent conditions to a DNA comprising the nucleotide sequence represented by SEQ ID NO: 5 or 7 and encodes a protein having activity substantially equivalent to that of acetamidase.
(37) A recombinant DNA, which comprises as a promoter a DNA according to (28), (32) or (34).
(38) A recombinant DNA, which comprises as a terminator a DNA according to (29) or (33).
(39) A protein, which comprises an amino acid sequence represented by any one of SEQ ID NOS: 4, 8, 12 and 16.
(40) A protein, which comprises an amino acid sequence wherein one or more amino acid residues are deleted, substituted and/or added in the amino acid sequence represented by any one of SEQ ID NOS: 4, 8, 12 and 16, and has activity equivalent to that of the protein comprising the amino acid sequence represented by any one of SEQ ID NOS: 4, 8, 12 and 16.
(41) A DNA, which encodes the protein according to (39) or (40).
(42) The DNA according to (41), which comprises a nucleotide sequence represented by any one of SEQ ID NOS: 1, 3, 5, 7, 9, 11, 13 and 15.
(43) A DNA, which hybridizes to the DNA according to (42) under stringent conditions, and encodes a protein having activity substantially equivalent to that of a protein comprising an amino acid sequence represented by any one of SEQ ID NOS: 4, 8, 12 and 16.
(44) An oligonucleotide, which comprises a nucleotide sequence that is identical to that of 15 to 60 consecutive nucleotides in a nucleotide sequence represented by any one of SEQ ID NOS: 1, 3, 5, 7, 9, 11, 13 and 15, or comprises a nucleotide sequence that is complementary to that of the oligonucleotide.

The present specification includes the contents as disclosed in the specification and/or the drawings of Japanese Patent Application No. 2000-195142, which is the priority document of the present application.

The present invention is hereinafter explained in detail.

### 1. Transformation method using a filamentous fungus belonging to the genus Monascus as a host

The transformation method of the present invention is characterized in that filamentous fungus belonging to the genus *Monascus* is used as a host.

The method of transforming filamentous fungi belonging to the genus *Monascus* has been completed by applying a transformation method for filamentous fungi belonging to the genus *Aspergillus* using the standard known host-vector system [Biochem. Biophys. Res. Commun., 112, 284-289 (1983), Gene, 26, 205-221 (1983), Enzyme Microb. Technol., 6, 386-389 (1984), EMBO J., 4, 475-479 (1985), J. Ferment. Bioeng., 74, 389-391 (1992), Gene, 37, 207-214 (1985), Agric. Biol. Chem., 51, 2549-2555 (1987), Gene, 78, 157-166 (1989), Mol. Gen. Genet., 218, 99-104 (1989), Mol. Gen. Genet., 206, 71-75 (1987), Curr. Genet., 11, 499-503 (1987)].

Key techniques in the transformation method using a host-vector system of filamentous fungi belonging to the genus *Monascus* are hereinafter described in detail. The technique for which explanation is omitted can be implemented according to the above known methods. In addition, methods that can be used as basic genetic engineering methods are described in Molecular Cloning, A Laboratory Manual, Second Edition, Cold Spring Harbor Laboratory (1989) (hereinafter referred to as "Molecular Cloning 2nd Edition"), Current Protocols in Molecular Biology, John Wiley & Sons (1987-1997) (hereinafter referred to as "Current Protocols in Molecular Biology"), DNA Cloning 1: Core Techniques, A Practical Approach, Second Edition, Oxford University Press (1995), and the like. Further, genetic engineering methods that can be used herein and are specifically used for filamentous fungi are described in Applied Molecular Genetics of Filamentous Fungi [Applied Molecular Genetics of Filamentous Fungi, R. Kinghorn and G. Turner ed., p1-p27, Blckie Academic & Professional (1992)] and the like. The term "gene of protein X" described below in the specification includes a region encoding the protein X in genomic DNA, an untranslated region when transcribed to mRNA, an intron, and a regulatory region, such as a promoter, a terminator or an enhancer, which is normally present adjacent to upstream or downstream of the region encoding protein X in the genomic DNA, and which regulates expression of the region.

### (1) Host

Filamentous fungi belonging to the genus *Monascus* used as hosts include all filamentous fungi identified as those belonging to the genus *Monascus*. Specific examples of such filamentous fungi include *Monascus purpureus, Monascus pilosus*, and *Monascus ruber. Monasucus purpureus* includes those strains previously known as *Monascus anka, Monascus major, Monascus albidus, Monascus araneosus*, and *Monascus rubiginosus*. In particular, *Monascus purpureus* is the most often used in industrial production, and is highly safe.

### (2) Vector

As a vector, any known vector that can be used in the host-vector system of filamentous fungi can be used. Examples of the vector include pUC18 [Gene, 33, 103-119 (1985), TAKARA SHUZO CO., LTD.], pUC118 [Methods Enzymol. 153, 3-11 (1987), TAKARA SHUZO CO., LTD.], pBluescript II SK (+) and pBluescript II SK (-) [for both, Nucleic Acids Res., 17, 9494 (1989), STRATAGENE], and pBluescript SK (+) (STRATAGENE).

### (3) A selection marker for selecting transformants

A preferred vector comprises DNA which encodes a selection marker for selecting a transformant. Since filamentous fungi belonging to the genus *Monascus* are highly resistant to known drugs, examples of a selection marker that can be preferably used herein include proteins other than those encoded by known drug-resistant genes, such as enzymes that are involved in assimilability of a carbon source or a nitrogen source, and are of metabolic system for various compounds acting as a carbon source or a nitrogen source; and enzymes that are involved in the requirement of nucleic acids and amino acids, and are of biosynthetic system of nucleic acids and amino acids.

Examples of a preferred selection marker include nitrate reductase, acetamidase, ornithine carbamyl transferase, and orotidine -5'- phosphate decarboxylase. Examples of DNAs encoding these selection markers include a nitrate reductase gene *niaD*, an acetamidase gene *amdS*, an ornithine carbamyl transferase gene *argB*, and an orotidine-5'-phosphate decarboxylase gene *pyrG* or *pyr4*. cDNAs derived from these genes can also be mentioned as examples of the DNAs encoding these selection markers.

The DNA encoding the above selection marker may be derived from either a host or an organism other than the host, so far as it can function as a selection marker in a host. Preferably, a gene derived from a host is used, which increases the chance of homologous incorporation of the introduced vector into the chromosome of the cell. Thus, reliable expression of a marker can be expected.

A gene as the selection marker derived from a filamentous fungus which is used as a host and belongs to the genus *Monascus* can be obtained from chromosomal DNA library by constructing a chromosomal DNA library of the filamentous fungus used as a host and performing plaque hybridization by use of genes derived from other filamentous fungi and having known nucleotide sequences as probes. Chromosomal DNA libraries of filamentous fungi belonging to the genus *Monascus* can be prepared as follows. First, chromosomal DNA of a filamentous fungus belonging to the genus *Monascus* is isolated, the DNA is cleaved into 5 to 20 kb in length using an appropriate restriction enzyme that enables insertion into the cloning site of a vector, such as *EcoR* I or *Bam*H I, and then the vector is inserted into an arm cleaved at the cloning site. As for the chromosomal DNA, the cells of the filamentous fungus of the genus *Monascus* are frozen by liquid nitrogen, homogenized rapidly with a pestle, mixed with a TE buffer solution [10 mmol/l Tris-HCl (pH 8.0), 1 mmol/l EDTA] for suspension, mixed with an equivalent amount of lytic solution [2% SDS, 0.1 mol/l NaCl, 10 mmol/l EDTA, 50 mmol/l Tris-HCl (pH 7.0)], and then kept warm at 37°C for 30 min for lysis. The resulting lysate is centrifuged (12,000xG) to collect a supernatant. Purification can be performed by subjecting the supernatant to, in sequence, phenol treatment, ethanol precipitation, RNase treatment, phenol treatment (twice), chloroform treatment and then ethanol precipitation. As a vector, lambda EMBL 3, lambda EMBL 4 [for both, J. Mol. Biol., 170, 827-842 (1983), STRATAGENE] and lambda DASH II (STRATAGENE) are used. After insertion of a chromosomal DNA fragment into a vector, in vitro packaging is performed using a kit, such as Gigapack Gold or Gigapack Gold III (both manufactured by STRATAGENE). Then, the packaging solution is infected with an *Escherichia coli* host which is appropriate for each vector. For example, when a vector is lambda EMBL 3, lambda EMBL 4 or lambda DASH II, the solution is infected with *E. coli* P 2392, *E. coli* XL1-Blue MRA or *E. coli* XL1-Blue MRA (P 2) (all. manufactured by STRATAGENE) to amplify the λ phage in the library, thereby preparing chromosomal DNA libraries. According to the instructions attached to the products of STRATAGENE, insertion of chromosomes into vectors, in vitro packaging, and infection and proliferation of lambda phages can be performed. Fragments of 0.5 kb or more of the genomic DNA or cDNA of the gene of other filamentous fungi are labeled with horseradish peroxidase or radioactive isotope ³²P and then used as probes. These fragments can be isolated by digestion with restriction enzymes from the genomic DNA clones or cDNA clones of the gene, or can be isolated after amplification by PCR using primers designed based on the nucleotide sequence information of the gene, and using chromosomal DNA of the filamentous fungus as a template. When horseradish peroxidase-labeled probes are used, labeling of the probes, hybridization, and detection of hybridized spots can be performed according to ECL Direct Nucleic Acid Labeling and Detection System (Amersham Pharmachia Biotech) and its instructions. Labeling with ³²P, hybridization and detection of hybridized spots can be performed according to Molecular Cloning 2nd Edition.

The nitrate reductase gene derived from a filamentous fungus belonging to the genus *Monascus* can be obtained from a chromosomal DNA library of the filamentous fungus of the genus *Monascus*, using *niaD* gene of *Aspergillus oryzae* [Biosci. Biotech. Biochem., 59, 1795-1797 (1995): GenBank Accession No. D49701] as a probe. A specific example of the thus obtained nitrate reductase gene derived from a filamentous fungus belonging to the genus *Monascus*, is a nitrate reductase gene derived from *Monascus purpureus* having the nucleotide sequence of SEQ ID NO: 1. In addition, DNA having the nucleotide sequence of SEQ ID NO: 3 also encodes nitrate reductase of *Monascus purpureus,* and thus can be used as a DNA encoding a selection marker. Further, *niaD* gene of *Aspergillus oryzae* itself functions as a nitrate reductase gene in filamentous fungi belonging to the genus *Monascus*, and thus can be used as a DNA encoding a selection marker.

The acetamidase gene derived from a filamentous fungus belonging to the genus *Monascus* can be obtained from the chromosomal DNA library of the filamentous fungus belonging to the genus *Monascus* using *amdS* gene of *Aspergillus oryzae* [Gene, 108, 91-98 (1991): GenBank Accession No. D10492] as a probe, or using *amdS* gene of *Aspergillus nidulans* [Gene, 26, 205-221 (1983): GenBank Accession No. M16371] as a probe. A specific example of the thus obtained acetamidase gene is acetamidase gene derived from *Monascus purpureus* having the nucleotide sequence of SEQ ID NO: 5. In addition, DNA having a nucleotide sequence represented by SEQ ID NO: 7 also encodes the acetamidase of *Monascus purpureus*, and thus can be used as a DNA encoding a selection marker.

The *amdS* genes derived from *Aspergillus nidulans* and *Aspergillus oryzae* cannot be directly used as DNAs encoding selection markers, because the genes cannot function in transformants of filamentous fungi belonging to the genus *Monascus*. To use the *amdS* gene as a selection marker gene, the gene is modified to have a sequence appropriate for filamentous fungi of the genus *Monascus* based on the nucleotide sequence information represented by SEQ ID NO: 5 or 7.

The ornithine carbamyl transferase gene derived from filamentous fungi belonging to the genus *Monascus* can be obtained from the filamentous fungi belonging to the genus *Monascus* using *argB* gene [Enzyme Microb. Technol., 6, 386-389 (1984)] of *Aspergillus nidulans* as a probe.

The orotidine-5'-phosphate decarboxylase gene derived from a filamentous fungus belonging to the genus *Monascus* can be obtained from a chromosomal DNA library of the filamentous fungus belonging to the genus *Monascus* using *pyrG* gene [Curr. Genet., 16, 159-163 (1989)] of *Aspergillus niger* or *pyr4* gene [Biochem. Biophys. Res. Commun., 112, 284-289 (1983)] of *Neurospora crassa* as a probe.

In addition, DNA which hybridizes to the whole or a part of the obtained DNA encoding a selection marker under stringent conditions can also be used as a DNA encoding a selection marker.

The DNA which hybridizes under stringent conditions means a DNA which can be obtained by colony hybridization, plaque hybridization, Southern blot hybridization or the like using as a probe the above-obtained DNA encoding a marker. Specifically, such a DNA can be identified by performing hybridization in the presence of 0.7 to 1.0 mol/l sodium chloride at 65°C using a filter having DNA derived from a colony or a plaque immobilized thereto; and washing the filter at a temperature condition of 65°C using SSC solution at 0.1 to 2 fold concentration (SSC solution at 1 fold concentration comprises 150 mmol/l sodium chloride and 15 mmol/l sodium citrate). Hybridization can be performed according to the methods described in Molecular Cloning 2nd Edition, Current Protocols in Molecular Biology, DNA Cloning 1: Core Techniques, A Practical Approach, Second Edition, Oxford University (1995), and the like. Hereinafter, the stringent conditions in the present specification denote the above conditions.

The DNA capable of hybridizing has, for example, at least 60% or more homology, preferably 80% or more homology, and further preferably 95% or more homology with the nucleotide sequence of the above-obtained DNA encoding the selection marker. A numerical value of homology described in the present specification may be the value calculated using a homology search program known by a person skilled in the art, such as BLAST [J. Mol. Biol., 215, 403-410 (1990)] or FASTA [Methods. Enzymol., 183, 63-98 (1990)], unless specifically stated. A preferred numerical value is calculated by BLAST using the default parameter (initial setting) or by FASTA using the default parameter (initial setting).

Whether the DNA capable of hybridizing under the above stringent conditions encodes a protein having activity substantially equivalent to that of nitrate reductase can be confirmed by introducing the DNA to express into a host of a mutant strain that is unable to use nitrate as nitrogen source (as described in (4)). The introduction confers assimilability of nitrate to the host, and the host becomes capable of assimilating nitrate. Thus, it is confirmed that the DNA encodes the protein when the host can grow in a minimal medium containing nitrate as a sole nitrogen source. Alternatively, it is confirmed that the DNA encodes the protein by using the DNA according to the methods described in 2 or 3 to allow the protein encoded by the DNA to be expressed in hosts filamentous fungi belonging to the genus *Monascus* or other organisms, or using *in vitro* translation; and measuring the activity of the protein (nitrate reductase) according to the method described in literature [Biochim. Biophys. Acta, 113, 51-56 (1966)].

Whether the DNA capable of hybridizing under the above stringent conditions encodes a protein having activity substantially equivalent to that of acetamidase can be confirmed by introducing the DNA into a host for expression. Since the capability of the host to assimilate acetamide is improved after introduction, it is confirmed that the DNA encodes the protein when the host can grow well in a minimal medium containing acetamide as a sole nitrogen or carbon source. Alternatively, it is confirmed that the DNA encodes the protein by using the DNA according to the methods described in 2 or 3 to allow the protein encoded by the DNA to be expressed in hosts filamentous fungi belonging to the genus *Monascus* or other organisms, or using *in vitro* translation; and measuring the activity of the protein (acetamidase) according to the method described in literature [J. Bacteriol., 111, 717-722 (1972)].

### (4) Host for selecting transformants

To screen for transformants, DNA encoding a selection marker is preferably introduced into a host which lacks the marker function or has low marker function. Examples of the host include filamentous fungi belonging to the genus *Monascus* having properties equivalent to those of hosts appropriate for each selection marker used in the known methods of transformation in the filamentous fungi belonging to the above genus *Aspergillus.*

When nitrate reductase is used as a selection marker, a preferred host is of a mutant strain which is unable to use nitrate as a nitrogen source because of mutation in nitrate reductase gene. Introduction of the nitrate reductase gene into the mutant strain can give assimilability of nitrate, so that transformants can be selected.

Mutant strains which are unable to use nitrate as a nitrogen source because of mutation in nitrate reductase gene can be obtained by inoculating and culturing the conidia of a filamentous fungus belonging to the genus *Monasucus* in a minimal medium containing a mutagen, obtaining strains which grow after inoculation, and examining these strains for the assimilability of a nitrogen source. Mutagens may be any of chemical mutagens, radioactive isotopes and the like known by a person skilled in the art, and are not specifically limited. Preferably, a chemical mutagen, more preferably, chlorate is used. The term "minimal medium" means a medium comprising minimum components essential for the growth of cells of the wild type when microorganims are cultured. For example, a minimal medium for culturing filamentous fungi comprises a carbon source, a nitrogen source and inorganic salts. Such a minimal medium containing a mutagen can be appropriately prepared by a person skilled in the art. An example of a minimal medium is a plate medium (pH 5.5) consisting of 3% sucrose, 10 mmol/l glutamic acid, 0.2% KH₂PO₄, 0.05% MgSO₄·7H₂O, 0.05% KCl and 470 mmol/l KClO₃. As filamentous fungi belonging to the genus *Monascus*, the desired species which should be used as a host can be employed. The temperature for culturing may be any temperature at which filamentous fungi to be used herein can grow, and is not specifically limited. A preferred temperature ranges from 15 to 40°C, and more preferably, is about 30°C. The time for culturing is not specifically limited because it can be appropriately set by a person skilled in the art, and is preferably from 15 to 20 days.

Whether strains having grown as described above can assimilate a nitrogen source can be examined by culturing these strains on minimal media containing various nitrogen sources and determining whether or not they grow on each medium. Nitrate, such as NaNO₃, is used as a nitrogen source in testing areas, and nitrite, ammonium salt, various amino acids and the like are used as nitrogen sources in control areas. An example of a minimal medium containing nitrate as a nitrogen source (testing area) is a basal plate medium (1% glucose, 0.1% KH₂PO₄, 0.05% MgSO₄·7H₂O, 0.05% KCl, 1.5% agar, pH 5.5) supplemented with 10 mmol/l NaNO₃; an example of a control area is a basal plate medium supplemented with 10 mmol/l NaNO₂, (NH₄)₂SO₄, proline, glutamic acid, alanine and the like. The temperature for culturing may be a temperature at which filamentous fungi to be used herein can grow, and is not specifically limited. A preferred temperature ranges from 15 to 40°C, and more preferably, is about 30°C. The time for culturing is not specifically limited because it can be appropriately set by a person skilled in the art, and is preferably about 3 days.

Nitrate reductase has activity to reduce nitrate to nitrite. Therefore, strains which do not grow in a minimal medium (testing area) containing nitrate as a nitrogen source, but grow in a minimal medium (control area) containing sources other than nitrate, such as nitrite, as a nitrogen source are selected as a mutant strain which is unable to use nitrate as a nitrogen source because of mutation in nitrate reductase gene.

When acetamidase is used as a selection marker, a wild type strain can be used as a host without obtaining the above mutant strain, because the filamentous fungi belonging to the genus *Monascus* has low assimilability of acetamide. Transformants can be selected using, as a marker, the host's enhanced assimilability of acetamide resulting from introduction of the acetamidase gene.

When ornithine carbamyl transferase is used as a selection marker, it is preferred to use as a host a mutant strain having ornithine carbamyl transferase gene showing arginine requirement. The strain grows to require no arginine by introducing ornithine carbamyl transferase gene into the mutant strain. Transformants can be selected in such a change from arginine requirement to arginine non-requirement as a marker. A mutant strain having ornithine carbamyl transferase gene can be obtained in the same manner as that for a mutant strain of *Aspergillus niger* having arginine requirement [Gene, 37, 207-214 (1985)]. Specifically, the conidia irradiated with ultraviolet rays to have gene mutation are cultured in a minimal medium containing no arginine (the medium comprises, for example, 1 % glucose, 10 mmol/l urea, 0.1% KH₂PO₄, 0.05% MgSO₄·7H₂O, 0.05% KCI, pH 5.5). Conidia that grow in the medium are removed by means of glass filter filtration. Then, conidia that do not grow are inoculated over a minimal medium supplemented with arginine, so that strains that grow are obtained. By examining whether the obtained strains require arginine and intermediates for arginine biosynthesis system, mutant strains having mutations of ornithine carbamyl transferase gene can be obtained. Specifically, each strain is cultured on a minimal medium (for example, the medium consists of 1% glucose, 10 mmol/l urea, 0.1% KH₂PO₄, 0.05% MgSO₄·7H₂O, 0.05% KCl, 1.5% agar, pH 5.5) and on minimal media supplemented respectively with ornithine, citrulline and arginine. Finally, the strains which do not grow in a minimal medium and in a minimal medium supplemented with ornithine, but grow in a medium supplemented with citrulline, and in a minimal medium supplemented with arginine are selected as mutant strains having mutations of ornithine carbamyl transferase gene.

When orotidine-5'-phosphate decarboxylase is used as a selection marker, it is preferred to use as a host a mutant strain showing uridine requirement. The strain becomes to require no uridine by introducing the orotidine-5'-phosphate decarboxylase gene into the mutant strain showing uridine requirement. Transformants can be selected in such a change from uridine requirement to uridine non-requirement as a marker. A mutant strain showing uridine requirement can be obtained as a strain resistant against 5-fluoro orotic acid in the presence of uridine, in the same manner as employed to obtain a mutant strain of *Aspergillus niger* showing uridine requirement [Mol. Gen. Genet., 206, 71-75 (1987)]. Specifically, the conidia irradiated with ultraviolet rays to have mutation are cultured in a minimal medium (for example, the minimal medium consists of 1% glucose, 10 mmol/l urea, 0.1% KH₂PO₄, 0.05% MgSO₄·7H₂O, 0.05% KCI, 1.5% agar, pH 5.5) containing 10 mmol/L uridine and I mg/ml 5-fluoro orotic acid. Strains that grow are obtained. By examining whether the obtained strains require uridine, mutant strains showing uridine requirement can be obtained. When cultured in a minimal medium and in a minimal medium supplemented with uridine, strains which cannot grow in a minimal medium, but can grow in the minimal medium supplemented with uridine, are selected as mutant strains showing uridine requirement.

A mutant strain can also be obtained by disrupting a target gene of each of the above selection marker genes in the same manner as the method [Gene, 108, 91-98 (1991)] which involves disrupting a target gene of *amdS* gene of *Aspergillus oryzae* and the mutant strain is used as a host. Specifically, a selection marker gene is obtained from a filamentous fungus belonging to the genus *Monascus* used as a host by the method described in (3). Using restriction enzyme sites within a region encoding the selection marker of the gene, a DNA of several 100 bp to several kb is inserted or deleted, thereby disrupting the selection marker gene to cause it to lose its function. Then, the gene is inserted into an appropriate plasmid vector for use in transformation described in (2). Next, a filamentous fungus belonging to the genus *Monascus* for use as a host is transformed using the disrupted selection marker gene by the method described in (6), thereby causing homologous recombination by which a wild type selection marker gene is substituted with the disrupted selection marker gene. After transformation, the protoplast is cultured in a medium in which the gene-disrupted strain can grow, according to each type of disrupted target gene. For example, in the case of nitrate reductase gene and acetamidase, the protoplast is cultured on a minimal medium consisting of 1% glucose, 10 mm urea, 0.1% KH₂PO₄, 0.05% MgSO₄·7H₂O, 0.05% KCl, 1.5% agar, and pH 5.5; in the case of ornithine carbamyl transferase gene, the protoplast is cultured on a minimal medium supplemented with arginine; and in the case of orotidine-5'-phosphate decarboxylase gene, the protoplast is cultured on a minimal medium supplemented with uridine. Strains that grow are cultured on selection media appropriate for each type of disrupted target gene. That is, in the case of nitrate reductase gene, the strain is cultured in a minimal medium having nitrate as a nitrogen source; in the case of acetamidase, the strain is cultured on a minimal medium having acetamidase as a nitrogen source; in the case of ornithine carbamyl transferase gene, the strain is cultured in a minimal medium which lacks arginine; and in the case of orotidine-5'-phosphate decarboxylase gene, the strain is cultured in a minimal medium which lacks uridine. The strain which cannot survive is selected as a strain containing its disrupted (target) selection marker gene for use as a host. When a wild type selection marker gene is substituted with a disrupted selection marker gene by homologous recombination, the restriction enzyme map of a genome gene in the region comprising the selection marker gene becomes different from that of a wild type strain. Therefore, chromosomal DNA is prepared from the selected strain, and Southern blot hybridization is performed using the selection marker gene as a probe, so that disruption of the target gene can be confirmed.

### (5) Expression of DNA encoding selection marker

DNA encoding a selection marker obtained in (3) is subcloned to an appropriate vector of (2), and then introduced into a host for expression of the selection marker. To express the selection marker in the host, a promoter is required to be present upstream of the DNA encoding the selection marker. As the promoter, the selection marker gene's own promoter can be used. Further, substitution of the promoter with a stronger promoter enables the selection marker to function more effectively. A terminator is not always required to be present downstream of the DNA encoding the selection marker. However, the expression efficiency of the selection marker can be enhanced by locating a terminator downstream of the DNA.

Examples of a strong promoter include those derived from genes of filamentous fungi belonging to the genus *Aspergillus* which are known to have a strong promoter, such as alcohol dehydrogenase gene *alc* (including *alcA, alcB, alcC*), acid phosphatase *aph*, glyceraldehyde-3-phosphate dehydrogenase gene *gpd*, phosphoglycerate kinase gene *pgk,* glucoamylase gene *glaA*, phytase gene *phy*, protease *gene pep,* and cellulase gene *cel.*

Furthermore, a preferred promoter is derived from a filamentous fungus belonging to the genus *Monascus* which has been obtained based on such known promoter information. Examples of such promoters include a promoter derived from a glyceraldehyde-3-phosphate dehydrogenase gene *gpdl* [GenBank Accession No. Z68498] of *Monascus purpureus* strain IFO4478; a promoter derived from a glyceraldehyde-3-phosphate dehydrogenase gene of *Monascus purpureus* strain IFO30873 obtained by PCR using a primer based on the sequence of the *gpd*1 gene; a promoter derived from a glyceraldehyde-3-phosphate dehydrogenase gene obtained by standard methods from filamentous fungi belonging to the genus *Monascus* using these glyceraldehyde-3-phosphate dehydrogenase genes as probes: a promoter derived from an alcohol dehydrogenase II gene obtained by standard methods from filamentous fungi belonging to the genus *Monascus* using *alcB* gene [Curr. Genetic., 29, 122-129 (1996): GenBank Accession No. Z48000] of *Aspergillus nidulans* as probes; and a promoter derived from an acid phosphatase gene obtained by standard methods from filamentous fungi belonging to the genus *Monascus* using *aph* gene [Gene, 133, 55-62 (1991): GenBank Accession No. L02420] of *Aspergillus niger* as probes. More specific examples of a promoter include those derived from genes, such as an alcohol dehydrogenase II gene derived from *Monascus purpureus* having a nucleotide sequence represented by SEQ ID NO: 9; an acid phosphatase gene derived from *Monascus purpureus* having a nucleotide sequence represented by SEQ ID NO: 13; and a glyceraldehyde-3-phosphate dehydrogenase gene derived from *Monascus purpureus* having a nucleotide sequence represented by SEQ ID NO: 17 or 18. Any sequence which can substantially function as a promoter can be used. More specific examples of a promoter include DNA having a nucleotide sequence of 50 or more consecutive nucleotides between positions 1 and 615 of the nucleotide sequence of SEQ ID NO: 9; DNA having a nucleotide sequence of 50 or more consecutive nucleotides between positions 1 to 1013 of the nucleotide sequence of SEQ ID NO: 13; DNA having a nucleotide sequence of 50 or more consecutive nucleotides between positions 1 and 1181 of the nucleotide sequence of SEQ ID NO: 17; and DNA having a nucleotide sequence of 50 or more consecutive nucleotides between positions 1 and 1078 of the nucleotide sequence of SEQ ID NO: 18.

Alcohol dehydrogenase gene is a type of housekeeping gene, and is highly expressed constantly. Thus the use of the promoter of this gene enables high expression of a recombinant gene. Further, expression of alcohol dehydrogenase II gene derived from *Monascus purpureus* is induced by addition of ethanol into the medium. Thus, the use of the promoter of the gene enables to induce by ethanol the expression of a recombinant gene.

An upstream sequence of the chromosome of a eukaryotic organism comprises a sequence which is capable of enhancing transcription, in addition to a promoter sequence comprising a transcription initiation site. To expect high expression of a gene, a DNA sequence having activity to enhance transcription is preferably located ahead of a promoter.

Examples of a DNA sequence which is capable of enhancing promoter activity include DNAs derived from genes of filamentous fungi belonging to the genus *Aspergillus* that are known to have strong promoters. Specifically, such DNA may be derived from an alcohol dehydrogenase gene *alc* (including *alcA, alcB, alcC*), acid phosphatase *aph*, glyceraldehyde-3-phosphate dehydrogenase gene *gpd*, phosphoglycerate kinase gene *pgk,* glucoamylase gene *glaA*, phytase gene *phy*, protease gene *pep,* or a cellulase gene *cel.*

Examples of a terminator that is located downstream of DNA encoding a marker include terminators derived from genes of filamentous fungi of the genus *Aspergillus* that are known to have strong promoters. Specifically, such a terminator that is preferably used herein is derived from an alcohol dehydrogenase gene *alc* (including *alcA, alcB, alcC*), acid phosphatase *aph*, glyceraldehyde-3-phosphate dehydrogenase gene *gpd*, phosphoglycerate kinase gene *pgk*, glucoamylase gene *glaA*, phytase gene *phy*, protease gene *pep,* or a cellulase gene *cel*. Furthermore, a preferred terminator is derived from a filamentous fungus belonging to the genus *Monascus* which has been obtained based on such known terminator sequence information. Examples of the terminator include a terminator derived from a glyceraldehyde-3-phosphate dehydrogenase gene *gpdl* of *Monascus purpureus*; a terminator derived from a glyceraldehyde-3-phosphate dehydrogenase gene obtained by standard methods from filamentous fungi belonging to the genus *Monascus* using the gene as a probe; a terminator derived from an alcohol dehydrogenase II gene obtained by standard methods from filamentous fungi belonging to the genus *Monascus* using *alcB* gene [Curr. Genetic., 29, 122-129 (1996)] of *Aspergillus nidulans* as a probe; and a terminator derived from an acid phosphatase gene obtained by standard methods from filamentous fungi belonging to the genus *Monascus* using *aph* gene [Gene, 133, 55-62 (1991)] of *Aspergillus niger* as a probe. Any sequence which can substantially function as a terminator can be used. Specific examples of the terminator include those derived from *Monascus purpureus*-derived genes, such as an alcohol dehydrogenase II gene having the nucleotide sequence represented by SEQ ID NO: 9; an acid phosphatase gene having the nucleotide sequence represented by SEQ ID NO: 13; or a terminator derived from a glyceraldehyde-3-phosphate dehydrogenase gene having the nucleotide sequence represented by SEQ ID NO: 18. More specifically, examples include DNA having a nucleotide sequence of 50 or more consecutive nucleotides between positions 1950 and 4142 of the nucleotide sequence of SEQ ID NO: 9; DNA having a nucleotide sequence of 50 or more consecutive nucleotides between positions 2633 and 3831 of the nucleotide sequence of SEQ ID NO: 13; and DNA having a nucleotide sequence of 50 or more consecutive nucleotides between positions 2347 and 2456 of the nucleotide sequence of SEQ ID NO: 18.

### (6) Transformation method

Transformation of filamentous fungi belonging to the genus *Monascus* using a vector comprising DNA encoding a selection marker can be performed by methods such as electroporation and a protoplast method, which involves introducing DNA into a cell of a filamentous fungus, and preferably, it is performed by the protoplast method. The protoplast method can be performed as follows. First, the cell walls of a filamentous fungus are lysed in an appropriate buffer by allowing an enzyme to react on the cells, preparing protoplasts. Specifically, filamentous fungi belonging to the genus *Monascus* are cultured statically in a nutrient medium (1% dextrin, 1% peptone, 1% yeast extract, 0.05% KH₂PO₄, 0.05% MgSO₄·7H₂O, pH 5.2) at 30°C for 4 to 5 days. Then, the cells are collected by filtration with a glass filter. After washing the cells with distilled water, 10 ml of a protoplast preparation solution [5 mg/ml lysing enzyme, 2.5 mg/ml Sumizyme C, 0.8 mol/l NaCl, 100 mmol/l phosphate buffer, pH 6.0] containing lysing enzymes [Sigma, catalog number L1412] and Sumizyme C (SHIN NIHON CHEMICAL CO., LTD) are added to 0.5g of the cells. The mixture is incubated at 30°C for 3 hours while gently shaking, thereby lysing the cell walls and preparing protoplasts. The prepared protoplasts are filtered with a glass filter to remove mycelial fragments, and then washed. The washing procedure involves adding 10 ml of 0.8 mol/l NaCl solution for re-suspending the protoplasts from which the supernatants have been removed by centrifugation at 700 x G; repeating twice a step of removing supernatants by centrifugation at 700 x G; adding 10 ml of solution 1 [10 mmol/l CaCl₂, 0.8 mol/l NaCl, 10 mmol/l Tris-HCl buffer solution (pH 7.5)] for suspension; and centrifuging the suspension at 700 x G. Next, DNA is introduced into the protoplast prepared using a buffer containing polyethylene glycol (PEG) and CaCl₂. Specifically, the above prepared and washed protoplast is suspended in three-fourth volume of solution 1 such that the protoplast density is 10⁸ cells/ml. Then, one-fourth volume of solution 2 [40% (w/v) PEG 4000, 50 mmol/l CaCl₂, 50 mmol/l Tris-HCI buffer solution (pH 7.5)] is added to the suspension, thereby preparing a protoplast solution. 20 µl of the DNA (0.5 to 0.8µg/µl) of the vector expressing a selection marker described in (5) is added to 200 µl of the protoplast solution, and then ice-cooled for 30 min. Subsequently, 1 ml of solution 2 is added to the solution, and then the solution is allowed to stand at room temperature for 20 min. Next, 10 ml of solution 1 is added to the solution to dilute PEG concentration, the solution is centrifuged at 700 x G, and then the precipitate is collected. The resulting precipitate is suspended in 200 µl of solution 1, and then the suspension is mixed with a 0.5% soft agar medium (a medium having the same composition as the following plate medium for selection, except for the concentration of agar). The mixture is inoculated on the plate medium for selection, and then cultured at 30°C for 10 to 14 days, thereby obtaining colonies of the transformant. The plate medium for selection is a minimal plate medium on which a host cannot grow or barely grow, but on which a host can grow well when the vector DNA is introduced therein and the selection marker is expressed. Examples of such a plate medium for selection that can be used herein include a medium (1% glucose, 0.1% KH₂PO₄, 0.05% MgSO₄·7H₂O, 0.05% KCl, 1.5% agar, pH 5.5) supplemented with 10 mmol/l NaNO₃ as a nitrogen source when a selection marker is nitrate reductase; a medium (1% glucose, 0.1% KH₂PO₄, 0.05% MgSO₄·7H₂O, 0.05% KCl, 1.5% agar, pH 5.5) supplemented with 10 mmol/l acetamide as a nitrogen source when an acetamidase gene is used as a selection marker; and a medium (1 % glucose, 10 mmol/l urea, 0.1% KH₂PO₄, 0.05% MgSO₄·7H₂O, 0.05% KCl, 1.5% agar, pH 5.5) which lacks uridine and arginine when ornithine carbamyl transferase or orotidine-5'-phosphate decarboxylase is used as a selection marker.

### 2. Method for producing a protein by the method for transforming filamentous fungi belonging to the genus Monascus

Any desired protein can be produced using filamentous fungi belonging to the genus *Monascus* by the transformation method of 1. above. When a desired protein is a protein derived from a filamentous fungus belonging to the genus *Monascus*, high expression efficiency can be obtained.

First, DNA encoding a desired protein is obtained. When the DNA cannot be easily obtained, for example, it cannot be supplied through the sample furnishment, an oligonucleotide comprising a 20 to 30 bp sequence at the 5' end and an oligonucleotide comprising a sequence complementary to a 20 to 30 bp sequence at the 3' end of a region encoding a desired protein are synthesized based on the nucleotide sequence information of the DNA using a DNA synthesizer. Then, PCR using as a template cDNA or genomic DNA of an organism expressing the desired protein and using both the oligonucleotides as primers is performed, so that the DNA can be amplified and then isolated. Isolation of cDNA and genomic DNA can be performed by the methods described in Molecular Cloning 2nd Edition or Applied Molecular Genetics of Filamentous Fungi, R. Kinghorn and G. Turner ed., Blckie Acadenic & Professional (1992). PCR can be performed by the methods described in PCR, A Practical Approach, Oxford University Press (1991).

To express DNA encoding a desired protein in filamentous fungi belonging to the genus *Monascus*, it is required to locate the promoter described in 1. (5) upstream of the DNA. This promoter is used for expressing the transformation marker in filamentous fungi belonging to the genus *Monascus*. When a desired protein is a protein of filamentous fungi belonging to the genus *Monascus*, the promoter of the gene of the protein itself can be used. However, when a desired protein is of an organism other than the filamentous fungi of the genus *Monascus*, a promoter derived from a gene of filamentous fungi belonging to the genus *Monascus* is more preferably used than the promoter of the gene of the protein itself. Moreover, examples of a strong promoter of filamentous fungi belonging to the genus *Monascus* that are preferably used include a promoter derived from an alcohol dehydrogenase gene, an acid phosphatase gene, or a glyceraldehyde-3-dehydrogenase gene. In addition, if necessary, expression efficiency of the protein can be enhanced by locating the terminator described in 1. (5) above, which is used for expressing a transformation marker in filamentous fungi belonging to the genus *Monascus*, downstream of the DNA; and locating the DNA sequence described in 1. (5) above, which is capable of enhancing promoter activity and is used for expressing a transformation marker in filamentous fungi belonging to the genus *Monascus*, upstream of the above promoter, respectively. Hereinafter, the DNA, and the promoter, the terminator and the DNA sequence capable of enhancing promoter activity, which are located upstream or downstream of the DNA, are together referred to as a "protein expression unit."

A vector is constructed by inserting the above protein expression unit into an expression vector for a selection marker described in 1. (5). Filamentous fungi belonging to the genus *Monascus* are transformed using the vector above according to the method of 1. (6), so that filamentous fungi belonging to the genus *Monascus* expressing desired proteins can be prepared. Alternatively, separately from the expression vector for the selection marker described in 1. (5), a vector is constructed by inserting the above protein expression unit into a vector described in 1. (2). Filamentous fungi belonging to the genus *Monascus* are co-transformed with both the vector and the expression vector for the selection marker according to the method of 1. (6), so that filamentous fungi belonging to the genus *Monascus* expressing desired proteins can be prepared. Co-transformation is performed according to the method of 1. (6) by simultaneously adding both the vectors (10 µl each) to protoplast, instead of adding 20 µl of the selection marker-expressing vector thereto. The thus prepared transformants of the filamentous fungus belonging to the genus *Monascus* are inoculated on DPC medium (2% dextrin, 0.3% peptone, 0.1% corn steep liquor, 0.05% MgSO₄·7H₂O), YPD medium (2% glucose, 2% polypeptone, 1% yeast extract) and phytase production medium (3% sucrose, 10 mmol/l NaNO₃, 0.05% MgSO₄·7H₂O, 0.05% KCl, 0.1% corn steep liquor), and then statically cultured at 30°C for 10 to 14 days. Thus, the protein is produced and accumulated in the culture comprising the cells and the media. In addition, when an alcohol dehydrogenase II gene of *Monascus purpureus* is used as a promoter, culturing is performed in media containing lower alcohol, preferably ethanol or methanol as a carbon source. Thus, the promoter is induced and the production amount of a desired protein can be increased. The term "lower alcohol" means alcohol having alkyl chain with carbon number of 1 to 7.

When a signal peptide is present at the N-terminus of a desired protein, the signal peptide of the protein is cleaved, the mature protein is secreted extracellularly from a host, i.e., the filamentous fungus belonging to the genus *Monascus*, and then the protein is accumulated in the medium. Further, when no signal peptide is present at the N-terminus of a desired protein, the protein is generally accumulated intracellularly.

In the latter case, a desired protein can be produced by extracellular secretion as follows. First, DNA encoding a desired protein in which the signal peptide of the secretory protein of a filamentous fungus has been added to N-terminus of the desired protein is obtained. An expression vector is constructed using the DNA similarly to the above method. The transformation is performed, and then transformants are cultured. As a result, added signal peptides are cleaved, and then the desired protein is secreted extracellularly. Thus the protein can be produced. It is expected that filamentous fungi belonging to the genus *Monascus* can perform effective secretory production of a desired protein, since they produce and secrete various enzymes. DNA encoding a desired protein in which the signal peptide of the secretory protein of a filamentous fungus has been added to N-terminus of the desired protein is obtained as follows. First, oligonucleotides A to D are synthesized by a DNA synthesizer: oligonucleotide A comprising at its 3' end a 10 to 30 bp sequence that is identical with the sequence of the 5' terminus of a region encoding the signal peptide of a secretory protein gene of a filamentous fungus, or the sequence located upstream of the 5' terminus of the region; oligonucleotide B in which a 15 to 30 bp sequence at the 5' terminus of a region encoding a desired protein has been added to the 3' end of a sequence that is identical with a 15 to 30 bp sequence at the 3' terminus of the region; oligonucleotide C having a sequence complementary to that of oligonucleotide B; and oligonucleotide D comprising at its 3' end a 15 to 30 bp sequence that is complementary to that at the 3' end of a region encoding a desired protein or that located downstream of the 3' end. PCR is performed using as a template genomic DNA, cDNA or isolated secretory protein gene of filamentous fungi, and using as primers oligonucleotides A and C. Thus, DNA (in which DNA encoding the N-terminus of a desired protein has been added to its 3' side) encoding a signal peptide is amplified. Subsequently, PCR is performed using DNA encoding the desired protein (obtained above) as a template, and using oligonucleotides B and D as primers. Thus, DNA (in which DNA encoding the C terminus of a signal peptide has been added to its 5' side) encoding the desired protein is amplified. Both the amplified DNA fragments and, oligonucleotides A and D, are mixed and PCR is performed. A region at the 3' end of a sense strand of DNA encoding the signal peptide and a region at the 5' end of an antisense strand of DNA encoding a desired protein are complementary to each other and thus hybridize to each other. Both the regions function as primers and templates to each other, thereby amplifying DNA encoding the desired protein in which the signal peptide of the secretory protein of filamentous fungi has been added to the N-terminus of the desired protein.

Examples of a signal peptide of the secretory protein of filamentous fungi that can be used for secretory production of the above desired protein include a signal peptide of phytase of *Aspergillus niger,* a signal peptide of acid phosphatase of *Monascus purpureus,* and a signal peptide of Taka-amylase A of *Aspergillus oryzae*.

In addition, a desired protein can also be expressed as a fusion protein with another protein or a peptide to facilitate its detection and purification. Examples of a protein or a peptide to be fused with a desired protein include β-galactosidase, protein A, IgG binding domain of protein A, chloramphenicol acetyltransferase, poly (Arg), poly (Glu), protein G, maltose binding protein, glutathione S-transferase, poly histidine chain (His-tag), S peptide, DNA-binding protein domain, Tac antigen, thioredoxin, green fluorescent protein, FLAG peptide and an epitope of any antibody [Akio YAMAKAWA, Experimental Medicine, 13, 469-474 (1995)].

A desired protein can be isolated and purified as described below from the culture of transformants of a filamentous fungus belonging to the genus *Monascus*.

When the protein is secreted extracellularly from transformants, the culture of the transformants is processed by, for example, filter filtration or centrifugal separation to obtain the culture supernatant. Purification from the culture supernatant can be performed by methods normally used to isolate and purify enzymes, such as a solvent extraction method, salting-out/desalination using ammonium sulfate or the like, sedimentation using an organic solvent, diethylaminoethyl (DEAE)-sepharose (Amersham Pharmacia Biotech), anion exchange chromatography using resin, e.g., DIAION HPA-75 (Mitsubishi Chemical Corporation), cation exchange chromatography using resin, such as S-SepharoseFF (Amersham Pharmacia Biotech), a hydrophobic chromatography method using resin, such as butyl sepharose or phenyl sepharose, gel filtration using a molecular sieve, an affinity chromatography method, a chromatofocusing method, and electrophoresis, such as isoelectric focusing.

When a desired protein is accumulated in a state of being lysed within the cells of transformants, the culture is centrifuged to collect cells within the culture. Then the cells are washed, and then homogenized using an ultrasonicator, french press, Manton Gaulin homogenizer, Dynomill or the like, thereby obtaining cell-free extract. Purification and isolation can be performed in the same manner similar to that employed for the above culture supernatant from the supernatant provided by centrifugation of the cell-free extract.

In addition, when a desired protein is expressed as intracellular insoluble particles, the cells are collected and homogenized in the same manner as described above and then centrifuged, thereby collecting fractions of the precipitate containing the insoluble particles of the protein. The insoluble particles of the protein is solubilized using a protein denaturation agent. Purification and isolation can also be performed by methods similar to the above methods. In this case, when the solubilization solution contains a protein denaturation agent, the solubilization solution is diluted or dialyzed until the concentration of the agent is lowered to the extent that no protein is denatured, so as to refold the protein to have the normal three-dimensional structure before isolation and purification.

Structural analysis on the target polypeptide (or partial polypeptide) purified herein can be made according to the method normally employed in protein chemistry, for example, the method described in protein structure analysis for gene cloning (Hisashi HIRANO, published by TOKYO KAGAKU DOZIN CO., LTD., 1993).

### 3. Protein derived from filamentous fungi belonging to the genus Monascus

### (1) DNA encoding protein derived from filamentous fungi belonging to the genus Monascus

The present invention encompasses a protein encoded by a gene derived from filamentous fungi belonging to the genus *Monascus* which is obtained by the methods described in 1. (3) and (5) above, and DNA encoding the protein. In addition, DNAs encoding other proteins derived from filamentous fungi belonging to the genus *Monascus* can also be obtained as described below.

DNA encoding a protein derived from filamentous fungi belonging to the genus *Monascus* can be obtained by preparing genomic DNA libraries or cDNA libraries according to the standard methods described in Molecular Cloning 2nd Edition, Current Protocols in Molecular Biology, DNA Cloning 1: Core Techniques, A Practical Approach, Second Edition, Oxford University Press (1995) and the like. Such DNA can also be obtained by preparing cDNA libraries using a commercially available kit, such as Superscript Choice System for cDNA Synthesis (Invitrogen) or ZAP-cDNA Synthesis Kit (STRATAGENE).

Any cloning vector can be used to prepare cDNA libraries, so far as it can autonomously replicate in *Escherichia coli* strain K12. For example, a phage vector or a plasmid vector may be used. Specific examples include ZAPExpress (STRATAGENE), pBluescript II SK (+) [Nucleic Acids Research, 17, 9494 (1989), STRATAGENE], ? ZAPII (STRATAGENE), ?gt10, ?gt11 [both from DNA Cloning, A Practical Approach, Oxford University Press (1985)], ? ExCell (Amersham Pharmacia Biotech), and pUC18 [Gene, 33, 103 (1985)].

Any microorganism belonging to *Escherichia coli* can be used as an microorganism into which a vector having cDNA incorporated therein can be introduced. Specifically, *Escherichia coli* XL1-Blue MRF' (STRATAGENE), *Escherichia coli* Y1088 [Science, 222, 778 (1983)], *Escherichia coli* Y1090 [Science, 222, 778 (1983)], *Escherichia coli* NM522 [J. Mol. Biol., 166, 1 (1983)], *Escherichia coli* JM105 [Gene, 38, 275 (1985)] and the like can be used.

The nucleotide sequences in the thus prepared genomic libraries or cDNA libraries are determined by a DNA sequencer. Then the sequences are compared with their corresponding gene sequences of closely related filamentous fungi, so that proteins encoded by the nucleotide sequences can be specified. Specific examples include a protein derived from a filamentous fungus belonging to the genus *Monascus* and a gene encoding the protein that are specified as described below.

The nitrate reductase gene and nitrate reductase of *Monascus purpureus* can be specified based on the nucleotide sequence of nitrate reductase gene *niaD* of *Aspergillus oryzae* (GenBank Accession No. D49701) and the amino acid sequence (SEQ ID NO: 2) of nitrate reductase encoded by the gene. As preferred examples, the nucleotide sequence on the genome of the nitrate reductase gene of *Monascus purpureus* is shown in SEQ ID NO: 1, the DNA sequence of the coding region is shown in SEQ ID NO: 3, and an amino acid sequence of the nitrate reductase of *Monascus purpureus* specified from these sequences is shown in SEQ ID NO: 4.

The acetamidase of *Monascus purpureus* can be specified based on the nucleotide sequence of acetamidase gene *amdS* of *Aspergillus oryzae* (GenBank Accession No. D10492) and the amino acid sequence (SEQ ID NO: 6) of acetamidase encoded by the gene. As preferred examples, the nucleotide sequence on the genome of the acetamidase gene of *Monascus purpureus* is shown in SEQ ID NO: 5, the DNA sequence of the coding region is shown in SEQ ID NO: 7, and an amino acid sequence of the acetamidase specified from these sequences is shown in SEQ ID NO: 8.

Alcohol dehydrogenase II of *Monascus purpureus* can be specified based on the nucleotide sequence of the alcohol dehydrogenase II gene *alcB* of *Monascus nidulans* (GenBank Accession No.: Z48000) and the amino acid sequence (SEQ ID NO: 10) of alcohol dehydrogenase II encoded by this gene. As preferred examples, the nucleotide sequence on the genome of the alcohol dehydrogenase II gene of *Monascus purpureus* is shown in SEQ ID NO: 9, the DNA sequence of the coding region is shown in SEQ ID NO: 11, and an amino acid sequence of the alcohol dehydrogenase II specified from these sequences is shown in SEQ ID NO: 12.

Acid phosphatase of *Monascus purpureus* can be specified based on the acid phosphatase gene *aph* of *Aspergillus niger* (GenBank Accession No. L02420) and the amino acid sequence (SEQ ID NO: 14) of the acid phosphatase encoded by the gene. As preferred examples, the nucleotide sequence on the genome of the acid phosphatase gene of *Monascus purpureus* is shown in SEQ ID NO: 13, the DNA sequence of the coding region is shown in SEQ ID NO: 15, and the amino acid sequence of acid phosphatase specified from these sequences is shown in SEQ ID NO: 16.

The above protein derived from a filamentous fungus belonging to the genus *Monascus* is the protein of the present invention, and can be efficiently expressed by the method described in 2. However, the protein of the present invention does not include a protein having an amino acid sequence which is identical to that of a known protein.

The protein of the present invention also includes a protein comprising an amino acid sequence derived from the amino acid sequence of the protein by deletion, substitution or addition of one or more amino acids, and having the same activity as that of the protein. Such protein can also be efficiently expressed by the method described in 2. The protein comprising an amino acid sequence derived from that of the above protein by deletion, substitution or addition of one or more amino acids, and having the same activity as that of the protein can be obtained by, for example, introducing a site-directed mutation into DNA encoding proteins comprising amino acid sequences represented by the above Sequence ID Numbers, according to a site-directed mutagenesis method described in, for example, Molecular Cloning 2nd Edition; Current Protocols in Molecular Biology; Nucleic Acids Research, 10, 6487 (1982); Proc. Natl. Acad. Sci. USA, 79, 6409(1982); Gene, 34, 315 (1985); Nucleic Acids Research, 13, 4431 (1985); and Proc. Natl. Acad. Sci. USA, 82, 488 (1985). The number of amino acids that is deleted, substituted or added is not specifically limited, and preferred is the number of amino acids that can be deleted, substituted or added by the known method, such as the above site-directed mutagenesis. A preferred number of amino acids to be modified herein'is 1 to several dozen, more preferably 1 to 20, further preferably 1 to 10, and further more preferably 1 to 5 amino acids.

An example of a protein which is modified by the above site-directed mutagenesis, and maintains its activity after modification is a protein that has at least 60% or more, preferably 80% or more, and further preferably 95% or more homology with the amino acid sequence of the unmodified protein when calculated by BLAST using initially set parameters. Alternatively, these numerical values of homology may be calculated by FASTA using default (initially set) parameters.

Examples of DNAs encoding proteins derived from a filamentous fungus belonging to the genus *Monascus* are those encoding the above proteins. Specific examples of such DNAs include a DNA comprising the nucleotide sequence represented by SEQ ID NO: 1, 3, 5, 7, 9, 11, 13 or 15. A DNA which hybridizes under stringent conditions to a part of or the whole of these DNAs can also be used as DNAs encoding proteins derived from a filamentous fungus belonging to the genus *Monascus*. The stringent conditions are similar to those in 1 (3). The DNA of the present invention also includes these DNAs. However, the DNA of the present invention does not include those comprising a nucleotide sequence which is the same as that of a known DNA.

### (2) Application of proteins derived from filamentous fungi belonging to the genus Monascus

Nitrate reductase of the present invention is useful for quantitative determination of nitric acid metabolism [Agric. Biol. Chem., 47, 2427-2433 (1983)]. Acetamidase of the present invention is useful for wastewater treatment because it has a degradation activity of acrylamide [Water Res., 16, 579-591, (1982), Genetica, 90, 133-145 (1993)]. The alcohol dehydrogenase II of the present invention is useful for modification of alcohol metabolism of microorganisms or living organisms [Gene, 51, 205-216 (1987)]. The acid phosphatase of the present invention is useful for improvement of the nutritional value of feed and efficient utilization of phosphorus (reduction of phosphorus excretion) by domestic animals, because it has a degradation activity of phytic acid contained in feed [J. Sci. Food Agric., 49, 315-324 (1989)].

### (3) Oligonucleotides of DNAs encoding proteins derived from filamentous fungi belonging to the genus Monascus

The present invention encompasses an oligonucleotide comprising a nucleotide sequence which is complementary to a part of or the whole nucleotide sequence of DNA encoding the protein of the present invention as described in (1) above. PCR using these oligonucleotides as a sense primer and antisense primer, respectively, enables specific amplification of DNA encoding the protein of the present invention, isolation of the DNA, and detection and quantitative determination of the DNA. When the oligonucleotides are used as primers for RT-PCR which involves extracting RNA from a sample, converting the RNA into cDNA, followed by PCR, the expression amount of a gene encoding the protein can be measured. Further, an oligonucleotide comprising a nucleotide sequence which is complementary to a part of the nucleotide sequence of DNA encoding the protein of the present invention can be used as an antisense oligonucleotide for regulation, such as suppression of expression of the protein.

Examples of the oligonucleotide include DNA comprising a nucleotide sequence which is the same as a sequence of 5 to 60 nucleotides, preferably 15 to 60 nucleotides, in the nucleotide sequence of DNA encoding the protein of the present invention described in (1), or DNA comprising a nucleotide sequence which is complementary to the DNA. Specific examples of the oligonucleotide include DNA comprising a nucleotide sequence which is the same as a sequence of 5 to 60 nucleotides, preferably 15 to 60 nucleotides in the nucleotide sequence represented by SEQ ID NO: 1, 3, 5, 7, 9, 11, 13 or 15, or DNA comprising a nucleotide sequence which is complementary to that of the DNA. Preferably, the oligonucleotides used as sense primers or antisense primers are the above-mentioned oligonucleotides, since the melting temperatures (Tm) and number of nucleotides of these oligonucleotides never differ significantly. Such oligonucleotides can be prepared by a DNA synthesizer from the nucleotide sequence information of the DNA.

Furthermore, the oligonucleotide of the present invention also includes derivatives of these oligonucleotides (hereinafter referred to as "oligonucleotide derivative"). Examples of the oligonucleotide derivative include an oligonucleotide derivative in which a phosphodiester bond in the oligonucleotide is converted to a phosphorothioate bond; an oligonucleotide derivative in which a phosphodiester bond in the oligonucleotide is converted to a N3'-P5' phosphoamidate bond; an oligonucleotide derivative in which ribose phosphodiester in the oligonucleotide is converted to a peptide nucleic acid bond; an oligonucleotide derivative in which uracil in the oligonucleotide is substituted with C-5 propynyl uracil; an oligonucleotide derivative in which uracil in the oligonucleotide is substituted with C-5 thiazole uracil; an oligonucleotide derivative in which cytosine in the oligonucleotide is substituted with C-5 propynyl cytosine; an oligonucleotide derivative in which cytosine in the oligonucleotide is substituted with phenoxazine-modified cytosine; an oligonucleotide derivative in which ribose in the oligonucleotide is substituted with 2'-O-propyl ribose; and an oligonucleotide derivative in which ribose in the oligonucleotide is substituted with 2'-methoxyethoxy ribose [Saibo Kogaku(Cell Technology), 16, 1463 (1997)].

### (4) Expression of proteins derived from a filamentous fungus belonging to the genus Monascus

The protein as described in (3) above can be expressed at high levels in the host-vector system of filamentous fungi belonging to the genus *Monascus* of the present invention. Further, this protein can also be expressed using host-vector systems of organisms or cells other than those of the genus *Monascus*. The above protein can be obtained by allowing the DNA of the present invention to be expressed in a host cell according to the methods described in Molecular Cloning 2nd Edition or Current Protocols in Molecular Biology, for example, the method as described below, in addition to the method using the host-vector system of filamentous fungi belonging to the genus *Monascus* of the present invention.

DNA encoding the protein of the present invention, for example, DNA comprising a sequence of any one of SEQ ID NOS: 1, 3, 5, 7, 9, 11, 13 and 15, or a DNA fragment of appropriate length including the region encoding the protein of the present invention is prepared, and the DNA is inserted downstream of a promoter of an appropriate expression vector, thereby preparing a recombinant vector. The recombinant vector is introduced into a host cell appropriate for the expression vector. If necessary, DNA is prepared to have a nucleotide sequence (section) encoding the protein of the present invention which is modified by substituting a nucleotide(s) to have optimum codons for expression in the host cell. This DNA is useful for efficient production of the protein of the present invention. As described above, the DNA of the present invention can also be applied for the purposes other than as a selection marker, expression promoter and terminator of gene recombination in host filamentous fungi.

### a) Hosts and vectors

Any cells, such as bacteria, yeast, filamentous fungi, animal cells, insect cells and plant cells, that can express a target gene can be used as host cells.

An expression vector that is used herein is capable of autonomously replicating in the above host cells or can be incorporated into a chromosome, and has a promoter at a position which enables transcription of DNA encoding the protein of the present invention.

When prokaryotes, such as bacteria, are used as host cells, DNA comprising a sequence containing no intron, for example, DNA comprising a sequence of SEQ ID NO: 3, 7, 11 or 15 is used as a DNA encoding the protein of the present invention. A preferred recombinant vector comprising the DNA encoding the protein of the present invention is capable of autonomously replicating in prokaryotes, and comprises a promoter, ribosome binding sequence, DNA encoding the protein of the present invention and a terminator. DNA regulating a promoter may also be included. Examples of the expression vector include pKK223-2, (Amersham Pharmacia Biotech), pGEX-2T (Amersham Pharmacia Biotech), pSE420 (Invitrogen), pLEX (Invitrogen), pET-3a (Novagen), pGEMEX-1 (Promega), pQE-30 (QIAGEN), pCAL-c (STRATAGENE), pEGFP (Clontech), pKYP10 (Japanese Published Unexamined Patent Application No. 110600/83), pKYP200 [Agric. Biol. Chem., 48, 669 (1984)], pTrS20 (Japanese Published Unexamined Patent Application No. 22979/91), and pTerm2 (Japanese Published Unexamined Patent Application No. 22979/91). Any promoter which can function in a host cell may be used. Examples of such a promoter include those derived from the gene of *Escherichia coli* or of *Escherichia coli* phage, such as trp promoter (Ptrp), lac promoter, P_{L} promoter, P_{R} promoter and T7 promoter. Further, artificially designed and modified promoters, such as a promoter having two Ptrp lined in series, tac promoter, lacT7 promoter and letI promoter may also be used. Preferably, a space between a Shine-Dalgarno sequence, which is a ribosome binding sequence, and the initiation codon is 6 to 18 bp. A terminator is not always necessary, but when located downstream of the DNA encoding the protein of the present invention, the expression efficiency can be enhanced.

Examples of the host cells include microorganisms belonging to the genus *Escherichia*, the genus *Serratia*, the genus *Bacillus*, the genus *Brevibacterium*, the genus *Corynebacterium*, the genus *Microbacterium*, the genus *Pseudomonas* and the like, such as *Escherichia coli, Serratia ficaria, Serratia fonticola, Serratia liquefaciens, Serratia marcescens, Bacillus subtilis, Bacillus amyloliquefacines, Brevibacterium ammoniagenes, Brevibacterium immariophilum, Brevibacterium saccharolyticum, Brevibacterium flavum, Brevibacterium lactofermentum, Corynebacterium glutamicum, Corynebacterium acetoacidophilum, Microbacterium ammoniaphilum, Pseudomonas putida* and the like, and *Escherichia coli* is preferred. Examples of *Escherichia coli* include *Escherichia coli* HB101, *Escherichia coli* JM105, *Escherichia coli* BL21, *Escherichia coli* GI724, *Escherichia coli* BL21 (DE3) pLysS, *Escherichia coli* JM109, *Escherichia coli* JM109 (DE 3), *Escherichia coli* M15 (pREP4) and *Escherichia coli* 13009 (pREP4). Any method for introducing a recombinant vector can be used, so far as it is a method for introducing DNA into the above host cells. Examples of such a method include a method using calcium ion [Proc. Natl. Acad. Sci. USA, 69, 2110 (1972)], a protoplast method (Japanese Published Unexamined Patent Application No. 248394/88), and a method described in Gene, 17, 107 (1982) and Mol. Gen. Genet., 168, 111 (1979).

When filamentous fungi are used as hosts, protein can be expressed in a manner similar to that employed for expression in filamentous fungi belonging to the genus *Monascus* in 2. A vector used herein comprises DNA encoding the protein of the present invention inserted downstream of a promoter that functions in the filamentous fungus acting as a host. Examples of promoters include promoters derived from alcohol dehydrogenase gene, acid phosphatase gene, glyceraldehyde-3-phosphate dehydrogenase gene, phosphoglycerate kinase gene, glucoamylase gene, phytase gene, protease gene, cellulase gene and the like.

Examples of a host include *Aspergillus nidulans, Aspergillus niger, Aspergillus oryzae* and *Trichoderma reesei.* Transformation can be performed by the protoplast method [Gene, 26, 205-221 (1983)].

When yeast is used as a host cell, examples of an expression vector include YEP13 (ATCC37115), YEp24 (ATCC37051), YCp50 (ATCC37419), pHS19 and pHS15. Any promoter which can be expressed in a yeast strain may be used. Examples of such a promoter include a promoter of the gene in glycolytic pathway, such as hexose kinase; PHO5 promoter, PGK promoter, GAP promoter, ADH promoter, gal 1 promoter, gal 10 promoter, heat-shock protein promoter, MFαI promoter and CUP 1 promoter.

Examples of host cells include microorganisms belonging to the genus *Saccharomyces*, the genus *Schizosaccharomyces*, the genus *Kluyveromyces,* the genus *Trichosporon*, the genus *Schwanniomyces*, the genus *Pichia*, the genus *Candida* and the like, such as *Saccharomyces cerevisiae*, *Schizosaccharomyces pombe*, *Kluyveromyces lactis*, *Trichosporon pullulans*, *Schwanniomyces alluvius* and *Candida utilis*. Any method for introducing a recombinant vector may be used, so far as it is a method for introducing DNA into yeast. Examples of such a method include electroporation [Methods Enzymol., 194, 182 (1990)], a spheroplast method [Proc. Natl. Acad. Sci. USA, 75, 1929 (1978)], a lithium acetate method [J. Bacteriol., 153, 163 (1983)] and a method described in Proc. Natl. Acad. Sci. USA, 75, 1929 (1978).

When animal cells are used as host cells, examples of an expression vector include pcDNA3.1 (+) (Invitrogen), pEF1/HisA (Invitrogen), pCMV-Script (STRATAGENE), pEGFP-Cl (Clontech) pAGE103 [J. Biochem., 101, 1307 (1987)], pAGE107 [Japanese Published Unexamined Patent Application No. 22979/91; Cytotechnology, 3, 133 (1990)], pAS3-3 (Japanese Published Unexamined Patent Application No. 227075/90), and pCDM8 [Nature, 329, 840 (1987)]. Any promoter which can function in an animal cell can be used. Examples of such a promoter include a promoter of IE (immediate early) gene of cytomegalic inclusion disease virus (CMV), SV40 early promoter, promoter of retrovirus, metallothionein promoter, heat shock promoter and SRα promoter. In addition, enhancer of IE gene of human CMV may be used together with the promoter.

Examples of a host cell include Namalwa cell which is a human cell, COS cell which is a monkey cell and CHO cell which is Chinese hamster cell. Any method for introducing a recombinant vector into an animal cell may be used, so far as it is a method for introducing DNA into an animal cell. Examples of such a method include electroporation [Cytotechnology, 3, 133 (1990)], a calcium phosphate method [Japanese Published Unexamined Patent Application No. 227075/90], and a lipofection method [Proc. Natl. Acad. Sci. USA, 84, 7413 (1987), Virology, 52, 456 (1973)].

When an insect cell is used as a host, protein can be expressed by methods described in, for example, Current Protocols in Molecular Biology; Baculovirus Expression Vectors, A Laboratory Manual, W. H. Freeman and Company, New York (1992); Bio/Technology, 6, 47 (1988); and the like. Specifically, protein can be expressed by co-introducing a recombinant gene introduction vector and Baculovirus into an insect cell so as to obtain a recombinant virus in the culture supernatant of the insect cell, and allowing the recombinant virus to infect insect cells.

Examples of a vector for gene introduction used in this method include pVL1392, pVL1393 and pBlueBac4.5 (all manufactured by Invitrogen). Examples of Baculovirus include Autographa californica nuclear polyhedrosis virus and the like, which infect insects of the family *Barathra*.

Examples of an insect cell that can be used herein include Sf9 and Sf21 which are cells of *Spodoptera frugiperda* [Baculovirus Expression Vectors, A Laboratory Manual, W. H. Freeman and Company, New York (1992)] and High 5 which is a cell of *Trichoplusia ni* (Invitrogen). Examples of a method for co-introducing the above recombinant gene-introduced vector and the above Baculovirus into insect cells so as to prepare a recombinant virus include a calcium phosphate method (Japanese Published Unexamined Patent Application No. 227075/90) and a lipofection method [Proc. Natl. Acad. Sci. USA, 84, 7413 (1987)].

When a plant cell is used as a host cell, examples of an expression vector include Ti plasmid and tobacco mosaic virus vector. Any promoter which can be expressed in a plant cell may be used. Examples of such a promoter include 35S promoter of cauliflower mosaic virus and rice actin 1 promoter. Examples of a host cell include cells of plants, such as tobacco, potato, tomato, carrot, soybean, rape, alfalfa, rice, wheat, barley and the like. Any method for introducing a recombinant vector may be used, so far as it is a method for introducing DNA into a plant cell. Examples of such a method include a method using Agrobacterium (Japanese Published Unexamined Patent Application No. 140885/84, Japanese Published Unexamined Patent Application No. 70080/85, WO 94/00977), electroporation (Japanese Published Unexamined Patent Application No. 251887/85), and a method using particle gun (a gene gun) (Japanese Patent Nos. 2606856 and 2517813).

### b) Culture of transformants and production of proteins

The protein of the present invention can be produced by culturing the transformants obtained as described above in media, allowing the protein to be produced and accumulated in the culture, and recovering the protein from the culture. The transformants of the present invention can be cultured in media according to the method normally employed for culturing hosts.

When transformants are obtained using prokaryotes, such as *Escherichia coli,* or eukaryotic microorganisms, such as filamentous fungi, yeast and the like as hosts, a medium for culturing the transformants may be either a natural or synthetic medium, so far as it contains sources and the like assimilable by the transformants, such as carbon sources, nitrogen sources and inorganic salts, so that the transformants can be efficiently cultured in the medium. Any carbon source which is assimilable by the transformants may be used. Examples of such a carbon source that can be used herein include glucose, fructose, sucrose, molasses containing these compounds, carbohydrate, such as starch and starch hydrolysate, organic acid, such as acetic acid and propionic acid, and alcohol, such as ethanol and propanol. Examples of a nitrogen source that can be used herein include ammonium salt of inorganic acid or organic acid, such as ammonia, ammonium chloride, ammonium sulfate, ammonium acetate and ammonium phosphate, other nitrogen-containing compounds, and peptone, meat extract, yeast extract, corn steep liquor, casein hydrolysate, soybean cake and soybean cake hydrolysate, and various fermentation microbial cells and digested products thereof. Examples of the inorganic salt that can be used herein include potassium dihydrogen phosphate, dipotassium hydrogen phosphate, magnesium phosphate, magnesium sulfate, sodium chloride, ferrous sulfate, manganese sulphate, copper sulfate and calcium carbonate.

Culturing is performed under aerobic conditions, such as shaking culture or submerged culture with aeration and agitation. Culturing temperature is not specifically limited, and is preferably 15 to 40°C. Culturing time is not specifically limited, and is preferably 16 hours to 7 days. pH during culturing is preferably maintained from 3.0 to 9.0. pH can be adjusted with inorganic or organic acids, alkali solution, urea, calcium carbonate, ammonia or the like. If necessary, antibiotics, such as ampicillin or tetracycline, may be added in media while culturing. When microorganisms transformed with recombinant vectors using inductive promoters are cultured, inducers may be added to media if necessary. For example, when microorganisms transformed with recombinant vectors using lac promoters are cultured, isopropyl-β-D-thiogalactopyranoside or the like may be added to media, and when microorganisms transformed with recombinant vectors using trp promoters are cultured, indole acrylic acid or the like may be added.

Examples of media that can be used for culturing transformants obtained using animal cells as hosts include generally employed RPMI1640 medium [JAMA, 199, 519 (1967)], Eagle's minimal essential medium (MEM) [Science, 122, 501 (1952)] Dulbecco's modified Eagle's medium (MEM) [Virology, 8, 396 (1959)], 199 medium [Proceeding of the Society for the Biological Medicine, 73, 1 (1950)] and these media supplemented with fetal calf serum or the like.

Various conditions for culturing are not specifically limited. Preferably, culturing is performed under conditions of pH 6 to 8 at 30 to 40°C in the presence of 5% CO₂ and the like for 1 to 7 days. If necessary, antibiotics such as kanamycin or penicillin may be added to media while culturing. Further, production amount can be elevated using a gene amplification system with dihydrofolate reductase gene or the like according to the method disclosed in Japanese Published Unexamined Patent Application No. 227075/90.

Examples of media for culturing transformants obtained using insect cells as hosts include generally employed TNM-FH medium [Pharmingen], Sf-900 II SFM medium (Invitrogen), ExCell400, ExCell405 [both manufactured by JRH Biosciences], and an insect medium (Grace) [Nature, 195, 788 (1962)]. Various conditions for culturing are not specifically limited. Preferably, culturing is performed under conditions of pH 6 to 7 at 25 to 30°C and the like for 1 to 5 days. If necessary, antibiotics, such as gentamycin, may be added to media while culturing.

Transformants obtained using plant cells as hosts can be cultured as cells, or cultured after their differentiation into plant cells or organs. Examples of media that can be used for culturing the transformants include generally employed Murashige and Skoog (MS) medium, and White medium, and these media supplemented with plant hormone such as auxin, cytokinin or the like. Various conditions for culturing are not specifically limited. Preferably, culturing is performed under conditions of pH 5 to 9 at 20 to 40°C and the like for 3 to 60 days. If necessary, antibiotics, such as kanamycin or hygromycin, may be added to media while culturing.

As described above, the protein of the present invention can be produced by culturing according to standard culturing methods transformants derived from microorganisms, animal cells or plant cells comprising recombinant vectors in which DNA encoding the protein has been incorporated; allowing the transformants to produce and accumulate the protein; and recovering the protein from the culture.

As the method for producing the protein of the present invention using a plant individual, mention may be made of a method comprising cultivating by a known method [Tissue Culture, 20 (1994), Tissue Culture, 21 (1995), Trends. Biotechnol., 15, 45 (1997)] a transgenic plant to which DNA encoding the protein has been introduced; allowing the protein to be produced and accumulated in the plant; and recovering the protein from the plant.

The protein of the present invention can be produced by a method by which the protein is produced within the host cell; a method by which the protein is secreted out of the host cell; or a method by which the protein is produced on the outer membrane of the host cell. Any of these methods can be selected, depending on alteration of the structure of host cells employed or of the protein to be produced. When the protein of the present invention is produced within a host cell or on the outer membrane of a host cell, the protein can be actively secreted out of the host cell according to the method of Paulson et al [J. Biol. Chem. 264, 17619 (1989)], the method of Lowe et al [Proc. Natl. Acad. Sci. USA, 86, 8227 (1989), Genes Develop., 4, 1288 (1990)] or methods disclosed in Japanese Published Unexamined Patent Application No. 336963/93, and WO 94/23021. In other words, by means of genetic recombination techniques, the protein of the present invention can be actively secreted out of the host cells, by allowing the protein to be expressed in such a form that a signal peptide is added to and ahead of, the polypeptide comprising the active site of the protein.

Further, the protein of the present invention can be expressed as a fusion protein with another protein or a peptide in order to facilitate the detection and purification. Examples of a protein or a peptide to be fused with the protein of the present invention include β-galactosidase, protein A, IgG binding domain of protein A, chloramphenicol acetyltransferase, poly (Arg), poly (Glu), protein G, maltose binding protein, glutathione S-transferase, polyhistidine chain (His-tag), S peptide, DNA-binding protein domain, Tac antigen, thioredoxin, green fluorescent protein, FLAG peptide and an epitope of any antibody [Akio YAMAKAWA, Experimental Medicine, 13, 469-474 (1995)].

Proteins having sugar chains added thereto can be obtained by expression of the protein of the present invention in any one of a filamentous fungus, yeast, animal cell, insect cell or plant cell.

Isolation and purification of the protein produced by the transformants of the present invention can be performed by the method described in 2.

### BRIEF DESCRIPTION OF THE DRAWINGS

Figure 1 shows detection of pMA-niaD and pANPHY1 that have been introduced into *Monascus purpureus* strain SN2-30-4, by Southern blotting of chromosomal DNA. Lanes 1 to 4 denote transformants in which only pMA-niaD has been introduced, lane 5 denotes host strain SN 2-30-4, and lanes 6 to 9 denote transformants in which pMA-niaD and pANPHY1 have been co-introduced. The numbers and lines on the left denote the position of each marker and chain length (kb), respectively.
Figure 2 shows Northern blotting detection of ADH2 mRNA of *Monascus purpureus* strain IFO 30873 which has been cultured in various media containing different carbon sources. Culture conditions for each lane (the carbon source in a medium, static culture or shaking culture) are shown on the right side.
Figure 3 shows a process for constructing pMGON-HLY. HLY represents a gene comprising the signal sequence of chicken lysozyme and a human lysozyme structural gene.
Figure 4 shows a process for constructing pMGB-TAA. TAA represents Taka-amylase A gene.
Figure 5 shows a process for constructing pMAB-PHY.
Figure 6 shows a process for constructing pMGB-PHY.
Figure 7 shows a process for constructing pMAPA-PHY.
Figure 8 shows a process for constructing phytase gene having the signal peptide of APH.
Figure 9 shows a process for constructing phytase gene having the signal peptide of Taka-amylase A.
Figure 10 shows a process for constructing pMGB-tPHY.

### BEST MODE FOR CARRYING OUT THE INVENTION

The present invention is more specifically described by the following examples. However, these examples are given only for illustrative purposes, and do not limit the technical scope of the present invention.

### Example 1 Isolation of nitrate reductase-deficient strain (niaD⁻ strain)

Using *Monascus purpureus* wild strain [Institute for Fermentation, Osaka (IFO) Accession No. IFO30873, hereinafter referred to as strain IFO30873], a nitrate reductase-deficient strain was isolated by the following method.

5 mm mycelial block was picked out, of *Monascus purpureus* strain IFO 30873 previously cultured on slant media for conservation [Bacto potato-dextrose (Difco) 24 g/l, 1.5% agar (Difco), pH 5.5], transferred to fresh potato-dextrose agar plate media (the plate medium was prepared to have the same composition as that of the above slant medium), and then cultured at 30°C for 7 to 10 days, so that the conidia started to grow on the media. A sterilized 5 ml of 0.01 % (v/v) Tween 80 aqueous solution was added to the plate medium, and then the conidia were suspended well in the solution using a inoculating loop. The suspension was filtered with G3 glass filter to recover the conidia.

The recovered conidia were inoculated into selective plate media (3% sucrose, 10 mmol/l glutamic acid, 0:2% KH₂PO₄, 0.05% MgSO₄·7H₂O, 0.05% KCl, 470 mmol/l KClO₃, pH 5.5), and then cultured at 30°C for 15 to 20 days. Colonies that had grown were used for the following experiments. Colonies capable of growing on the selective plate media appeared at a rate of one colony per about 10,000 conidia.

### Example 2 Identification of nitrate reductase-deficient strain (niaD⁻ strain)

A deficient gene locus on the nitric acid metabolic pathway in the mutant strain provided in Example 1 above was identified by examining the presence or absence of growth on six types of plates as shown below.
(1) 10 mmol/l NaNO₃+ basal plate medium (1% glucose, 0.1% KH₂PO₄, 0.05% MgSO₄·7H₂O, 0.05% KCl, 1.5% agar, pH 5.5)
(2) 10 mmol/l NaNO₂+ basal plate medium
(3) 10 mmol/l (NH₄)₂SO₄+ basal plate medium
(4) 10 mmol/l proline + basal plate medium
(5) 10 mmol/l glutamic acid + basal plate medium
(6) 10 mmol/l alanine + basal plate medium

The mutant strains inoculated on the above 6 types of plates were cultured at 30°C for 3 days. As a result, the mutant strains obtained in Example 1 grew on any one of plates (2) to (6); but did not grow on plate (1), suggesting a possible deficiency in their nitrate reductase activity. One of the strains was used as a host for gene introduction and transformation as *Monascus purpureus* strain SN2-30-4.

### Example 3 Preparation of a chromosomal library

Similarly to Example 1, 5 mm mycelial block was scraped, of *Monascus purpureus* strain IFO30873 previously cultured on slant media for conservation, and then transferred into a 500 ml Erlenmeyer flask with baffle containing 100 ml of dextrin-peptone medium (2% dextrin, 1% peptone, 0.5% KH₂PO₄, 0.1% MgSO₄·7H₂O), and then subjected to static culture at 30°C for 10 days. This culture solution was filtered with G1 glass filter, and then washed twice with sterilized water, thereby collecting approximately 0.5 g of mycelia.

The collected mycelia were placed between pieces of paper towel, compressed for dewatering, and then the mycelia were put into a mortar cooled at -80°C. After liquid nitrogen was poured into the mortar, the mycelia were crushed with a pestle quickly. The crushed mycelia were put into Eppendorf tubes, and suspended by the addition of 0.3 ml of TE buffer [10 mmol/l Tris-HCl (pH 8.0), 1 mmol/l EDTA]. Further, 0.3 ml of lysis solution [2% SDS, 0.1 mol/l NaCl, 10 mmol/l EDTA, 50 mmol/l Tris-HCl (pH 7.0)] was added to the suspension, and the solution was maintained at 37°C for 30 min to perform lysis. The obtained lysate solution was centrifuged at 12,000xg, thereby collecting a supernatant. The supernatant was subjected in sequence to phenol treatment, ethanol precipitation, RNase treatment, phenol treatment (twice), chloroform treatment and then ethanol precipitation. Thus, chromosomal DNA was purified. Approximately 50 µg of chromosomal DNA was obtained by these procedures.

2 µg of the purified chromosomal DNA was digested with restriction enzyme *Bam*H I at 37°C for 2 hours. Then, the product was ligated to Lambda EMBL3-*Bam*H I arm (STRATAGENE) using T4 ligase. The obtained phage DNA was packaged using Gigapack Gold (STRATAGENE), and then infected with *Escherichia coli* strain P2392 (STRATAGENE), thereby constructing chromosomal DNA libraries.

### Example 4 Isolation of DNA encoding nitrate reductase derived from Monascus purpureus

Nitrate reductase gene was isolated from the above chromosomal DNA library by a standard plaque hybridization method. As a probe for plaque hybridization, a 5 kb *Hin*d m fragment of plasmid pND300 [Biosci. Biotech. Biochem., 59, 1795-1797 (1995)] comprising *niaD* gene derived from *Aspergillus oryzae* was used. To perform labeling of the probe, hybridization, and detection of signals, ECL Direct Nucleic Acid Labeling and Detection System (Amersham Pharmacia Biotech) was used. As a result, eight clones to which the above DNA probe hybridized were obtained from approximately 10,000 plaques.

Phage DNA was prepared by liquid culture from these positive plaques according to a standard method. Then, the DNA was digested with restriction enzyme *Bam*H I, and then subjected to 0.8% agarose gel electrophoresis, thereby obtaining an about 12 kb DNA fragment. The fragment was subcloned to *Bam*H I site of pUC18 according to the standard method to construct pMA-niaD. *Escherichia coli* strain HB101 was transformed with pMA-niaD. pMA-niaD was prepared in large quantities, and then the nucleotide sequence of the DNA fragment subcloned was determined using a DNA sequencer (ABI377, Perkin Elmer).

As a result, the nucleotide sequence represented by SEQ ID NO: 1 comprising *niaD* gene derived from *Monascus purpureus* was obtained. The DNA sequence was compared with a previously reported sequence of *niaD* gene of *Aspergillus oryzae* (SEQ ID NO: 2) and the position of exon [Biosci. Biotech. Biochem., 59, 1795-1797 (1995): GenBank Accession No. D49701], so that the coding region of the protein was predicted for the DNA of *Monascus purpureus* (SEQ ID NO: 1). The nucleotide sequence of the coding region of the protein is shown in SEQ ID NO: 3; the amino acid sequence of nitrate reductase of *Monascus purpureus* that is encoded by this region is shown in SEQ ID NO: 4.

In addition, the obtained *Escherichia coli* transformant, *Escherichia coli* HB101/pMA-niaD comprising *niaD* gene derived from *Monascus purpureus*, was deposited on March 2, 2000 at the International Patent Organism Depositary, National Institute of Advanced Industrial Science and Technology (Central 6, 1-1-1, Higashi, Tsukuba-shi, Ibaraki, Japan) (formerly, National Institute of Bioscience and Human-technology, Agency of Industrial Science and Technology, Japan, 1-1-3, Higashi, Tsukuba-shi, Ibaraki, Japan) under the accession No. FERM BP-7065.

### Example 5 Transformation method of filamentous fungi belonging to the genus Monascus

### (1) Preparation of protoplast

100 ml of a nutrient medium (1% dextrin, 1% peptone, 1% yeast extract, 0.05% KH₂PO₄, 0.05% MgSO₄·7H₂O, pH 5.2) was dispensed into a 500ml Erlenmeyer flask, and then the media were sterilized. 5 mm mycelial block was scraped with a inoculating loop from the plate media on which *Monascus purpureus* strain SN2-30-4 had been previously cultured, and then inoculated in the nutrient media. Static culture was performed at 30°C for 4 to 5 days. The obtained culture solution was filtered with pre-sterilized G1 glass filter, thereby collecting the cells. Sterilized water was added to wash the cells, and then 0.5g of wet cells was put into each of sterilized test tubes A and B. Next, 10 ml of protoplast preparation solution 1 [5 mg/ml lysing enzymes; Sigma, catalog number L1412, 2.5 mg/ml Sumizyme C (SHIN NIHON CHEMICAL CO., LTD), 0.8 mol/l NaCl, 100 mmol/l phosphate buffer, pH 6.0] previously sterilized by filtration was added to the test tube A; and 10 ml of protoplast preparation solution 2 [5 mg/ml lysing enzymes, Sigma, 0.8 mol/l NaCl, 100 mmol/l phosphate buffer, pH 6.0] previously sterilized by filtration was added to test tube B to suspend the cells. The cells were incubated at 30°C for 3 hours while gently shaking, so that protoplasts were prepared.

### (2) Purification of protoplast

The above two types of enzyme reacted solutions were filtered respectively with G3 glass filter to remove mycelial fragments. Then, the protoplast suspension that had passed through the filter was centrifuged at 700 x G to remove supernatant. 10 ml of 0.8 mol/l NaCl solution was added to the obtained protoplast for re-suspension, and then the mixture was centrifuged at 700 x G. This procedure was repeated twice. Further, 10 ml of solution 1 [10 mmol/l CaCl₂, 0.8 mol/l NaCl, 10 mmol/l Tris-HCl buffer (pH 7.5)] was added to the precipitate for suspension. Then, the suspension was centrifuged at 700 x G for washing the protoplast. The protoplast was suspended in solution 1, and then solution 2 [40% (w/v) PEG 4000, 50 mmol/l CaCl₂, 50 mmol/l Tris-HCl buffer solution (pH 7.5)] was added thereto, thereby preparing a protoplast solution. The protoplast solution consisted of three-fourths volume of solution 1 and one-fourth volume of solution 2, and had a protoplast density of 10⁸/ml.

### (3) Transformation

20 µl of 0.5 to 0.8 µg/µl pMA-niaD plasmid DNA (hereinafter, all plasmid concentrations in transformation are 0.5 to 0.8 µg/µl) was added to two types of 200 µl of protoplast solutions obtained in (2) above, and then ice-cooled for 30 minutes. Next, 1 ml of solution 2 was added to the solutions, and then the solutions were allowed to stand at room temperature for 20 minutes. Next, 10 ml of solution 1 was added to dilute PEG concentration, and then the solutions were centrifuged at 700 x G to collect precipitate. The resulting two types of precipitates were suspended separately in 200 µl of solution 1. The suspension was mixed with a 0.5% soft agar medium (the medium has the same composition as a plate medium described below except for the agar concentration), and then poured onto the basal plate medium containing 10 mmol/l NaNO₃ as a nitrogen source. The plate medium was incubated at 30°C for 10 to 14 days, thereby obtaining transformants.

The transformation efficiency was 1 to 3 colonies/µg DNA when protoplast preparation solution 1 containing lysing enzymes (Sigma) and Sumizyme C (SHIN NIHON CHEMICAL CO., LTD) had been used to prepare protoplasts. However, no transformant was obtained when protoplast preparation solution 2 containing only lysing enzymes (Sigma) had been used to prepare protoplasts. Therefore, better results could be obtained when both lysing enzymes (Sigma) and Sumizyme C (SHIN NIHON CHEMICAL CO., LTD) were used for protoplast preparation in transformation.

Furthermore, chromosomal DNA was prepared from the above transformants, and then integration of the introduced pMA-niaD plasmid was confirmed by Southern hybridization. Southern hybridization was performed as follows. After digestion of 4 µg of chromosomal DNA of *Monascus purpureus* transformant using restriction enzyme *Xba* I, the product was separated by 0.8% agarose gel electrophoresis. The separated DNA was blotted on a nylon membrane filter by capillary blotting. Using ECL Direct Nucleic Acid Labeling and Detection System (Amersham Pharmacia Biotech), hybridization was performed with 100 ng of pUC18 plasmid as a probe according to the attached protocol of the kit.

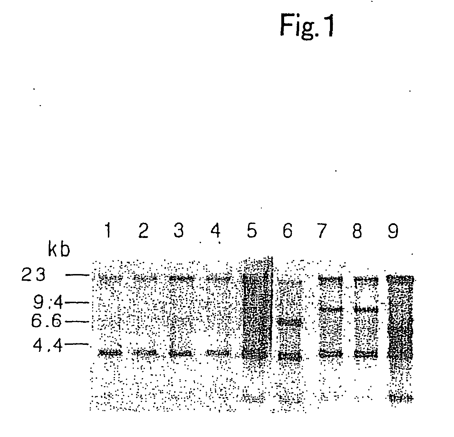

Figure 1 shows the results. In Fig. 1, lanes 1 to 4 denote chromosomal DNA derived from transformants of *Monascus purpureus* strain SN2-30-4 into which pMA-niaD plasmid had been introduced. Lane 5 denotes chromosomal DNA derived from *Monascus purpureus* strain SN 2-30-4 used as a host. For the transformants (lanes 1 to 4), the hybridization bands were observed at around 20 kb in addition to the bands around 4 kb that were observed also for the host DNA (lane 5). Thus, it was confirmed that pMA-niaD plasmid had been introduced into the transformants.

### Example 6 Preparation of Monascus purpureus transformant into which phytase gene derived from Aspergillus niger introduced, and secretory production of phytase by the transformant

### (1) Preparation of protoplast

100 ml of a nutrient medium (1% dextrin, 1% peptone, 1% yeast extract, 0.05% KH₂PO₄, 0.05% MgSO₄·7H₂O, pH 5.2) was dispensed in a 500 ml Erlenmeyer flask, and then the solution was sterilized. 5 mm agar piece was excised using an inoculating loop from the plate media on which *Monascus purpureus* strain SN2-30-4 had been cultured. The piece was inoculated into the nutrient media, and then cultured statically at 30°C for 4 to 5 days. The obtained culture solution was filtered with a pre-sterilized G1 glass filter, thereby recovering the cells. Further, after sterilized water was added for washing the cells, 0.5 g of wet cells was put into a sterilized test tube. Next, 10 ml of a filter-sterilized protoplast preparation solution [5 mg/ml lysing enzymes (Sigma), 2.5 mg/ml Sumizyme C (SHIN NIHON CHEMICAL CO., LTD), 0.8 mol/l NaCl, 100 mmol/l phosphate buffer, pH 6.0] was added to the tube, thereby suspending the cells. The cells were incubated while gently shaking at 30°C for 3 to 5 hours, so that the protoplasts were dissociated.

### (2) Purification of protoplast

The above enzyme reacted solutions were respectively filtered with G3 glass filter to remove mycelial fragments. Then, the protoplast suspension that had passed through the filter was centrifuged at 700 x G to remove supernatant. Further, 10 ml of 0.8 mol/l NaCl solution was added to the obtained precipitate for re-suspension, and then the suspension was centrifuged at 700 x G This procedure was repeated twice. Furthermore, 10 ml of solution 1 (10 mmol/l CaCl₂, 0.8 mol/l NaCl, 10 mmol/l Tris-HCI buffer, pH7.5) was added to the precipitate for suspension, and then the suspension was centrifuged at 700 x G, thereby washing the protoplast. The protoplast was suspended in solution 1, and then solution 2 [40% (w/v) PEG 4000, 50 mmol/l CaCl₂, 50 mmol/l Tris-HCl buffer (pH 7.5)] was added thereto, thereby preparing a protoplast solution. The protoplast solution consisted of three-fourths volume of solution 1 and one-fourth volume of solution 2, and had a protoplast density of 10⁸/ml.

### (3) Transformation

Ten µl of pMA-niaD plasmid and 10 µl of plasmid pANPHY1 (WO97/38096) comprising 4.6 kb of phytase gene *phyA* derived from *Aspergillus niger* were added to 200 µl of the protoplast solution obtained in (2), and then the solution was ice-cooled for 30 min. Next, 1 ml of solution 2 was added to the solution, and then allowed to stand at room temperature for 20 min. Then, 10 ml of solution 1 was added to dilute the protoplast solution, and then the solution was centrifuged at 700 x G, thereby recovering precipitate. The precipitates were suspended respectively in 200 µl of solution 1. The suspension was placed on a basal plate medium containing 10 mmol/l NaNO₃ as a nitrogen source. Further, a 0.5% soft agar medium (the medium has the same composition as the above plate medium except for the agar concentration) was added to the suspension and mixed with it on the plate. Incubation was performed on the plate at 30°C for 10 to 14 days, thereby obtaining transformants having both plasmids co-introduced therein.

Southern hybridization was performed using pUC18 as a probe according to the method described in Example 5 for 4 strains of the obtained transformants (strains named TF1, TF2, TF3 and TF4, respectively), 4 control strains of the transformants comprising only pMA-niaD introduced therein, and host strain SN2-30-4. (pANPHY1 is the plasmid constructed by inserting *phyA* gene into plasmid pUC118 derived from pUC18, and hybridizes with pUC18.) Figure 1 shows the results. Lanes 1 to 4 denote chromosomal DNA samples extracted from the transformants comprising only selection marker plasmid pMA-niaD introduced therein; lane 5 denotes a chromosomal DNA sample extracted from host strain SN 2-30-4; and lanes 6 to 9 denote chromosomal DNA samples extracted respectively from the transformant TF1 to TF4 to which pANPHY1 plasmid comprising *phyA* and pMA-niaD plasmid co-introduced therein. For *phyA* gene-co-introduced transformants of lanes 6 to 9, novel hybridization bands could be detected at around 5 to 9 kb, while such bands were not detected for the transformants of lanes 1 to 4 comprising only pMA-niaD introduced therein. These results suggest that both plasmids, pANPHY1 comprising *phyA* gene and pMA-niaD, were successfully introduced.

### (4) Production of phytase by transformant

The transformant strains TF1, TF2, TF3 and TF4 obtained in (3) were inoculated into a 500 ml Erlenmeyer flask containing 100 ml of a phytase production medium (3% sucrose, 10 mmol/l NaNO₃, 0.05% MgSO₄·7H₂O, 0.05% KCl, 0.1% corn steep liquor) and then subjected to static culture at 30°C for 14 days. As a control, the transformants comprising only pMA-niaD introduced therein were cultured similarly. The cells were removed from the culture solution by filtration to obtain a culture supernatant, and the supernatant was used as a crude enzyme solution.

Phytase activity in the above crude enzyme solution was measured as follows. Enzyme activity was measured at 37°C, wherein 0.2 mol/l acetate buffer (pH5.5) containing 2.5 mmol/l sodium phytate was used as a substrate, and a mixture of acetone: 2.5 mmol/l sulfuric acid: 10 mmol/l ammonium molybdate at 2:1:1 was used as a solution to stop reaction. After 0.5ml of the substrate was incubated for 5 min, 0.5 ml of the crude enzyme solution was added to start the reaction. 10 minutes later, 2 ml of the solution to stop the reaction was added, and then the solution was stirred. Furthermore, the solution was mixed with 0.1 ml of 1 mol/l citric acid. Absorbance at 380 nm was measured by a spectrophotometer, so that the released phosphorus was determined. Under conditions of pH 5.5 and 37°C, enzymatic activity which isolates inorganic phosphorus at a rate of 1 µmol per minute was defined as 1 unit (U). As a result, as shown in Table 1, enzymatic activities of the transformants ranged from 1.4 to 18.0 mU/ml, which were 2 to 20 times greater than the control.

**Table 1**

| Phytase activity of transformant | | |
|---|---|---|
| Transformant strain | Introduction plasmid | Phytase activity (mU/ml) |
| Control | pMA-niaD | 0.8 |
| TF1 | pMA-niaD, pANPHY1 | 18.0 |
| TF2 | pMA-niaD, pANPHY1 | 2.6 |
| TF3 | pMA-niaD, pANPHY1 | 1.4 |
| TF4 | pMA-niaD, pANPHY1 | 16.0 |

These results showed that the improved amount of secretory production of phytase was observed in the transformants of the filamentous fungus belonging to the genus *Monascus* in which plasmid pANPHY1 comprising *phyA* gene derived from *Aspergillus niger* had been introduced. This is because *phyA* gene of *Aspergillus niger* introduced in the transformant was expressed so that phytase of *Aspergillus niger* was produced by secretion.

### Example 7 Isolation of DNA encoding acetamidase derived from Monascus purpureus

### (1) Construction of a chromosomal library

In the same manner as in Example 3, 2 µg of chromosomal DNA purified from *Monascus purpureus* strain IFO30873 was digested with restriction enzyme *EcoR* I at 37°C for 2 hours. Then, the digested product was ligated to lambda EMBL4-*Eco*R I arm (STRATAGENE) using T4 ligase. The resulting phage DNA was packaged using Gigapack Gold (STRATAGENE), and then infected with *Escherichia coli* strain P2392, thereby constructing a chromosomal DNA library.

### (2) Isolation of DNA encoding acetamidase

DNA encoding acetamidase was isolated from the above chromosomal DNA library by a standard plaque hybridization method. An about 2.5 kb fragment of *amdS* gene [Gene, 108, 91 (1991)] derived from *Aspergillus oryzae* (the fragment had been amplified by PCR using genomic DNA of *Aspergillus oryzae* as a template and using sequences of SEQ ID NOS: 19 and 20 as DNA primers) was used as a probe for plaque hybridization. ECL Direct Nucleic Acid Labeling and Detection System (Amersham Pharmacia Biotech) was used to perform labeling of probes, hybridization and signal detection. As a result, 8 clones to which the above DNA probe hybridized were obtained from about 10,000 plaques.

Phage DNA was prepared by liquid culture according to a standard method from these positive plaques. The phage DNA was digested with restriction enzyme *Sal* I, and then an about 6.0 kb DNA fragment was obtained using 0.8% agarose. The fragment was subcloned to *Sal* I site of pUC18 according to a standard method, to construct pMA-amdS. Then, *Escherichia coli* strain HB101 was transformed with pMA-amdS. pMA-amdS was prepared in large quantities, and then the nucleotide sequence of the subcloned DNA fragment was determined using a DNA sequencer (ABI 377, Perkin Elmer).

The thus provided nucleotide sequence comprising *amdS* gene derived from *Monascus purpureus* is shown in SEQ ID NO: 5. The DNA sequence was compared with the previously reported sequence of *amdS* gene of *Aspergillus oryzae* (SEQ ID NO: 6) and the position of exon [Gene, 108, 91-98 (1991): GenBank Accession No. D10492], so as to assume the protein coding region of *Monascus purpureus* DNA of SEQ ID NO: 5. A nucleotide sequence of the protein coding region is shown in SEQ ID NO: 7, and an amino acid sequence of acetamidase of *Monascus purpureus* encoded by this region is shown in SEQ ID NO: 8.

In addition, the resulting *Escherichia coli* transformant HB101/pMA-amdS comprising *amdS* gene derived from *Monascus purpureus* was deposited under Accession No. FERM BP-7064 at the International Patent Organism Depositary, National Institute of Advanced Industrial Science and Technology, (Central 6, 1-1-1, Higashi, Tsukuba-shi, Ibaraki, Japan) (formerly, National Institute of Bioscience and Human-Technology, 1-1-3, Higashi, Tsukuba-shi, Ibaraki, Japan) on March 2, 2000.

### Example 8 Isolation of DNA encoding alcohol dehydrogenase II derived from Monascus purpureus

### (1) Construction of a chromosomal library

In the same manner as in Example 3, 2 µg of chromosomal DNA purified from *Monascus purpureus* strain IFO30873 was digested with restriction enzyme *Eco*R I at 37°C for 2 hours. Then, the digested product was ligated to lambda DASHII-*Eco*R I arm (STRATAGENE) using T4 ligase. The resulting phage DNA was packaged using Gigapack Gold (STRATAGENE), and then infected with *Escherichia coli* strain P2392, thereby constructing a chromosomal DNA library.

### (2) Isolation of DNA encoding alcohol dehydrogenase II

DNA encoding alcohol dehydrogenase II was isolated from the above chromosomal DNA library by a standard plaque hybridization method. An about 0.8 kb fragment corresponding to position 1009 to 1776 of the nucleotide sequence of *alcB* gene [Curr. Genet., 29, 122-129 (1996), SEQ ID NO: 10] derived from *Aspergillus nidulans* (the fragment had been amplified by PCR using genomic DNA of *Aspergillus nidulans* as a template and using sequences of SEQ ID NOS: 21 and 22 as DNA primers) was used as a probe for plaque hybridization. ECL Direct Nucleic Acid Labeling and Detection System (Amersham Pharmacia Biotech) was used to perform labeling of probes, hybridization and signal detection. As a result, 6 clones to which the above DNA probe hybridized were obtained from about 10,000 plaques.

Phage DNA was prepared by liquid culture according to a standard method from these positive plaques. The phage DNA was digested with restriction enzyme *EcoR* I, and then subjected to 0.8% agarose gel electrophoresis, thereby obtaining about a 8.0 kb DNA fragment. The fragment was subcloned into *Eco*R I site of pUC18 according to a standard method to construct pMA-alcB. Then, *Escherichia coli* strain HB101 was transformed with pMA-alcB. pMA-alcB was prepared in large quantities, and then the nucleotide sequence of the subcloned DNA was determined using a DNA sequencer (ABI 377, Perkin Elmer).

The thus provided nucleotide sequence comprising *alcB* gene derived from *Monascus purpureus* is shown in SEQ ID NO: 9. The DNA sequence was compared with the previously reported nucleotide sequence of *alcB* gene of *Aspergillus nidulans* (SEQ ID NO: 10) and the position of exon [Curr. Genet., 29, 122-129 (1996) : GenBank Accession No. Z48000], so as to assume the protein coding region of *Monascus purpureus* DNA of SEQ ID NO: 9. The assumed protein coding region is shown in SEQ ID NO: 11, and an amino acid sequence of alcohol dehydrogenase II of *Monascus purpureus* encoded by this region is shown in SEQ ID NO: 12.

In addition, the resulting *Escherichia coli* transformant JM110/pMA-alcB comprising *alcB* gene derived from *Monascus purpureus* was deposited under Accession No. FERM BP-7066 at the International Patent Organism Depositary, National Institute of Advanced Industrial Science and Technology, (Central 6, 1-1-1, Higashi, Tsukuba-shi, Ibaraki, Japan) (formerly, National Institute of Bioscience and Human-Technology, 1-1-3, Higashi, Tsukuba-shi, Ibaraki, Japan) on March 2, 2000.

### Example 9 Transcriptional regulation of alcB gene derived from Monascus purpureus

*Monascus purpureus* strain IFO30873 was shake-cultured at 28°C for 4 days using potato-dextrose medium. Then the cells were recovered using 3G1 glass filter, and then washed with sterilized water. The cells were transferred into 10 mmol/l phosphate buffer (pH 6.0) and shaken at 28°C for 24 hours, recovered using 3G1 glass filter, and then washed. Next, the cells were transferred respectively to induction media [2% carbon source (fructose, ethanol or glucose), 0.3% NaNO₃, 0.05% MgSO₄·7H₂O, 0.05% KCI, 0.1% KH₂PO₄, pH 5.5], and then cultured at 28°C for 24 hours (shaking culture or static culture). Immediately after culturing, the cells were recovered using 3G1 glass filter, washed, and then frozen with liquid nitrogen. Total RNAs were prepared from the respective cells using ISOGEN (NIPPON GENE CO., LTD.) according to the protocols attached to the product. 20 µg of the obtained RNAs were applied to agarose gel containing formaldehyde, and then subjected to electrophoresis at 30V for 1 hour and then at 60V for 2 hours. The RNAs that had been subjected to electrophoresis were transferred to nylon membrane. According to a standard method, the RNAs were subjected to Northern hybridization using as a probe a 4 kb *Hind* III fragment of plasmid pMA-alcB comprising *alcB* gene derived from *Monascus purpureus* isolated in Example 8. Hybridization was performed at 65°C.

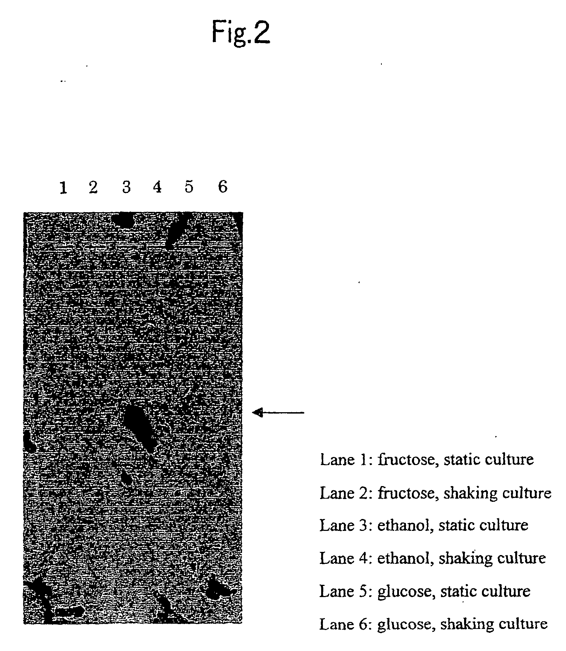

Figure 2 shows the results. In Fig. 2, lane 1 denotes total RNA derived from *Monascus purpureus* strain IFO30873 that was statically cultured in a medium supplemented with fructose as a carbon source for 24 hours; lane 2 denotes total RNA derived from *Monascus purpureus* strain IFO30873 that was shake-cultured in a medium supplemented with fructose as a carbon source for 24 hours; lane 3 denotes total RNA of *Monascus purpureus* strain IFO30873 that was statically cultured in a medium supplemented with ethanol as a carbon source for 24 hours; lane 4 denotes total RNA derived from *Monascus purpureus* strain IFO30873 that was shake-cultured in a medium supplemented with ethanol as a carbon source for 24 hours; lane 5 denotes total RNA derived from *Monascus purpureus* strain IFO30873 that was statically cultured in a medium supplemented with glucose as a carbon source for 24 hours; and lane 6 denotes total RNA derived from *Monascus purpureus* strain IFO30873 that was shake-cultured in a medium supplemented with glucose as a carbon source for 24 hours.

As shown in Fig. 2, when a 4 kb fragment of *alcB* gene derived from *Monascus purpureus* was used as a probe, signals were detected for the RNA prepared from the cells that had been shake-cultured using ethanol as a carbon source. This result suggests that transcription of *alcB* gene is induced by ethanol. Since suppression of transcription of *alcB* gene of *Aspergillus nidulans* by ethanol was reported [Curr. Genet., 29, 122-129 (1996)], it was shown that *alcB* gene of *Monascus purpureus* and that of *Aspergillus nidulans* differ in their transcriptional regulation.

These results suggest that transcription of alcohol dehydrogenase II gene, *alcB*, of *Monascus purpureus* is induced by ethanol.

### Example 10 Isolation of DNA encoding acid phosphatase derived from Monascus purpureus

DNA encoding acid phosphatase was isolated by a standard plaque hybridization method from the chromosomal DNA library of *Monascus purpureus* strain IFO30873 obtained in Example 3. As a probe for plaque hybridization, a 1.5 kb fragment (amplified by PCR using genomic DNA of *Aspergillus niger* as a template and sequences of SEQ ID NOS: 23 and 24 as primers) of *aph* gene [Gene, 133, 55-62 (1993)] derived from *Aspergillus niger* (awamori) was used. ECL Direct Nucleic Acid Labeling and Detection System (Amersham Pharmacia Biotech) was used to perform labeling of probes, hybridization and signal detection. As a result, 5 clones to which the above DNA probe hybridized were obtained from an about 10,000 plaques.

Phage DNA was prepared by liquid culture according to a standard method from these positive plaques. The phage DNA was digested with restriction enzyme *Bam*H I, and then about 10 kb DNA fragment was obtained by 0.8% agarose gel electrophoresis. The fragment was subcloned into *Bam*H I site of pUC18 according to a standard method to construct pMA-aph. Then, *Escherichia coli* strain HB101 was transformed with pMA-aph. pMA-aph was prepared in large quantities, and then the nucleotide sequence of the subcloned DNA was determined using a DNA sequencer (ABI 377, Perkin Elmer).

The thus provided nucleotide sequence comprising *aph* gene derived from *Monascus purpureus* is shown in SEQ ID NO: 13. The nucleotide sequence was compared with the nucleotide sequence (SEQ ID NO: 14) of *aph* gene of *Aspergillus niger* (awamori) and the position of exon [Gene, 133, 55-62 (1993): GenBank Accession No. L02420], so as to assume the protein coding regionof *Monascus purpureus* DNA of SEQ ID NO: 13. The assumed nucleotide sequence of the protein coding region is shown in SEQ ID NO: 15, and an amino acid sequence of acid phosphatase of *Monascus purpureus* encoded by this region is shown in SEQ ID NO: 16.

In addition, the resulting *Escherichia coli* transformant HB101/pMA-aph comprising *aph* gene derived from the genus *Monascus* was deposited under Accession No. FERM BP-7187 at the International Patent Organism Depositary, National Institute of Advanced Industrial Science and Technology, (Central 6, 1-1-1, Higashi, Tsukuba-shi, Ibaraki, Japan) (formerly, National Institute of Bioscience and Human-Technology, 1-1-3, Higashi, Tsukuba-shi, Ibaraki, Japan) on June 15, 2000.

### Example 11 Expression of human lysozyme gene in a filamentous fungus of the genus Monascus

### (1) Construction of expression plasmid of human lysozyme gene

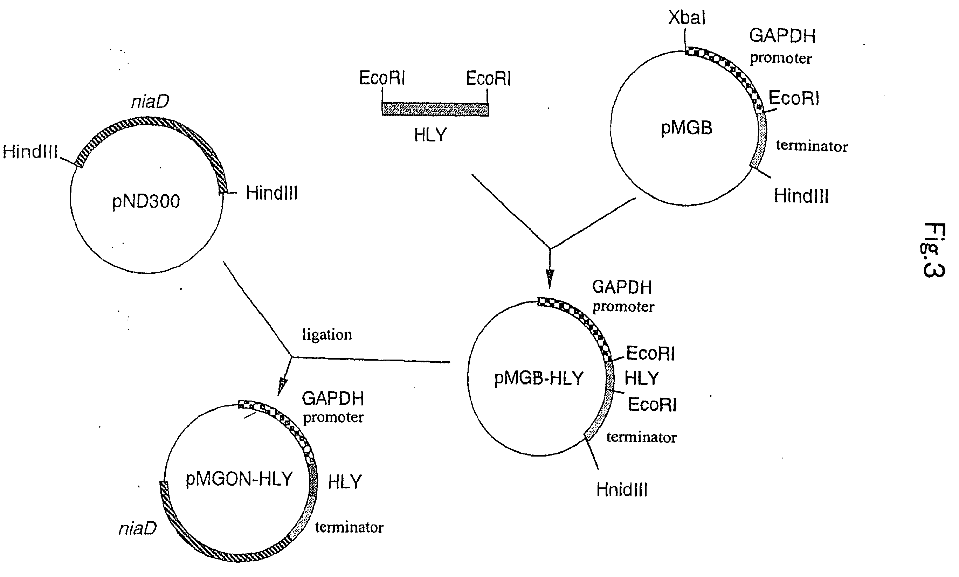

A construction process for expression plasmid pMGON-HLY of human lysozyme gene is shown in Fig. 3. At first pMGB vector having a promoter and a terminator of glyceraldehyde-3-phosphate dehydrogenase (hereinafter abbreviated as GAPDH) gene derived from *Monascus purpureus* was constructed. To obtain a promoter and a terminator of GAPDH gene by PCR, primers were designed based on the nucleotide sequence (GenBank Accession No. Z68498: the deposited sequence is shown in SEQ ID NO: 18. The region of *gpdl* from position 1079 to 2346 of SEQ ID NO: 18 is assumed to encode GAPDH) of GAPDH gene, *gpdl,* of *Monascus purpureus* strain IFO4478 deposited with GenBank. PCR was performed using a DNA sequence of SEQ ID NO: 25 as a sense primer and a DNA sequence of SEQ ID NO: 26 as an antisense primer, and using the genomic DNA of *Monascus purpureus* strain IFO30873 as a template. Specifically, PCR was performed for 34 cycles, each cycle consisting of denaturation at 94°C for 1 min, annealing at 50°C for 2 min, and elongation at 72°C for 3 min. Thus, a region corresponding to positions 1 to 1076 of SEQ ID NO: 18 comprising the promoter of GAPDH gene was amplifed, and then *Xba* I site and *Eco* RI site were added to the 5' side and 3' side, respectively.

Further, PCR was performed using DNA of a nucleotide sequence of SEQ ID NO: 27 as a sense primer and DNA of a nucleotide sequence of SEQ ID NO: 28 as an antisense primer, and, as in the above amplification of the promoter section, using the genomic DNA of *Monascus purpureus* strain IFO30873 as a template. Specifically, PCR was performed for 34 cycles, each cycle consisting of denaturation at 94°C for 1 min, annealing at 50°C for 2 min, and elongation at 72°C for 3 min. Thus, a region corresponding to positions 2350 to 2456 of SEQ ID NO: 18 comprising the terminator of GAPDH gene was amplified, and then *Eco*R I site and *Hin*d III site were added to the 5' side and 3 'side, respectively. These reaction solutions were subjected to electrophoresis, so that a 1.1 kb (promoter) fragment and a 0.1 kb (terminator) fragment were recovered and purified. The promoter fragment was digested with *Xba* I and *Eco*R I, and the terminator fragment was digested with *Eco*R I and *Hin*d III. Subsequently, the digested fragments were inserted between *Xba* I-*Hin*d III sites of vector pBluescript II SK (-) (STRATAGENE), thereby constructing plasmid pMGB. Determination of the nucleotide sequence of the above promoter section in pMGB revealed that it comprised a 1181 bp-long sequence shown in SEQ ID NO: 17 that differs partially from the sequence from positions 1 to 1076 of SEQ ID NO: 18. Further, comparison of the sequences of SEQ ID NO: 17 and SEQ ID NO: 18 showed insertion to 9 positions accounting for 105 nucleotides in total, and sequence substitution at 6 positions. Specifically, 9 insertions were found at position 405 (g), position 476 (g), a region from positions 680 to 770, position 779 (c), a region from positions 842 to 848, position 944 (c), position 966 (c), position 1030 (c), and position 1046 (c); and 6 sequence substitutions were found at position 76 (a → t), position 417 to 418 (cg → gc), position 851 to 852 (tt → cc), and position 855 (c → g).

*Escherichia coli* strain JM109 was transformed with pMGB. The thus obtained *Escherichia coli* transformant JM109/pMGB comprising the promoter and terminator of GDPDH gene derived from the genus *Monascus* was deposited on May 16, 2001 at the International Patent Organism Depositary, National Institute of Advanced Industrial Science and Technology, (Central 6, 1-1-1, Higashi, Tsukuba-shi, Ibaraki) (formerly, National Institute of Bioscience and Human-Technology, 1-1-3, Higashi, Tsukuba-shi, Ibaraki, Japan) under Accession No. FERM BP-7588.

To enable constitutive expression of a human lysozyme gene under regulation of the promoter derived from *Monascus purpureus,* pMGB-HLY was constructed by inserting a human lysozyme structural gene [Gene, 43, 273-279 (1986)] prepared to have *Eco*R I site at both of its ends and added with a chicken-lysozyme signal sequence to the *Eco*R I site existing at the junction between the promoter and terminator of GAPDH gene of pMGB plasmid. Next, a fragment comprising the full length *niaD* gene was excised by *Hin*d III digestion from pND300 plasmid [Biosci. Biotech. Biochem., 59, 1795-1797, (1995)] comprising, as a selection marker gene for transformation of filamentous fungi of the genus *Monascus, niaD* gene derived from *Aspergillus oryzae*. The fragment was then inserted to *Hind* III site of pMGB-HLY, thereby constructing PMGON-HLY plasmid.

### (2) Preparation and purification of protoplast

In the same manner as in Example 6 (1) and (2), a protoplast solution of *Monascus purpureus* strain SN2-30-4 was prepared.

### (3) Transformation

20 µl of pMGON-HLY plasmid DNA prepared in (1) above was added to 200 µl of the protoplast solution prepared in (2) above, and then the solution was ice-cooled for 30 min. Subsequently, 1 ml of solution 2 was added to the solution, and then the solution was allowed to stand at room temperature for 20 min. Then, 10 ml of solution 1 was added to the solution to dilute PEG concentration. The solution was then centrifuged at 700 x G, thereby recovering a precipitate. The precipitate was suspended in 200 µl of solution 1, and then the suspension was placed on a basal plate medium containing 10 mmol/l NaNO₃ as a nitrogen source. Further, 0.5% soft agar medium (the medium had the same composition as that of the above plate medium except for agar concentration) was added and mixed on the plate for inoculation. The above plate was incubated at 30°C for 10 to 14 days, thereby obtaining transformants. As a result, it was confirmed that *niaD* gene of *Aspergillus oryzae* functions in filamentous fungi of the genus *Monascus* and can be used as DNA encoding a selection marker for transformation of filamentous fungi of the genus *Monascus*.

### (4) Production of human lysozyme and measurement of its activity

A 5 mm agar piece was inoculated into an Erlenmeyer flask containing 25 ml of YPD medium (2% glucose, 2% poly peptone, 1% yeast extract) from the plate medium on which the transformants obtained in (3) had been cultured. After static culturing at 30°C for 10 days, the cells were removed to obtain a culture supernatant. Using the supernatant as a crude enzyme solution, lysozyme activity was measured as follows. 800 µl of 50 mmol/l phosphate buffer (pH 6.4) and 5 µl of *Micrococcus lysodeikticus* suspension (20 mg/ml) were mixed in a microcell of a spectral photometer, and then 200 µl of the crude enzyme solution was added to start the enzyme reaction at 25°C. The activity was measured by measuring a decrease in absorbance at 450 nm. In this measurement, 1 unit was an enzyme level at which absorbance at 450 nm decreased by 0.001 for 1 min. In addition, protein content of lysozyme was calculated from activity measured when using 100,000 units/mg of specific activity of lysozyme [Biosci. Biotech. Biochem., 58, 1292-1296 (1994)]. As a result, the obtained transformants produced, at maximum, 20 µg/l human lysozyme. These results confirmed that DNA having the nucleotide sequence of SEQ ID NO: 17 functions as a promoter in filamentous fungi of the genus *Monascus*; and that the DNA can also be used for expression of the protein in filamentous fungi of the genus *Monascus* by inserting upstream of DNA encoding a desired protein.

### Example 12 Expression of Taka-amylase A gene derived from Aspergillus oryzae in a filamentous fungus of the genus Monascus

### (1) Construction of expression plasmid of Taka-amylase A gene

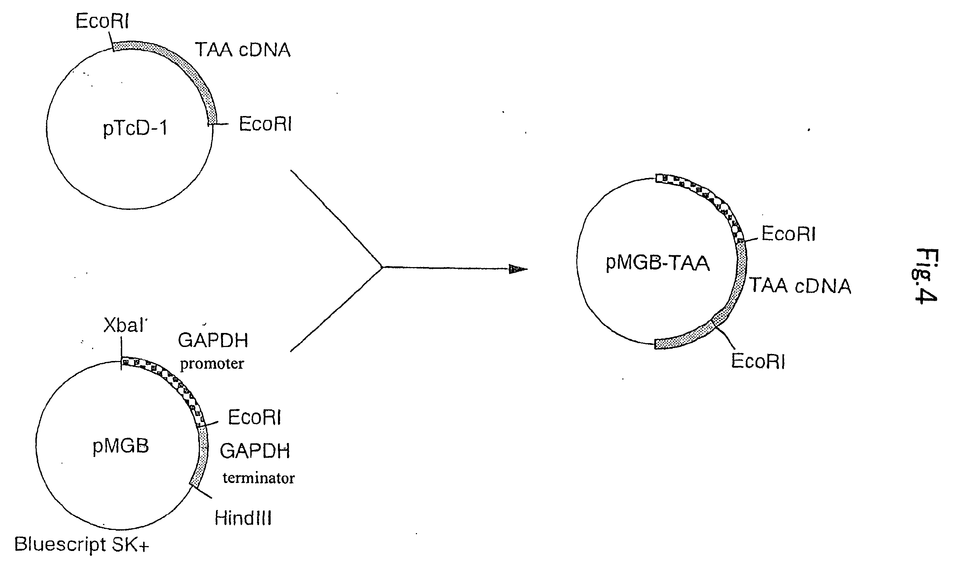

Figure 4 shows a process for constructing expression plasmid pMGB-TAA of Taka-amylase A gene.

A fragment comprising cDNA of Taka-amylase A was excised by *Eco*R I digestion from plasmid pTcD-1 [Nagashima T. et al., Biosci. Biotech. Biochem., 56, 207-210 (1992)] comprising the cDNA of Taka-amylase A of *Aspergillus oryzae*. The fragment was then inserted to *Eco*R I site existing at the junction between GAPDH promoter and the terminator of pMGB plasmid, thereby constructing pMGB-TAA.

### (2) Preparation and purification of protoplast

In the same manner as in Example 6 (1) and (2), a protoplast solution of *Monascus purpureus* strain SN2-30-4 was prepared.

### (3) Transformation

Ten µl of pMGB-TAA plasmid prepared in (1) above and 10 µl of pMA-niaD plasmid comprising the selection marker gene, *niaD*, were added simultaneously to 200 µl of the protoplast solution prepared in (2) above, and then the solution was ice-cooled for 30 min. Subsequently, 1 ml of solution 2 was added to the solution, and then the solution was allowed to stand at room temperature for 20 min. Then, 10 ml of solution 1 was added to the solution to dilute PEG concentration. The solution was then centrifuged at 700 x G, thereby recovering a precipitate. The precipitate was suspended in 200 µl of solution 1, and then the suspension was placed on a basal plate medium containing 10 mmol/l NaNO₃ as a nitrogen source. Further, 0.5% soft agar medium (the medium had the same composition as that of the above plate medium except for agar concentration) was added and mixed on the plate for inoculation. The above plate was incubated at 30°C for 10 to 14 days, thereby obtaining transformants.

### (4) Production of Taka-amylase A and measurement of its activity

A 5 mm agar piece was inoculated into an Erlenmeyer flask containing 25 ml of DPC medium (2% dextrin, 0.3% peptone, 0.1% corn steep liquor, 0.05% MgSO₄·7H₂O) from the plate medium on which the obtained transformants had been cultured. After static culturing at 30°C for 14 days, the cells were removed to obtain a culture supernatant. Amylase activity was measured using the supernatant as a crude enzyme solution in accordance with Official Methods of Analysis of National Tax Administration Agency, Japan (BREWING SOCIETY OF JAPAN, 1993), and the protein content was calculated using 2200 units/mg as specific activity [Biosci. Biotech. Biochem., 58, 1292-1296 (1994)]. As a result, the obtained transformant strain produced, at maximum, 104 mg/l Taka-amylase A.

### Example 13 Expression of phytase gene derived from Aspergillus niger in a filamentous fungus of the genus Monascus by promoter of alcohol dehydrogenase II gene

### (1) Plasmid construction for expression of- phytase gene by promoter of alcohol dehydrogenase II gene

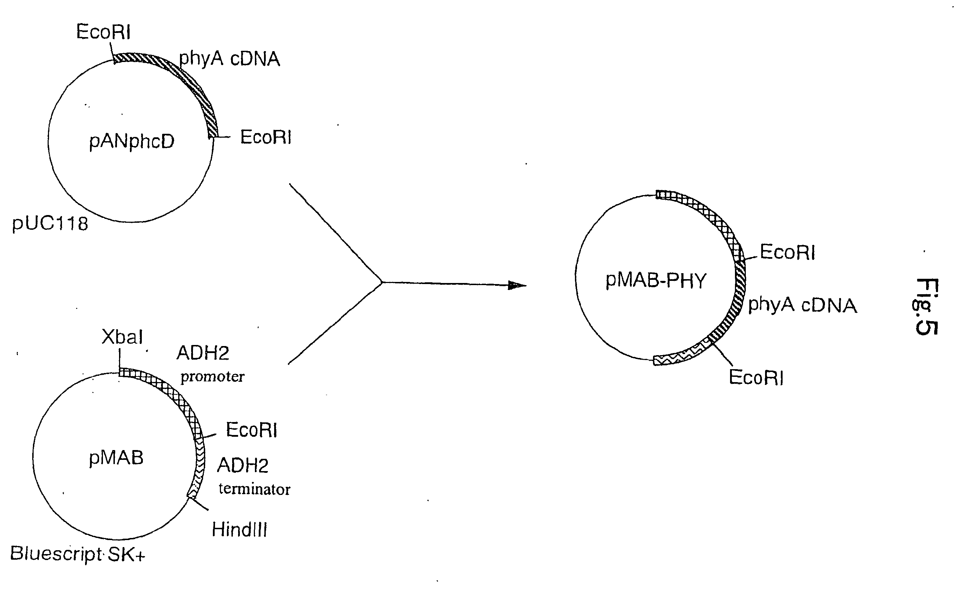

Figure 5 shows a method for constructing expression plasmid pMAB-PHY of phytase gene. At first pMAB vector having the promoter and terminator of alcohol dehydrogenase II gene, *alcB*, derived from *Monascus purpureus* was constructed. To obtain the promoter and terminator of *alcB* gene by PCR, primers were designed based on the sequence (SEQ ID NO: 9) of *alcB* gene of *Monascus purpureus.* PCR was performed using a DNA sequence of SEQ ID NO: 29 as a sense primer and a DNA sequence of SEQ ID NO: 30 as an antisense primer, and using a 8 kb DNA fragment of *alcB* gene of *Monascus purpureus* comprising the sequence of SEQ ID NO: 9 obtained in Example 8 as a template. Specifically, PCR was performed for 35 cycles, each cycle consisting of denaturation at 94°C for 1 min, annealing at 52°C for 2 min, and elongation at 72°C for 3 min. Therefore, the promoter region of *alcB* gene (a region comprising a sequence from position 1 to 611 of SEQ ID NO: 9) was amplified, and then *Bam*H I site and *Eco*R I site were added to the 5' end and 3' end, respectively.

Further, PCR was performed using a DNA sequence of SEQ ID NO: 31 as a sense primer and a DNA sequence of SEQ ID NO: 32 as an antisense primer, and similar to amplification of the above promoter region, using a 8 kb DNA fragment of *alcB* gene of *Monascus purpureus* comprising the sequence of SEQ ID NO: 9 as a template. Specifically, PCR was performed for 35 cycles, each cycle consisting of denaturation at 94°C for 1 min, annealing at 52°C for 2 min, and elongation at 72°C for 3 min. Thus, the terminator region of *alcB* gene was amplified. The reaction solution was subjected to electrophoresis, and then the 1.2 kb (promoter) and 0.3 kb (terminator) fragments were recovered and purified. The promoter fragment was digested using *EcoR* I and *Bam*H I, and the terminator fragment was digested using *Eco*R I and *Xho* I. Both the fragments were inserted between *Bam*H I-*Xho I* sites of pBluescriptSK (+) vector (STRATAGENE), thereby constructing pMAB vector.

Plasmid pANphcD prepared by subcloning cDNA of *phyA* from *Aspergillus niger* (WO 97/38096) to pUC118 (TAKARA SHUZO CO., LTD.) was digested using *EcoR* I. A fragment comprising cDNA of *phyA* was excised, and then inserted to *EcoR* I site existing at the junction between the promoter and terminator of *alcB* gene of pMAB vector, thereby constructing plasmid pMAB-PHY.

### (2) Preparation and purification of protoplast

In the same manner as in Example 6 (1) and (2), a protoplast solution of *Monascus* *purpureus* strain SN2-30-4 was prepared.

### (3) Transformation

Ten µl of pMAB-PHY plasmid prepared in (1) above and 10 µl of pMA-niaD plasmid comprising the selection marker gene, *niaD*, were added simultaneously to 200 µl of the protoplast solution prepared in (2) above, and then the solution was ice-cooled for 30 min. Subsequently, 1 ml of solution 2 was added to the solution, and then the solution was allowed to stand at room temperature for 20 min. Then, 10 ml of solution 1 was added to the solution to dilute PEG concentration. The solution was then centrifuged at 700 x G, thereby recovering a precipitate. The precipitate was suspended in 200 µl of solution 1, and then the suspension was placed on a basal plate medium containing 10 mmol/l NaNO₃ as a nitrogen source. Further, 0.5% soft agar medium (the medium had the same composition as that of the above plate medium except for agar concentration) was added and mixed on the plate for inoculation. The above plate was incubated at 30°C for 10 to 14 days, thereby obtaining transformants.

### (4) Production of phytase and activity measurement

A 5 mm agar piece was inoculated into an Erlenmeyer flask containing 25 ml of DPC medium (2% dextrin, 0.3% peptone, 0.1% corn steep liquor, 0.05% MgSO₄·7H₂O) from the plate medium on which the transformants obtained in (3) had been cultured. After static culturing at 30°C for 14 days, the cells were removed to obtain a culture supernatant. Phytase activity was measured using the supernatant as a crude enzyme solution by the method described in Example 6, and the protein content was calculated using 150 units/mg [Appl. Environ. Microbiol., 65, 4682-4684 (1999)] as the specific activity of phytase. As a result, the obtained transformants produced, at maximum, 0.64 mg/l phytase.

### Example 14 Expression of phytase gene derived from Aspergillus niger in a filamentous fungus of Monascus by promoter of GAPDH gene

### (1) Plasmid construction for expression of phytase gene by promoter of GAPDH gene

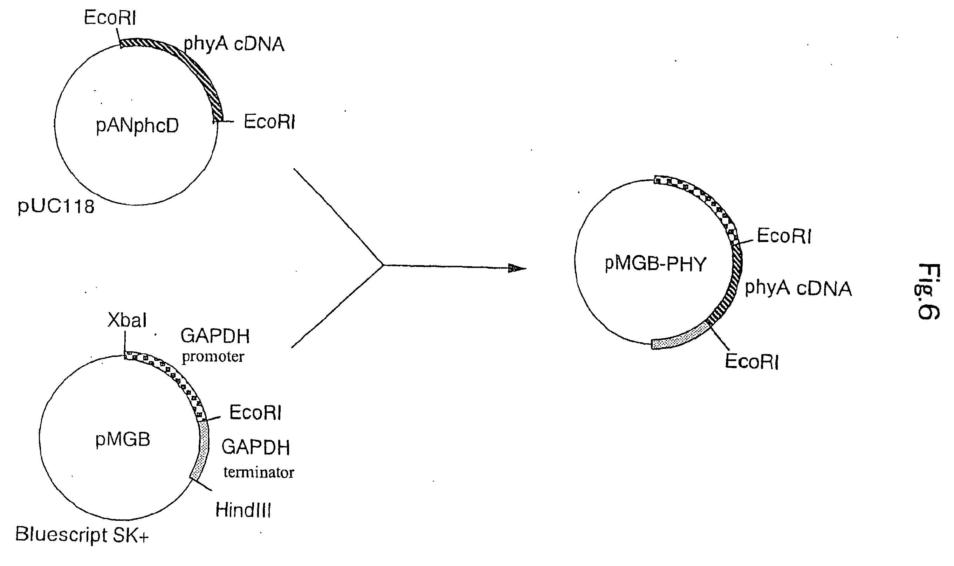

Figure 6 shows a method for constructing expression plasmid pMGB-PHY of phytase gene. Plasmid pANphcD comprising cDNA of phyA from *Aspergillus niger* was digested with *Eco*R I, and a fragment comprising the cDNA of phyA was excised. The fragment was inserted at *Eco*R I site existing at the junction between the promoter and terminator of GAPDH gene of pMGB plasmid, thereby constructing pMGB-PHY.

### (2) Preparation and purification of protoplast

In the same manner as in Example 6 (1) and (2), a protoplast solution of *Monascus purpureus* strain SN2-30-4 was prepared.

### (3) Transformation

Ten µl of pMGB-PHY plasmid prepared in (1) above and 10 µl of pMA-niaD plasmid comprising the selection marker gene, *niaD*, were added simultaneously to 200 µl of the protoplast solution prepared in (2) above, and then the solution was ice-cooled for 30 min. Next, 1 ml of solution 2 was added to the solution, and then the solution was allowed to stand at room temperature for 20 min. 10 ml of solution 1 was then added to dilute PEG concentration. The diluted solution was centrifuged at 700 x G to recover a precipitate. The precipitate was suspended in 200 µl of solution 1. The suspension was placed on a basal plate medium containing 10 mmol/l NaNO₃ as a nitrogen source, and then 0.5% soft agar medium (the medium had the same composition as that of the above plate medium except for agar concentration) was added and mixed on the plate for inoculation. The above plate was incubated at 30°C for 10 to 14 days, so as to obtain transformants.

### (4) Production of phytase and activity measurement

A 5 mm agar piece was inoculated into an Erlenmeyer flask containing 25 ml of DPC medium (2% dextrin, 0.3% peptone, 0.1% corn steep liquor, 0.05% MgSO₄·7H₂O) from the plate medium on which the transformants obtained in (3) had been cultured. After static culturing at 30°C for 14 days, the cells were removed, thereby obtaining a culture supernatant. Phytase activity was measured using the supernatant as a crude enzyme solution by the method described in Example 6. The protein content was calculated in the manner same as in Example 13. As a result, the obtained transformants produced, at maximum, 6.9 mg/l phytase.

### Example 15 Expression of phytase gene derived from Aspergillus niger in filamentous fungi of the genus Monascus by promoter of acid phosphatase gene

### (1) Plasmid construction for expression of phytase gene by promoter of acid phosphatase gene

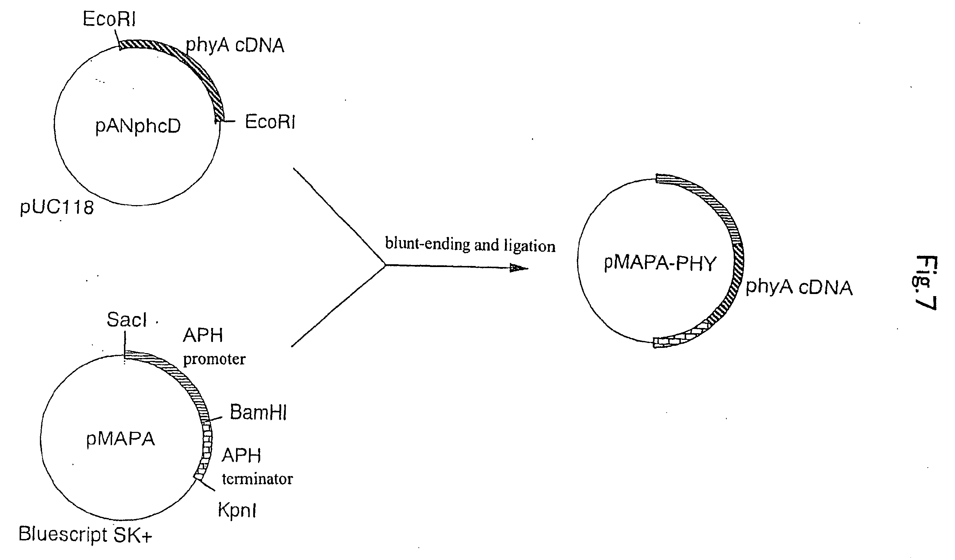

Figure 7 shows a method for constructing expression plasmid pMAPA-PHY of phytase gene. First, vector pMAPA having a promoter and terminator of acid phosphatase gene *aph* derived from *Monascus purpureus* was constructed. To obtain the promoter and terminator of *aph* gene by PCR, primers were designed based on a nucleotide sequence (a region encoding acid phosphatase is present at positions 1014 to 2732 of SEQ ID NO: 13) of *aph* gene of *Monascus purpureus.* PCR was performed using a DNA sequence of SEQ ID NO: 33 as a sense primer, and a DNA sequence of SEQ ID NO: 34 as an antisense primer, and using a 10 kb DNA fragment of *aph* gene of *Monascus purpureus* comprising the sequence of SEQ ID NO: 13 obtained in Example 10 as a template. Specifically, PCR was performed for 35 cycles, each cycle consisting of denaturation at 94°C for 1 min, annealing at 52°C for 2 min, and elongation at 72°C for 3 min. Thus, a region from positions 14 to 1013 of SEQ ID NO: 13 comprising the promoter of *aph* gene was amplified, and then *Sac* I site and *Bam*H I site were added to the 5' side and 3' side, respectively. In addition, PCR was performed using a DNA sequence of SEQ ID NO: 35 as a sense primer, and a DNA sequence of SEQ ID NO: 36 as an antisense primer, and similar to amplification of the above promoter region, using a 10 kb DNA fragment of *aph* gene of *Monascus purpureus* comprising the sequence of SEQ ID NO: 13 as a template. Specifically, PCR was performed for 35 cycles, each cycle consisting of denaturation at 94°C for 1 min, annealing at 52°C for 2 min, and elongation at 72°C for 3 min. Thus, a region from positions 2741 to 3011 of SEQ ID NO: 13 comprising the terminator of *aph* gene was amplified, and then *Bam*H I site and *Kpn* I site were added to the 5' side and 3' side, respectively. The reacted solutions were subjected to electrophoresis, and then 1.0 kb (promoter) and 0.3 kb (terminator) fragments were recovered and purified. The promoter fragment was digested using *Sac* I and *Bam*H I, and the terminator fragment was digested with *Kpn* I and *Bam*H I. The digested products were inserted between *Sac* I-*Kpn* I sites of pBluescriptSK (+) vector, thereby constructing pMAPA vector.

Next, pMAPA vector was cleaved at *Bam*H I site existing at the junction of the promoter and terminator of *aph* gene, and then blunt-ended. Then, a fragment comprising cDNA encoding phytase of *Aspergillus niger* as obtained by digesting pANphcD with *Eco*R I and allowing both ends of the digested pANphcD to be blunt-ended was inserted to the vector, thereby constructing pMAPA-PHY.

### (2) Preparation and purification of protoplast

In the same manner as in Example 6 (1) and (2), a protoplast solution of *Monascus purpureus* strain SN2-30-4 was prepared.

### (3) Transformation

Ten µl of pMAPA-PHY plasmid prepared in (1) above and 10 µl of pMA-niaD plasmid comprising the selection marker gene, *niaD*, were added simultaneously to 200 µl of the protoplast solution prepared in (2) above, and then the solution was ice-cooled for 30 min. Next, 1 ml of solution 2 was added to the solution, and then the solution was allowed to stand at room temperature for 20 min. 10 ml of solution 1 was then added to dilute PEG concentration. The diluted solution was centrifuged at 700 x G to recover a precipitate. The precipitate was suspended in 200 µl of solution 1. The suspension was placed on a basal plate medium containing 10 mmol/l NaNO₃ as a nitrogen source, and then 0.5% soft agar medium (the medium had the same composition as that of the above plate medium except for agar concentration) was added and mixed on the plate for inoculation. The above plate was incubated at 30°C for 10 to 14 days, so as to obtain transformants.

### (4) Production of phytase and measurement of its activity

A 5 mm agar piece was inoculated in an Erlenmeyer flask containing 25 ml of DPC medium (2% dextrin, 0.3% peptone, 0.1% corn steep liquor, 0.05% MgSO₄·7H₂O) from the plate medium on which the transformants obtained in (3) had been cultured. After static culturing at 30°C for 14 days, the cells were removed, thereby obtaining a culture supernatant. Phytase activity was measured using the supernatant as a crude enzyme solution by the method described in Example 6. The protein content was calculated in the same manner as in Example 13. As a result, the obtained transformants produced, at maximum, 25 mg/l phytase. These results confirmed that DNA having a nucleotide sequence from positions 14 to 1013 of SEQ ID NO: 13 functions as a promoter in filamentous fungi of the genus *Monascus*; and that the DNA can also be used for expression of the protein in filamentous fungi of the genus *Monascus* by inserting the DNA upstream of a DNA encoding a desired protein.

### Example 16 Expression of phytase gene derived from Aspergillus niger in a filamentous fungus of the genus Monascus using signal peptide of acid phosphatase

### (1) Construction of a plasmid for expressing phytase using a signal peptide of acid phosphatase of Monascus purpureus

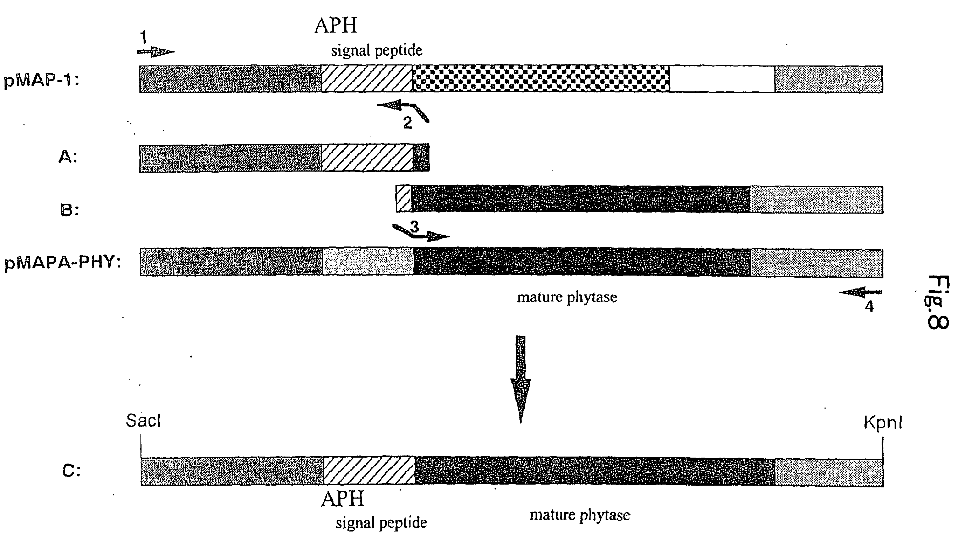

Figure 8 shows a method for constructing phytase gene having the signal peptide of acid phosphatase. First, DNA encoding the signal peptide of acid phosphatase derived from *Monascus purpureus* was amplified by PCR as described below. PCR was performed using a DNA sequence of SEQ ID NO: 37 as sense primer 1, a DNA sequence of SEQ ID NO: 38 as antisense primer 2, and a 10 kb DNA fragment of *aph* gene of *Monascus purpureus* comprising the sequence of SEQ ID NO: 13 obtained in Example 10 as a template. Specifically, PCR was performed for 34 cycles, each cycle consisting of denaturation at 94°C for 1 min, annealing at 52°C for 1 min, and elongation at 72°C for 2 min. The thus amplified DNA fragment (A) had *Sac* I site at its 5' end and comprised the promoter and signal peptide encoding region of acid phosphatase gene of *Monascus purpureus*.

Further, PCR was performed using a DNA sequence of SEQ ID NO: 39 as sense primer 3, and a DNA sequence of SEQ ID NO: 40 as antisense primer 4, and using the plasmid pMAPA-PHY obtained in Example 15 for expressing phytase gene of *Aspergillus niger* as a template. Specifically, PCR was performed for 34 cycles, each cycle consisting of denaturation at 94°C for 1 min, annealing at 52°C for 1 min, and elongation at 72°C for 2 min. The DNA fragment (B) comprising *Kpn* I site at its 3' end, and comprising a region which encodes mature polypeptide of phytase was amplified. The reaction solution was subjected to electrophoresis, and then a 1.0 kb fragment [DNA fragment (A)] and a 1.5 kb fragment [DNA fragment (B)] were recovered and purified. Using both the purified fragments, sense primer 1 and antisense primer 4, PCR was performed for 34 cycles, each cycle consisting of denaturation at 94°C for 1 min, annealing at 42°C for 1 min, and elongation at 72°C for 2 min. The DNA fragment (C) comprising *Sac* I site at its 5' end, *Kpn* I site at its 3' end, and a region that encodes mature phytase fused downstream of the promoter and signal peptide encoding region of acid phosphatase gene of *Monascus purpureus* was amplified.

The obtained fusion DNA fragment (C) was digested with *Sac* I and with *Kpn I,* and then inserted between *Sac* I-*Kpn* I sites of pUC18, thereby constructing pMAPA-aPHY.

### (2) Preparation and purification of protoplast

In the same manner as in Example 6 (1) and (2), a protoplast solution of *Monascus purpureus* strain SN2-30-4 was prepared.

### (3) Transformation

10 µl of pMAPA-aPHY plasmid prepared in (1) above and 10 µl of pMA-niaD plasmid comprising the selection marker gene, *niaD*, were added simultaneously to 200 µl of the protoplast solution prepared in (2) above, and then the solution was ice-cooled for 30 min. Next, 1 ml of solution 2 was added to the solution, and then the solution was allowed to stand at room temperature for 20 min. 10 ml of solution 1 was then added to dilute PEG concentration. The diluted solution was centrifuged at 700 x G to recover a precipitate. The precipitate was then suspended in 200 µl of solution 1. The suspension was placed on a basal plate medium containing 10 mmol/l NaNO₃ as a nitrogen source, and then 0.5% soft agar medium (the medium had the same composition as that of the above plate medium except for agar concentration) was added and mixed on the plate for inoculation. The above plate was incubated at 30°C for 10 to 14 days, so as to obtain transformants.

### (4) Production of phytase and measurement of its activity

A 5 mm agar piece was inoculated into an Erlenmeyer flask containing 25 ml of DPC medium (2% dextrin, 0.3% peptone, 0.1% corn steep liquor, 0.05% MgSO₄·7H₂O) from the plate medium on which the obtained transformants had been cultured. After static culturing at 30°C for 14 days, the cells were removed, thereby obtaining a culture supernatant. Phytase activity was measured using the supernatant as a crude enzyme solution by the method described in Example 6. The protein content was calculated in the same manner as Example 13. As a result, the obtained transformants produced, at maximum, 16 mg/l phytase. These results confirmed that the signal peptide of acid phosphatase of *Monascus purpureus* can be used for secretory production of proteins other than acid phosphatase in filamentous fungi belonging to the genus *Monascus*.

### Example 17 Expression of phytase gene derived from Aspergillus niger in a filamentous fungus of the genus Monascus using signal peptide of Taka-amylase A

### (1) Plasmid construction for expression of phytase gene using signal peptide of Taka-amylase A

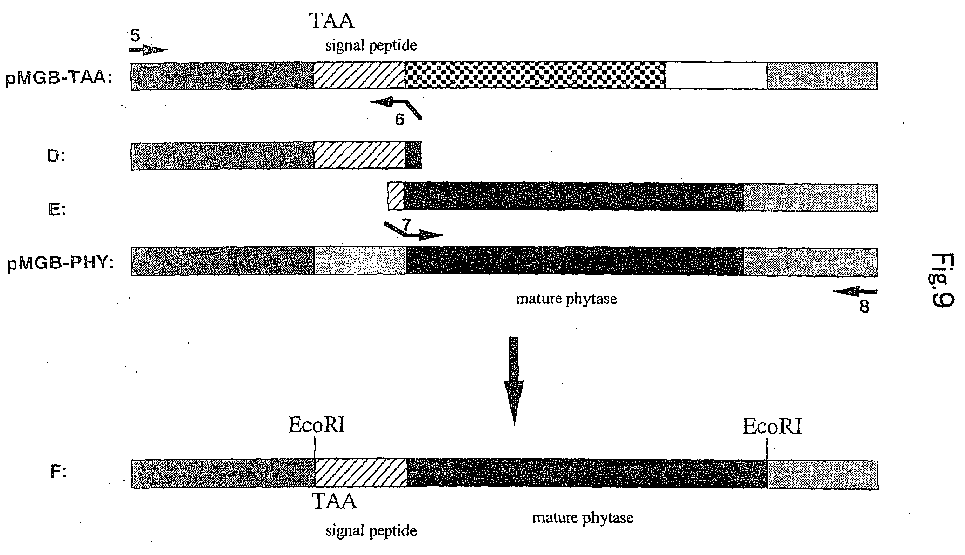

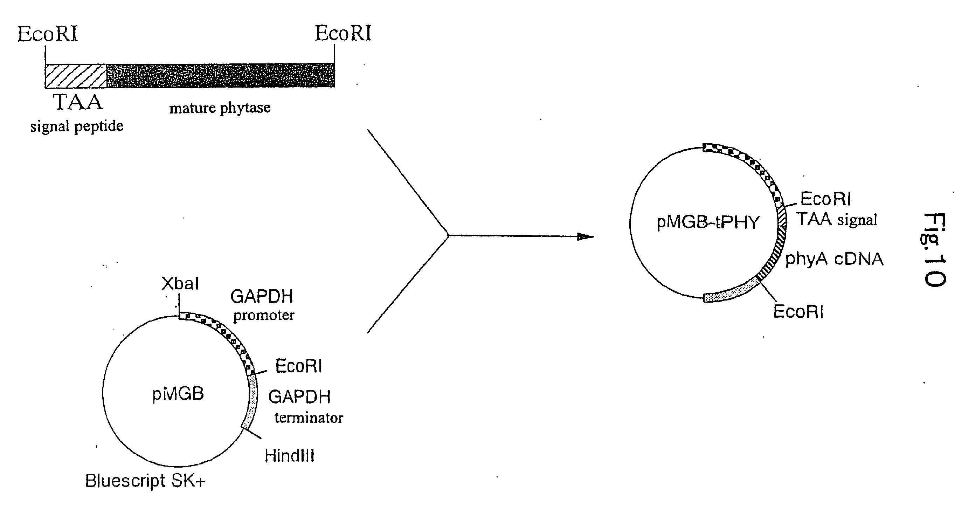

Figure 9 shows a method for constructing phytase gene having the signal peptide of Taka-amylase A, and Figure 10 shows a method for constructing pMGB-tPHY plasmid for expressing the gene. First, DNA encoding the signal peptide of Taka-amylase A derived from *Aspergillus oryzae* was amplified by PCR as follows. PCR was performed using a DNA sequence of SEQ ID NO: 41 as sense primer 5, and a sequence of SEQ ID NO: 42 as antisense primer 6, and using plasmid pMGB-TAA for expression of Taka-amylase A gene as a template. Specifically, PCR was performed for 34 cycles, each cycle consisting of denaturation at 94°C for 1 min, annealing at 48°C for 1 min, and elongation at 72°C for 2 min. The DNA fragment (D) comprising a region encoding the signal peptide of Taka-amylase A was amplified.

Further, PCR was performed using a sequence of SEQ ID NO: 43 as sense primer 7 and a sequence of SEQ ID NO: 44 as antisense primer 8, and using plasmid pMGB-PHY for expression of phytase gene as a template. Specifically, PCR was performed for 34 cycles, each cycle consisting of denaturation at 94°C for 1 min, annealing at 48°C for 1 min, and elongation at 72°C for 2 min. The DNA fragment (E) comprising a region which encodes mature polypeptide of phytase was amplified. The reaction solution was subjected to electrophoresis, and then a 1.1 kb fragment [DNA fragment (D)] and a 1.5 kb fragment [DNA fragment (E)] were collected and purified. Using both the purified fragments, sense primer 5 and antisense primer 8, PCR was performed for 34 cycles, each cycle consisting of denaturation at 94°C for 1 min, annealing at 48°C for 1 min, and elongation at 72°C for 2 min. The DNA fragment (F) comprising a region which encodes the mature polypeptide of phytase fused downstream of a region encoding the signal peptide of Taka-amylase A was amplified.

The amplified DNA fragment (F) was digested with *Eco*R I (*Eco*R I site is present at the 5' end of a region encoding the signal peptide of Taka-amylase A, and at the 3' end of a region encoding the mature polypeptide of phytase), and then inserted at *EcoR* I site existing at the junction between the promoter and terminator of GAPDH gene of pMGB plasmid, thereby constructing pMGB-tPHY.

### (2) Preparation and purification of protoplast

In the same manner as in Example 6 (1) and (2), a protoplast solution of *Monascus purpureus* strain SN2-30-4 was prepared.

### (3) Transformation

10 µl of pMGB-tPHY plasmid prepared in (1) above and 10 µl of pMA-niaD plasmid comprising the selection marker gene, *niaD*, were added simultaneously to 200 µl of the protoplast solution prepared in (2) above, and then the solution was ice-cooled for 30 min. Next, 1 ml of solution 2 was added to the solution, and then the mixture was allowed to stand at room temperature for 20 min. 10 ml of solution 1 was then added to dilute PEG concentration. The diluted solution was centrifuged at 700 x G to recover a precipitate. The precipitate was then suspended in 200 µl of solution 1. The suspension was placed on a basal plate medium containing 10 mmol/l NaNO₃ as a nitrogen source, and then 0.5% soft agar medium (the medium had the same composition as that of the above plate medium except for agar concentration) was added and mixed on the plate for inoculation. The above plate was incubated at 30°C for 10 to 14 days, so as to obtain transformants.

### (4) Production of phytase and measurement of its activity

A 5 mm agar piece was inoculated into an Erlenmeyer flask containing 25 ml of DPC medium (2% dextrin, 0.3% peptone, 0.1% corn steep liquor, 0.05% MgSO₄·7H₂O) from the plate medium on which the obtained transformants had been cultured. After static culturing at 30°C for 14 days, the cells were removed, thereby obtaining a culture supernatant. Phytase activity was measured using the supernatant as a crude enzyme solution by the method described in Example 6. The protein content was calculated in the same manner as Example 13. As a result, the obtained transformants produced, at maximum, 50 mg/l phytase. These results confirmed that the signal peptide of Taka-amylase A of *Aspergillus oryzae* can be used for secretory production of proteins other than Taka-amylase A in filamentous fungi belonging to the genus *Monascus*, and that the production amount and secretion amount are high.

Table 2 summarizes plasmids used for expression of foreign genes in Examples 6 and 12 to 17 and the production amount of heterologous proteins.

**Table 2**

| Expression Plasmid | Promoter | Foreign gene | Signal sequence | Production amount (mg/l) |
|---|---|---|---|---|
| pMGON-HLY | *M. purpureus* GAPDH | human lysozyme | chicken lysozyme | 0.02 |
| pMGB-TAA | *M. purpureus* GAPDH | *A.oryzae* Taka-amylase A | Same as left | 104 |
| pANPHY1 | *A. niger PhyA* | *A. niger* Phytase | Same as left | 0.18 |
| pMAB-PHY | *M. purpureus alcB* | *A. niger* Phytase | Same as left | 0.64 |
| pMGB-PHY | *M. purpureus* GAPDH | *A. niger* Phytase | Same as left | 6.9 |
| pMAPA-PHY | *M. purpureus aph* | *A. niger* Phytase | Same as left | 25 |
| pMAPA-aPHY | *M. purpureus aph* | *A. niger* Phytase | *M. purpureus Aph* | 16 |
| pMGB-tPHY | *M. purpureus* GAPDH | *A. niger* Phytase | *A.oryzae* Taka-amylase A | 50 50 |

### INDUSTRIAL APPLICABILITY

Using a filamentous fungus host belonging to the genus *Monascus*, red koji mold, which has been consumed as a food for a long time and is highly safe, we have developed reproducible transformation methods, including a method for introducing a gene and a method for selecting a recombinant strain, and thus established a novel efficient system for producing a useful substance. Further, we have isolated *niaD* gene and *amdS* gene derived from *Monascus purpureus* and shown their applicability as a selection marker used for transformation of filamentous fungi. We have also isolated *alcB* gene, *aph* gene and GAPDH gene having a novel promoter sequence derived from *Monascus purpureus*, and have shown the possibility of their application as a gene comprising a strong promoter and terminator to an expression system using a filamentous fungus host. These novel DNAs can be used not only for selecting a recombinant strain and expressing a recombinant DNA, but also as a gene for producing *Monascus purpureus*-derived nitrate reductase, alcohol dehydrogenase II, acetamidase and acid phosphatase.

According to the present invention, a method for expressing recombinant proteins at high levels using a filamentous fungus belonging to the genus *Monascus* as a host can be provided.

All publications, patents and patent applications cited herein are incorporated herein by reference in their entirety.

### Sequence Listing Free Text

SEQ ID NO: 19-sense primer for amplification of *Aspergillus oryzae amdS* gene
SEQ ID NO: 20-antisense primer for amplification of *Aspergillus oryzae amdS* gene
SEQ ID NO: 21-sense primer for amplification of *Aspergillus nidulans alcB* gene
SEQ ID NO: 22-antisense primer for amplification of *Aspergillus nidulans alcB* gene
SEQ ID NO: 23-sense primer for amplification *of Aspergillus niger aph* gene
SEQ ID NO: 24-antisense primer for amplification of *Aspergillus niger aph* gene
SEQ ID NO: 25-sense primer for amplification of the promoter region of *Monascus purpureus* GAPDH gene
SEQ ID NO: 26-antisense primer for amplification of the promoter region of *Monascus purpureus* GAPDH gene
SEQ ID NO: 27-sense primer for amplification of the terminator region of *Monascus purpureus* GAPDH gene
SEQ ID NO: 28-antisense primer for amplification of the terminator region of *Monascus purpureus* GAPDH gene
SEQ ID NO: 29-sense primer for amplification of the promoter region of *Monascus purpureus alcB* gene
SEQ ID NO: 30-antisense primer for amplification of the promoter region of *Monascus purpureus alcB* gene
SEQ ID NO: 31-sense primer for amplification of the terminator region of *Monascus purpureus alcB* gene
SEQ ID NO: 32-antisense primer for amplification of the terminator region of *Monascus purpureus alcB* gene
SEQ ID NO: 33-sense primer for amplification of the promoter region of *Monascus purpureus aph* gene
SEQ ID NO: 34-antisense primer for amplification of the promoter region of *Monascus purpureus aph* gene
SEQ ID NO: 35-sense primer for amplification of the terminator region of *Monascus purpureus aph* gene
SEQ ID NO: 36-antisense primer for amplification of the terminator region of *Monascus purpureus aph* gene
SEQ ID NO: 37-sense primer for amplification of the promoter region and signal sequence of *aph* gene of *Monascus purpureus*
SEQ ID NO: 38-antisense primer for amplification of the promoter region and signal sequence of *Monascus purpureus aph* gene
SEQ ID NO: 39-sense primer for amplification of DNA encoding a mature polypeptide of *Aspergillus niger* phytase
SEQ ID NO: 40-antisense primer for amplification of DNA encoding a mature polypeptide of *Aspergillus niger* phytase
SEQ ID NO: 41-sense primer for amplification of DNA encoding a signal peptide of *Aspergillus oryzae* Taka-amylase A
SEQ ID NO: 42-antisense primer for amplification of DNA encoding a signal peptide of *Aspergillus oryzae* Taka-amylase A
SEQ ID NO: 43-sense primer for amplification of DNA encoding a mature polypeptide of *Aspergillus niger* phytase
SEQ ID NO: 44-antisense primer for amplification of DNA encoding a mature polypeptide of *Aspergillus niger* phytase

## Claims

1. In a method for transforming filamentous fungi, the improvement comprising using a filamentous fungus belonging to the genus *Monascus* as a host.

2. The method according to claim 1, wherein the filamentous fungus belonging to the genus *Monascus* is *Monascus purpureus*.

3. The method according to claim 1 or 2, which comprises introducing into a host a recombinant DNA that is obtainable by incorporating into one vector a DNA encoding a marker for selecting a transformant and a DNA encoding a desired protein.

4. The method according to claim 1 or 2, which comprises introducing into a host two types of recombinant DNAs, one of which is obtainable by incorporating a DNA encoding a marker for selecting a transformant into a vector, and the other of which is obtainable by incorporating a DNA encoding a desired protein into a vector.

5. The method according to claim 3 or 4, wherein the DNA encoding a marker for selecting a transformant is selected from the group consisting of DNA encoding nitrate reductase of filamentous fungi, DNA encoding acetamidase of filamentous fungi, DNA encoding ornithine carbamyl transferase of filamentous fungi and DNA encoding orotidine -5'- phosphate decarboxylase of filamentous fungi.

6. The method according to claim 3 or 4, wherein the DNA encoding a marker for selecting a transformant is a DNA comprising a nucleotide sequence represented by any one of SEQ ID NOS: 1, 2, 3, 5 and 7.

7. The method according to claim 3 or 4, wherein the DNA encoding a marker for selecting a transformant is a DNA hybridizing to the DNA comprising the nucleotide sequence represented by SEQ ID NO: 1 or 3 under stringent conditions, and encoding a protein having activity substantially equivalent to nitrate reductase; or a DNA hybridizing to the DNA comprising the nucleotide sequence represented by SEQ ID NO: 5 or 7 under stringent conditions, and encoding a protein having activity substantially equivalent to acetamidase.

8. The method according to claim 3 or 4, wherein the recombinant DNA has a promoter which is located upstream of the DNA encoding a desired protein and is derived from a gene selected from the group consisting of an alcohol dehydrogenase gene, an acid phosphatase gene, a glyceraldehyde-3-phosphate dehydrogenase gene, a phosphoglycerate kinase gene, a glucoamylase gene, a phytase gene, a protease gene and a cellulase gene.

9. The method according to claim 3 or 4, wherein the recombinant DNA has a terminator which is located downstream of the DNA encoding a desired protein and is derived from a gene selected from the group consisting of an alcohol dehydrogenase gene, an acid phosphatase gene, a glyceraldehyde-3-phosphate dehydrogenase gene, a phosphoglycerate kinase gene, a glucoamylase gene, a phytase gene, a protease gene and a cellulase gene.

10. The method according to claim 8 or 9, wherein the alcohol dehydrogenase gene, the acid phosphatase gene or the glyceraldehyde-3-phosphate dehydrogenase gene is derived from the filamentous fungi belonging to the genus *Monascus*.

11. The method according to claim 10, wherein the alcohol dehydrogenase gene comprises the nucleotide sequence represented by SEQ ID NO: 9, the acid phosphatase gene comprises the nucleotide sequence represented by SEQ ID NO: 13, and the glyceraldehyde-3-phosphate dehydrogenase gene comprises the nucleotide sequence represented by SEQ ID NO: 17 or 18.

12. The method according to claim 8, wherein the promoter is capable of enhancing gene expression in the presence of lower alcohol.

13. The method according to claim 12, wherein the lower alcohol is ethanol or methanol.

14. The method according to claim 8, wherein the promoter comprises a DNA having a nucleotide sequence of 50 or more consecutive nucleotides between positions 1 and 615 of the nucleotide sequence of SEQ ID NO: 9.

15. The method according to claim 8, wherein the promoter comprises a DNA having a nucleotide sequence of 50 or more consecutive nucleotides between positions 1 and 1013 of the nucleotide sequence of SEQ ID NO: 13.

16. The method according to claim 8, wherein the promoter comprises a DNA having a nucleotide sequence of 50 or more consecutive nucleotides in the nucleotide sequence of SEQ ID NO: 17.

17. The method according to claim 8, wherein the promoter comprises a DNA having a nucleotide sequence of 50 or more consecutive nucleotides between positions 1 and 1078 of the nucleotide sequence of SEQ ID NO: 18.

18. The method according to claim 9, wherein the terminator comprises a DNA having a nucleotide sequence of 50 or more consecutive nucleotides between positions 1950 and 4142 of the nucleotide sequence of SEQ ID NO: 9.

19. The method according to claim 9, wherein the terminator comprises a DNA having a nucleotide sequence of 50 or more consecutive nucleotides between positions 2633 and 3831 of the nucleotide sequence of SEQ ID NO: 13.

20. The method according to claim 9, wherein the terminator comprises a DNA having a nucleotide sequence of 50 or more consecutive nucleotides between positions 2347 and 2456 of the nucleotide sequence of SEQ ID NO: 18.

21. The method according to claim 3 or 4, wherein the DNA encoding a desired protein comprises the nucleotide sequence represented by any one of SEQ ID NOS: 1, 3, 5, 7, 9, 11, 13 and 15.

22. The method according to claim 3 or 4, wherein the desired protein is selected from the group consisting of nitrate reductase, acetamidase, alcohol dehydrogenase II and acid phosphatase that are derived from filamentous fungi belonging to the genus *Monascus*, and phytase that is derived from *Aspergillus niger*.

23. The method according to claim 3 or 4, wherein the desired protein comprises an amino acid sequence represented by any one of SEQ ID NOS: 4, 8, 12 and 16.

24. The method according to claim 3 or 4, wherein the DNA encoding a desired protein is a DNA encoding a protein comprising a desired protein and a signal peptide of the secretory protein of a filamentous fungus which peptide has been added to the N-terminus of the desired protein.

25. The method according to claim 24, wherein the signal peptide of the secretory protein of the filamentous fungus is a signal peptide of phytase of *Aspergillus niger,* acid phosphatase of *Monascus purpureus,* or Taka-amylase A of *Aspergillus oryzae*.

26. A transformant of a filamentous fungus belonging to the genus *Monascus*, which is obtainable by any one of the methods according to claims 1 to 25.

27. A method for producing a protein, which comprises culturing the transformant according to claim 26, until a desired protein is produced and accumulated in a culture, and recovering the protein therefrom.

28. A DNA, which comprises a nucleotide sequence of 50 or more consecutive nucleotides between positions 1 to 615 of the nucleotide sequence of SEQ ID NO: 9.

29. A DNA, which comprises a nucleotide sequence of 50 or more consecutive nucleotides between positions 1950 and 4142 of the nucleotide sequence of SEQ ID NO: 9.

30. The DNA according to claim 28, which is capable of enhancing gene expression in the presence of lower alcohol.

31. The DNA according to claim 30, wherein the lower alcohol is ethanol or methanol.

32. A DNA, which comprises a nucleotide sequence of 50 or more consecutive nucleotides between positions 1 and 1013 of the nucleotide sequence of SEQ ID NO: 13.

33. A DNA, which comprises a nucleotide sequence of 50 or more consecutive nucleotides between positions 2633 and 3831 of the nucleotide sequence of SEQ ID NO: 13.

34. A DNA, which comprises the nucleotide sequence of SEQ ID NO: 17.

35. A recombinant DNA, which comprises as a selection marker a DNA comprising the nucleotide sequence represented by SEQ ID NO: 1 or 3 or a DNA which hybridizes under stringent conditions to a DNA comprising the nucleotide sequence represented by SEQ ID NO: 1 or 3 and encodes a protein having activity substantially equivalent to that of nitrate reductase.

36. A recombinant DNA, which comprises as a selection marker a DNA comprising the nucleotide sequence represented by SEQ ID NO: 5 or 7 or a DNA which hybridizes under stringent conditions to a DNA comprising the nucleotide sequence represented by SEQ ID NO: 5 or 7 and encodes a protein having activity substantially equivalent to that of acetamidase.

37. A recombinant DNA, which comprises as a promoter the DNA according to claim 28, 32 or 34.

38. A recombinant DNA, which comprises as a terminator the DNA according to claim 29 or 33.

39. A protein, which comprises an amino acid sequence represented by any one of SEQ ID NOS: 4, 8, 12 and 16.

40. A protein, which comprises an amino acid sequence wherein one or more amino acid residues are deleted, substituted and/or added in the amino acid sequence represented by any one of SEQ ID NOS: 4, 8, 12 and 16, and has activity equivalent to that of the protein comprising the amino acid sequence represented by any one of SEQ ID NOS: 4, 8, 12 and 16.

41. A DNA, which encodes the protein according to claim 39 or 40.

42. The DNA according to claim 41, which comprises a nucleotide sequence represented by any one of SEQ ID NOS: 1, 3, 5, 7, 9, 11, 13 and 15.

43. A DNA, which hybridizes to the DNA according to claim 42 under stringent conditions, and encodes a protein having activity substantially equivalent to that of a protein comprising an amino acid sequence represented by any one of SEQ ID NOS: 4, 8, 12 and 16.

44. An oligonucleotide, which comprises a nucleotide sequence that is identical to that of 15 to 60 consecutive nucleotides in a nucleotide sequence represented by any one of SEQ ID NOS: 1, 3, 5, 7, 9, 11, 13 and 15, or a nucleotide sequence that is complementary to that of the oligonucleotide.


SEQUENCE LISTING

<110> KYOWA HAKKO KOGYO CO., LTD
Shin Nihon Chemical Co., Ltd

<120> Host-Vector System of fungi of genera Monascus

<130> PH-1245-PCT

<150> JP 2000-195142
<151> 2000-06-28

<160> 44

<170> PatentIn Ver. 2.1

<210> 1
<211> 4527
<212> DNA
<213> Monascus purpureus

<220>
<221> exon
<222> (1222)..(1498)

<220>

<221> exon
<222> (1574)..(1673)

<220>
<221> exon
<222> (1759)..(2017)

<220>
<221> exon
<222> (2072)..(2307)

<220>
<221> exon
<222> (2372)..(2595)

<220>
<221> exon
<222> (2658)..(3281)

<220>
<221> exon
<222> (3338)..(4239)

<400> 1
gggacagacg tttacctaca tgaacgaaat ggccaccatt cccagcccaa ctaccacgat 60

cctttggcgt ctgccgcctt cctggctctg ctcgctgcca ttgtcgcggc tgacgtgact 120

gtctgaatcg ctggattccc cgattctatc taagccattg ggagtattgc cagcgttgcc 180

atggacaggc ctaccgtccg tctgctcatc cagtggcatt gctgcaaagt gaaaacttgg 240

gtgaggattt cgggtgaata tttaggtagg taggttccta caaagagcat ccagggagct 300

ccctcgctaa taaacggagt atagcctgct accgagtaga ctacagaaac gccgttggcc 360

aggcacaaga tctctttgag aaccagcgga ccacgcaatt gcgcagccag gggaaccagg 420

ctgatgattc gcctgaagaa tacggttgac attctgttct gggcatccag ccttccttgc 480

catgatttgc atctccgcgg ggagttggca tgaagataat tacgcataat gaaggcgcaa 540

gcaaatcaat acgacacgtc ttcctcggag atccgacttg gaatcatgtt agtgatgttg 600

tttgcttatc agaccacatc gtgcagcagc gctttgccgg ctttcatcgc actgtttagg 660

tttatttcat gccgtaaagg cgaaacctgg cgatgctcct gaagccgttg tctcgccaga 720

agagtatctg ctatgctgta cggagggatt tgctgtcaaa tcccggctgt catatcccgg 780

tgccgatact gttgattcaa gccttctctt tgcacttcgt ttctgtcggg ttcaaccgca 840

tcgataatac ggagaaatat tagccattaa ccaggggtcg ccttatcgct gtgcagaata 900

ctctattctc tgagcattcc ccgcggagat agtcaatgtg agtacggagt atatcacctc 960

caagacgact gtggagacag atgtttcccc aaaaatgatc atgatattgt tcaattttca 1020

ttacgggatg cttccgtttt tcacatcaaa tgttatgaaa tcatttttgt tccatttttc 1080

ggcattggaa ctacaattag tcaatatact tgatccctgc acatacacgg acatcctact 1140

ctcagctaca tggatctact agtttcgtga agagcctcca tcgctttaac agagtactgt 1200

tctaattgcc tctgtaaaac catgggctcc ctcactcaag tccaaacgga accggtcacc 1260

accgcaaccc attccatcgg gtcagttcag ttgaaggtcg aagaagactc ctcgatgtct 1320

tctcctgatc tagcggaaat aatcccgctc cctccgccat cgagagaacc ctctgaggtt 1380

ttaaatatag acaagaacac tcccgacgga cacgttcctc gcgatcctcg actactgaga 1440

cttacgggtg cgcatccgtt caattgtgag ccaccgctta cggagctcta cagccaaggt 1500

agttatatct ttgagccttg ttgtgtttcc cctccgtaga caaggaaacg acgtctgatg 1560

taccagggaa aaggttttct cacaccaccc gaattgcatt ttgtcagaaa tcacggaccc 1620

gtcccccatg ttcaggatga tgatattccg gattgggaat taagcatcga agggttagtt 1680

gaccttgatg acatgtgtat ttgtttcttt agccttttga aaaatgaagg gggaaaaaat 1740

ctgctaatcc tcccgcagtc ttgtggagag acccgttgtc tggacattcc aacagatttt 1800

agacgaattt gagcaaataa cggcccctgt cactctcgta tgtgctggta atcgccgaaa 1860

ggaacaaaat caagtgcgga aaacgaaagg atttcctgg ggttctgcag gcatttctac 1920

gtcgctctac acaggaccct tgatgggaga catcctacga cgagcaaagc ctcttcgtcg 1980

agctaaatat gtatgcatgg agggggctga tgtgttagta tgttatcatt cttaagaagg 2040

aggaggacag aagaaaaact gactctcata gcctaatgga cactatggca catcgatcaa 2100

gctcaactgg gccctggatt tcaatcgagg tatcatgctc gcccacaaaa tgaacgggga 2160

acctctccgt ccagatcacg gtcgtccttt gcgcgttgtt gttcccggcc aaataggcgg 2220

acgcagtgtc aaatggctga agagactgat cctcacagac tccccgagta cgaattggta 2280

tcatatcaat gacaaccgat tgctaccgtg agttcttcca cttcagatat aatttgtttg 2340

cgggaacctg acaatatcgc caaatgtgaa gaacgatggt ctccccagag atggcatccg 2400

aagaccccaa gtggtggcga gatgaccgat atgccatctt cgacctaaat gtcaattctt 2460

ccgttgtata tccagaaaac aacgaggagc ttgtgatagc ttcagcccct tcgacatata 2520

ccgttaaagg atatgcttac tccggtggtg gccgacggat tacaagagtt gagatttcct 2580

tggataaggg aagatgtatg tacaagtgca tacgtatccc gaaaagtgaa tttctgccta 2640

actaattatc aatttagcat ggcaccttgc gcacattgat tacgccgaag acaagtatcg 2700

caactttgaa ggcgaccttt ttggcgggaa agtagacatg tactggcggg aaacttgctt 2760

ctgctggtct ttctggtccc ttgacattcc agtgtcggat ctacaggcta gtgatgccat 2820

tttggtgcgg gcaatggacg agtctttggc tgttcaaccc cgtgatatgt attggtctgt 2880

ccttggaatg atgaacaatc cgtggttccg cgtcaccatc acaaatgaaa acggaagatt 2940

gaaatttgag catccgacac acccaactaa gactggtggc tggatggaac gggtcaagaa 3000

agccggagga gatctggcga acggttactg gggagagaca gttcaagggg aagcaccggc 3060

ccagcaggag tctgcgaaag agataaatat gagaagggaa gggctgagta ggctgatcga 3120

actacaagag ctcaaggatc atgttagcaa tggagaacct tggtttatag tcaacggtga 3180

ggtatatgac ggcactgaat ttttaaggga tcacccagga ggcgctcaga gcatcatttc 3240

ttctgccgga atggacgttt cggaggagtt cctcgccatt cgtaagtcgt ctgaatggaa 3300

accggcaagt tatcacaaga ctaatattcg attatagaca gtgaaactgc aaggattatg 3360

atgccgggtt atcacatcgg aacattaagc acatcagccc tggctgttct tcaagataat 3420

ggcctggagg aacagaacaa ctcaactgaa cctcgcaaga catttctcca gtctcgatac 3480

tggtccaaaa caacactggt acggaagaag attgtatcct cggatagtcg gatttttacg 3540

ttcgagcttg aacatccaaa acagaccctg ggtctaccag tcggccggca tcttatgatc 3600

agagtcccag acccaaccaa gaagaacgag tgtatcatca gatcttatac tcctatttct 3660

ggtattacac aggagggaac catggatatc ctagtcaaag tttactttga tactgctacc 3720

caaccaggcg gcaaaatgac aacggccctt gatagacttc ccttgggctc cacgatcgat 3780

tgcaaaggtc caactggcag gtttgagtac ctcggcaatg gcaacatcct aataggtgac 3840

caggagcgtc atgtcaagtc cttccggatg atttgtggag gtagtggggt cacaccaatc 3900

ttccaggttc tgcgtgccgt gatgcaagat ccggacgatc caacgacctg cgtggtgctt 3960

aatggaaaca gacgcgagga ggacattctt tgtcgggctg agctagacgc tcttgtcgct 4020

ctcaacaacg caaaatgcac catgattcac actttgacca aggcgcctga gacatgggct 4080

ggccatcgtg gccgtatctc cgagacgctg ctgaaggaat atgctatgct caatgacgac 4140

tgtatggtgc tggtttgtgg tccagagagc atggagcgcg atgttcaaaa gatactactt 4200

ggtcttggat gggaagagtc gaatctacat ttcttctagg atctttattt accttcagat 4260

accatggatg ggttagacga catgagcccg aagagcatgc acgtatgttg gatagagcgg 4320

tatatactcc ggactacaac tctaatatct gcatagaaat acatgaatga gagagccagg 4380

atggtcaatg tagttcattg atggcaatga tggaatgacc ctaataatcc caatcggaag 4440

tcatttaggg catgtattac acgtgatcag agccaatcag ggtcctgaga tctctatctt 4500

cagttgcaag tttaagttcc tcactga 4527

<210> 2
<211> 5140
<212> DNA
<213> Aspergillus oryzae

<220>
<221> exon
<222> (1200)..(1470)

<220>
<221> exon
<222> (1569)..(1668)

<220>
<221> exon
<222> (1719)..(1977)

<220>
<221> exon

<222> (2026)..(2261)

<220>

<221> exon

<222> (2320)..(2543)

<220>

<221> exon

<222> (2603)..(3226)

<220>

<221> exon

<222> (3283)..(4175)

<400> 2

aagcttaaca ggccccaaat tcaattaatt gcacctgtta ttattagtct tactacaagt 60

ttgcatatcg gcatctcaat aaaaacccgc atactacgaa agggtctatt acatcacgag 120

ctcttcgggg tatggtgtgg gtaccatccc tgttcctctt aagaatagat gaaagggaag 180

gtcatctcat tccacccatg agacctacaa ttaagcactg tattggtatg tgaacgccag 240

tctggtaaat cgcgccccct tgttgcctca ggtttcacca caggtcataa atattgtacg 300

taagacaaat cctaagttag atgccgatat atccggcact cttcaagcat catagcaatg 360

gcgttttaaa tcacgcagtt aggttggtgt tcttctcatg tggtaaatcc tcaagggtgt 420

aactacaagt atacggtaga cttccaagat tggcaaaaaa agccagatcg agcgattttt 480

gcctggattt aaaggatcct ggagtgccaa ttaaacgtga gcaatatccc tctaacaaac 540

ttaccgagaa ttcctttgaa gggtacgtac agagtagtag ctccgctcta acagccgtga 600

gctcccatct ggcccattct ccacgcgaac caggctcatt cggacgataa gcaacaacac 660

ttctaattgg aagttgcaca tggcttcacg gcattgtctc catcattctt caaagaacca 720

attaatatga cgaaaaagaa atcctgcaag gttgagcgga caaatggctg gagcctcgag 780

agtgttgtgt gggtcaacgc gatttatttg cctcaacgct tggttaagcg gtgggacgcc 840

gtccagcctg aaggcttgcc ctaatttcga gcgtcccacc tcccagaatg agctgatttt 900

gggactccgc ggaaatcgct ggtgggttcc taggagcatt gcttattgtg acctttctcc 960

gaggcgttat atggtaacaa ggagttactg ccgtgaccta atagaatgtc cactgcccgc 1020

gcatgggtaa cacgtactgc gtgccatcat acgataggca agagtatttt agtgtggtgg 1080

attgcctcct tatttggagt taatgcactg gctgaccatt cgacttattc atactcagca 1140

tccttgttca tgctccctcc ctcgtaattt tactttcatt ggccttccct gccggtaac 1199

atg gca acc atc acc gag gtg cgg acg gat gcg ctc gtc cca act gac 1247

Met Ala Thr Ile Thr Glu Val Arg Thr Asp Ala Leu Val Pro Thr Asp
1               5                   10                  15

ctc gtt ctt aag aca ggt cag atc aaa atc gaa agc gaa gag atc tcg    1295
Leu Val Leu Lys Thr Gly Gln Ile Lys Ile Glu Ser Glu Glu Ile Ser
            20                  25                  30

acg aga gac ctg tct gat atc cct ctg cca ccg cca tca aaa cgg ccg    1343
Thr Arg Asp Leu Ser Asp Ile Pro Leu Pro Pro Pro Ser Lys Arg Pro
        35                  40                  45

aca gaa gtg ctg agc gta gat aaa gga act cca gat agc cat gtt cct    1391
Thr Glu Val Leu Ser Val Asp Lys Gly Thr Pro Asp Ser His Val Pro
    50                  55                  60

cgt gac cct cgg ctt atc aga tta acg ggt gtt cat ccg ttt aat gtt    1439
Arg Asp Pro Arg Leu Ile Arg Leu Thr Gly Val His Pro Phe Asn Val
65                  70                  75                  80

gag cca cct ctc aca gat ctg tat aaa gaa g gtatgagtta taactgctcc    1490
Glu Pro Pro Leu Thr Asp Leu Tyr Lys Glu
                85                  90

actcctatcc ttatcaggtt gcttgcaccg gctgtcatgc ttgtcccctt gagccgttac 1550

attctcacac tctgaaag gg ttt tta aca tcg ccg gag ctc ttc tat gtt    1600
                    Gly Phe Leu Thr Ser Pro Glu Leu Phe Tyr Val
                                95                  100

cga aat cat ggc cca gtc cct cat atc aag gat gaa gat atc cct cac 1648
Arg Asn His Gly Pro Val Pro His Ile Lys Asp Glu Asp Ile Pro His
            105                 110                 115

tgg gaa att agc atc gaa gg gttagtatag tgctaggttc tctgccaaac 1698
Trp Glu Ile Ser Ile Glu Gly
        120

atccgttaac caaagtatag a ctg gta gag aag cct ttg gta cta aac ttc 1749
Leu Val Glu Lys Pro Leu Val Leu Asn Phe
125                 130

cga caa gtg ttg cag cag tac gac caa ata aca gcg cct atc acc ctc 1797
Arg Gln Val Leu Gln Gln Tyr Asp Gln Ile Thr Ala Pro Ile Thr Leu
135                 140                 145                 150

gta tgt gct ggc aat cga cgc aaa gag caa aac aat gta cgt aaa acg 1845
Val Cys Ala Gly Asn Arg Arg Lys Glu Gln Asn Asn Val Arg Lys Thr
                155                 160                 165

aaa ggt ttt tcc tgg gga tcg gcg gga cta tcg act gcc ctc ttc act 1893
Lys Gly Phe Ser Trp Gly Ser Ala Gly Leu Ser Thr Ala Leu Phe Thr
            170                 175                 180

ggc cca ttg ctg gcg gat att ctc cgc agt gcg aaa ccc ctg cgt aaa 1941
Gly Pro Leu Leu Ala Asp Ile Leu Arg Ser Ala Lys Pro Leu Arg Lys
        185                 190                 195

gcg aaa tac gtc tgt atg gaa gga gcg gat aag ctg gtatgctgta 1987
Ala Lys Tyr Val Cys Met Glu Gly Ala Asp Lys Leu
    200                 205                 210

cctctatctt atgatgataa ttgctaagtt cgccgcag ccc aat ggt cac tac 2040
Pro Asn Gly His Tyr
                215

ggc aca tct att aaa ttg aac tgg gcc ctg gac ccc aac agg ggg atc 2088
Gly Thr Ser Ile Lys Leu Asn Trp Ala Leu Asp Pro Asn Arg Gly Ile
                220                 225                 230

atg ctt gca cat aaa atg aac ggg gag tct ctt cgc cca gat cat ggt 2136
Met Leu Ala His Lys Met Asn Gly Glu Ser Leu Arg Pro Asp His Gly
            235                 240                 245

cgt ccg ctg agg gcc gtc gtg ccc ggt caa ata gga gga cga agt gtt 2184
Arg Pro Leu Arg Ala Val Val Pro Gly Gln Ile Gly Gly Arg Ser Val
        250                 255                 260

aag tgg ctg aag agg ctg atc ttg acc gat gca cca agc gac aac tgg 2232
Lys Trp Leu Lys Arg Leu Ile Leu Thr Asp Ala Pro Ser Asp Asn Trp
    265                 270                 275

tac cat atc aat gac aac cgc gtc tta cc gtatgtctaa agggcactta 2281
Tyr His Ile Asn Asp Asn Arg Val Leu Pro
280                 285

ttttatattt ccatcatttg tctaactccc taacccag a aca atg gtc tcg cct 2335
Thr Met Val Ser Pro
290

gag atg gca tca aat aac cga aat tgg tgg cac gat gag cgg tat gcg 2383
Glu Met Ala Ser Asn Asn Arg Asn Trp Trp His Asp Glu Arg Tyr Ala
295                 300                 305                 310

att tat gac cta aac acc aac tcc gcc gtt gca tat ccc caa aac aat 2431
Ile Tyr Asp Leu Asn Thr Asn Ser Ala Val Ala Tyr Pro Gln Asn Asn
                315                 320                 325

gag gtc tta aat ctc ctg gtc gca ggg ccg tca tat act gtc aga gga 2479
Glu Val Leu Asn Leu Leu Val Ala Gly Pro Ser Tyr Thr Val Arg Gly
            330                 335                 340

tat gca tac gcc ggt ggg ggc cgc agg gtt acc agg gta gaa ata tcc 2527
Tyr Ala Tyr Ala Gly Gly Gly Arg Arg Val Thr Arg Val Glu Ile Ser
        345                 350                 355

cta gac aaa ggc aaa t gtacgcaccc tcgctcgctc gatgtgtgag aatgcttatc 2583
Leu Asp Lys Gly Lys
    360

aaagctaacg gacttatag ct tgg aga ttg gcg gaa atc gaa tat gcc gaa 2634
Ser Trp Arg Leu Ala Glu Ile Glu Tyr Ala Glu
    365                 370

gac aag tat cgt gat ttt gaa ggc gag ctt ttt gga ggc aaa gta gat 2682
Asp Lys Tyr Arg Asp Phe Glu Gly Glu Leu Phe Gly Gly Lys Val Asp
375                 380                 385                 390

atg tac tgg cgc gaa act tgc ttc tgc tgg tgt ttt tgg tct cta agc 2730
Met Tyr Trp Arg Glu Thr Cys Phe Cys Trp Cys Phe Trp Ser Leu Ser
                395                 400                 405

atc acc atc cca gag ctt gag aac agt gat gcc atc ctt gta aga gcc 2778
Ile Thr Ile Pro Glu Leu Glu Asn Ser Asp Ala Ile Leu Val Arg Ala
            410                 415                 420

atg gac gaa gca ttg ggc gtg cag cct cgc gat atg tac tgg tcc gtt 2826
Met Asp Glu Ala Leu Gly Val Gln Pro Arg Asp Met Tyr Trp Ser Val
        425                 430                 435

ctc gga atg atg aac aac ccg tgg ttc cgg gtt aca att acg aag gaa 2874
Leu Gly Met Met Asn Asn Pro Trp Phe Arg Val Thr Ile Thr Lys Glu
    440                 445                 450

aac ggg aac ttg aga ttc gag cac cct acc cac cct agt atg cct aca 2922
Asn Gly Asn Leu Arg Phe Glu His Pro Thr His Pro Ser Met Pro Thr
455                 460                 465                 470

gga tgg atg gaa cgc gtc aaa aag gct ggg ggt gac ctg acg aat ggt 2970
Gly Trp Met Glu Arg Val Lys Lys Ala Gly Gly Asp Leu Thr Asn Gly
                475                 480                 485

aac tgg gga gaa aga cac gaa gga gag gag ccg acg gag ccg gag ccc 3018
Asn Trp Gly Glu Arg His Glu Gly Glu Glu Pro Thr Glu Pro Glu Pro
            490                 495                 500

gtg caa gac att aat atg aag aaa gac ggg cta agc cga gtg att ggt 3066
Val Gln Asp Ile Asn Met Lys Lys Asp Gly Leu Ser Arg Val Ile Gly
        505                 510                 515

ttt gaa gaa ttc aag gag aat tcc tgc gat gag aag cca tgg ttc atc 3114
Phe Glu Glu Phe Lys Glu Asn Ser Cys Asp Glu Lys Pro Trp Phe Ile
    520                 525                 530

gtg aat gga gaa gtg tat gat ggt caa gca ttt ctt gaa ggc cac cct 3162
Val Asn Gly Glu Val Tyr Asp Gly Gln Ala Phe Leu Glu Gly His Pro
535                 540                 545                 550

ggc gga gcg cag agt att atc tcc tct gct ggt ctg gat gtc tct gag 3210
Gly Gly Ala Gln Ser Ile Ile Ser Ser Ala Gly Leu Asp Val Ser Glu
                555                 560                 565

gaa ttc ctt gct att c gtgagtccca aaaatatcac actgcaattg taccatctat 3266
Glu Phe Leu Ala Ile
            570

tgacacctat ccatag at agc gag acg gca aag gcg atg atg cct gag tac 3317
His Ser Glu Thr Ala Lys Ala Met Met Pro Glu Tyr
            575                 580

cat att gga acg atg gat ccg gaa ggt tta aaa gca ctc aag gat gat 3365
His Ile Gly Thr Met Asp Pro Glu Gly Leu Lys Ala Leu Lys Asp Asp
    585                 590                 595

gca tca tcc tcc acc gat gaa att cgc cca gtg ttc ctc caa tca cgg 3413
Ala Ser Ser Ser Thr Asp Glu Ile Arg Pro Val Phe Leu Gln Ser Arg
600                 605                 610                 615

tct tgg aca aag gca aca ttg aaa gaa agg aaa gac ata tcc tgg gat 3461
Ser Trp Thr Lys Ala Thr Leu Lys Glu Arg Lys Asp Ile Ser Trp Asp
                620                 625                 630

aca cga ata ttt agt ttc aaa ttg gaa cac gaa gat caa aca ttg ggt 3509
Thr Arg Ile Phe Ser Phe Lys Leu Glu His Glu Asp Gln Thr Leu Gly
            635                 640                 645

tta cca gtc ggc cag cat ctt atg atc aaa gtc ctc gac aca tca tcc 3557
Leu Pro Val Gly Gln His Leu Met Ile Lys Val Leu Asp Thr Ser Ser
        650                 655                 660

aac aac gaa gcc atc atc cgc tca tac acc cca att tct gaa acc agc 3605
Asn Asn Glu Ala Ile Ile Arg Ser Tyr Thr Pro Ile Ser Glu Thr Ser
    665                 670                 675

cag aaa ggg acc gtg gac ttg ctg gtt aaa gta tac ttt gca aca gcc 3653
Gln Lys Gly Thr Val Asp Leu Leu Val Lys Val Tyr Phe Ala Thr Ala
680                 685                 690                 695

acc tcg gca ggc ggc aag atg acg atg gcc ctg gat agg ctg cca ttg 3701
Thr Ser Ala Gly Gly Lys Met Thr Met Ala Leu Asp Arg Leu Pro Leu
                700                 705                 710

ggc tcc gtg gtg gaa tgc aag ggt ccg aca ggc aga ttc gaa tac ctt 3749
Gly Ser Val Val Glu Cys Lys Gly Pro Thr Gly Arg Phe Glu Tyr Leu
            715                 720                 725

gga aat gga cga gtt gtc ata agt ggg aag gaa cgc cat gtt cgg tcg 3797
Gly Asn Gly Arg Val Val Ile Ser Gly Lys Glu Arg His Val Arg Ser
        730                 735                 740

ttt aag atg att tgt gga gga acc ggt atc aca ccg atc ttc cag gtc 3845
Phe Lys Met Ile Cys Gly Gly Thr Gly Ile Thr Pro Ile Phe Gln Val
    745                 750                 755

ttg cgc gcc gtg gtt cag gac cgg caa gat ccc acc tct tgt aca gtc 3893
Leu Arg Ala Val Val Gln Asp Arg Gln Asp Pro Thr Ser Cys Thr Val
760                 765                 770                 775

ctc aat gga aac aga cag gag gaa gat atc ctt tgc cgg gct gag ctc 3941
Leu Asn Gly Asn Arg Gln Glu Glu Asp Ile Leu Cys Arg Ala Glu Leu
                780                 785                 790

gac ggc ttc atg gca acc gac agc aga agg tgt aat ata ata cac acc 3989
Asp Gly Phe Met Ala Thr Asp Ser Arg Arg Cys Asn Ile Ile His Thr
            795                 800                 805

cta tcc aaa gcg ccg gac tca tgg act ggc cgc cga gga cgc ata tcc 4037
Leu Ser Lys Ala Pro Asp Ser Trp Thr Gly Arg Arg Gly Arg Ile Ser
        810                 815                 820

gaa gag ctc cta aag gag tac gcg gct cca gaa gat gag agt atg gtc 4085
Glu Glu Leu Leu Lys Glu Tyr Ala Ala Pro Glu Asp Glu Ser Met Val
    825                 830                 835

ctg att tgt ggt ccg cca gcc atg gaa gaa tcg gct cgg agg ata ctg 4133
Leu Ile Cys Gly Pro Pro Ala Met Glu Glu Ser Ala Arg Arg Ile Leu
840                 845                 850                 855

ttg gcg gaa gga tgg aaa gaa tca gac ctt cac ttc ttc taaattggga 4182
Leu Ala Glu Gly Trp Lys Glu Ser Asp Leu His Phe Phe
                860                 865

ttatccaagg gaatgactta atgagtatgt aagacatggg tcataacggc gttcgaaaca 4242

tatacagggt tatgtttggg aatagcacac gaataataac gttaataggt accaaagtcc 4302

ttgatacatt agcacggtag aaaaagaata atacaacgag ctgggaatat tctttaatat 4362

aaaactccaa gaagagctgg tgcggtggag cttgttttcg actctcagta atatttcctc 4422

atatccaagc gcgctaggag gtggtcgaat acacatgtag gcgcttctct ggatgcaaaa 4482

gtcgtgccgg acctgccgaa agactttgaa gatgcgttca cgccatctaa gttgcgtaga 4542

taattcacaa aaagggatgt ttgtttccgg aatgtagcaa agagctgata ggcaatagcc 4602

tcagtttcgt ggcgcacgcc gctcgttcca tccatcctcg acaatggagc aaatgtcaaa 4662

atcgtaccga aaatactttc cagcagcttc gctgcatcag catgtctttt gctgagaaag 4722

agcgcaaaaa gcatttgatc gagaatatct tcatgataat ctctaagtct agggacagaa 4782

tgtgctgctt ctatcgtgcc atcaatatca ccgcggtcga ggcagcgttc aatcttagcc 4842

aggctatctt ggaaccgctg ccaagtcgag ccaatgccga catgaaagca ataatcactc 4902

aatgagagca cgaaatgctg gcagtcaatg cgaaatttct ggtacacgtt tcgagggtgc 4962

ccagataggg agtctctccc cgtagaatca cgaatgagac ctttgacgac cgaaaccatt 5022

cgaaggagtc gaagcagatg cttgaaaaga cgatcatact tgttaagcga tcgcgacgta 5082

atgatagctt ccaggacgtc tgatggtttg tattgaagac gtaggaaatc caaagctt 5140

<210> 3

<211> 2622

<212> DNA

<213> Monascus purpureus

<400> 3

atgggctccc tcactcaagt ccaaacggaa ccggtcacca ccgcaaccca ttccatcggg 60

tcagttcagt tgaaggtcga agaagactcc tcgatgtctt ctcctgatct agcggaaata 120

atcccgctcc ctccgccatc gagagaaccc tctgaggttt taaatataga caagaacact 180

cccgacggac acgttcctcg cgatcctcga ctactgagac ttacgggtgc gcatccgttc 240

aattgtgagc caccgcttac ggagctctac agccaaggtt ttctcacacc acccgaattg 300

cattttgtca gaaatcacgg acccgtcccc catgttcagg atgatgatat tccggattgg 360

gaattaagca tcgaaggtct tgtggagaga cccgttgtct ggacattcca acagatttta 420

gacgaatttg agcaaataac ggcccctgtc actctcgtat gtgctggtaa tcgccgaaag 480

gaacaaaatc aagtgcggaa aacgaaagga ttttcctggg gttctgcagg catttctacg 540

tcgctctaca caggaccctt gatgggagac atcctacgac gagcaaagcc tcttcgtcga 600

gctaaatatg tatgcatgga gggggctgat gtgttaccta atggacacta tggcacatcg 660

atcaagctca actgggccct ggatttcaat cgaggtatca tgctcgccca caaaatgaac 720

ggggaacctc tccgtccaga tcacggtcgt cctttgcgcg ttgttgttcc cggccaaata 780

ggcggacgca gtgtcaaatg gctgaagaga ctgatcctca cagactcccc gagtacgaat 840

tggtatcata tcaatgacaa ccgattgcta ccaacgatgg tctccccaga gatggcatcc 900

gaagacccca agtggtggcg agatgaccga tatgccatct tcgacctaaa tgtcaattct 960

tccgttgtat atccagaaaa caacgaggag cttgtgatag cttcagcccc ttcgacatat 1020

accgttaaag gatatgctta ctccggtggt ggccgacgga ttacaagagt tgagatttcc 1080

ttggataagg gaagatcatg gcaccttgcg cacattgatt acgccgaaga caagtatcgc 1140

aactttgaag gcgacctttt tggcgggaaa gtagacatgt actggcggga aacttgcttc 1200

tgctggtctt tctggtccct tgacattcca gtgtcggatc tacaggctag tgatgccatt 1260

ttggtgcggg caatggacga gtctttggct gttcaacccc gtgatatgta ttggtctgtc 1320

cttggaatga tgaacaatcc gtggttccgc gtcaccatca caaatgaaaa cggaagattg 1380

aaatttgagc atccgacaca cccaactaag actggtggct ggatggaacg ggtcaagaaa 1440

gccggaggag atctggcgaa cggttactgg ggagagacag ttcaagggga agcaccggcc 1500

cagcaggagt ctgcgaaaga gataaatatg agaagggaag ggctgagtag gctgatcgaa 1560

ctacaagagc tcaaggatca tgttagcaat ggagaacctt ggtttatagt caacggtgag 1620

gtatatgacg gcactgaatt tttaagggat cacccaggag gcgctcagag catcatttct 1680

tctgccggaa tggacgtttc ggaggagttc ctcgccattc acagtgaaac tgcaaggatt 1740

atgatgccgg gttatcacat cggaacatta agcacatcag ccctggctgt tcttcaagat 1800

aatggcctgg aggaacagaa caactcaact gaacctcgca agacatttct ccagtctcga 1860

tactggtcca aaacaacact ggtacggaag aagattgtat cctcggatag tcggattttt 1920

acgttcgagc ttgaacatcc aaaacagacc ctgggtctac cagtcggccg gcatcttatg 1980

atcagagtcc cagacccaac caagaagaac gagtgtatca tcagatctta tactcctatt 2040

tctggtatta cacaggaggg aaccatggat atcctagtca aagtttactt tgatactgct 2100

acccaaccag gcggcaaaat gacaacggcc cttgatagac ttcccttggg ctccacgatc 2160

gattgcaaag gtccaactgg caggtttgag tacctcggca atggcaacat cctaataggt 2220

gaccaggagc gtcatgtcaa gtccttccgg atgatttgtg gaggtagtgg ggtcacacca 2280

atcttccagg ttctgcgtgc cgtgatgcaa gatccggacg atccaacgac ctgcgtggtg 2340

cttaatggaa acagacgcga ggaggacatt ctttgtcggg ctgagctaga cgctcttgtc 2400

gctctcaaca acgcaaaatg caccatgatt cacactttga ccaaggcgcc tgagacatgg 2460

gctggccatc gtggccgtat ctccgagacg ctgctgaagg aatatgctat gctcaatgac 2520

gactgtatgg tgctggtttg tggtccagag agcatggagc gcgatgttca aaagatacta 2580

cttggtcttg gatgggaaga gtcgaatcta catttcttct ag 2622

<210> 4

<211> 873

<212> PRT

<213> Monascus purpureus

<400> 4

Met Gly Ser Leu Thr Gln Val Gln Thr Glu Pro Val Thr Thr Ala Thr
1               5                   10                  15

His Ser Ile Gly Ser Val Gln Leu Lys Val Glu Glu Asp Ser Ser Met
            20                  25                  30

Ser Ser Pro Asp Leu Ala Glu Ile Ile Pro Leu Pro Pro Pro Ser Arg
        35                  40                  45

Glu Pro Ser Glu Val Leu Asn Ile Asp Lys Asn Thr Pro Asp Gly His
    50                  55                  60

Val Pro Arg Asp Pro Arg Leu Leu Arg Leu Thr Gly Ala His Pro Phe
65                  70                  75                  80

Asn Cys Glu Pro Pro Leu Thr Glu Leu Tyr Ser Gln Gly Phe Leu Thr
                85                  90                  95

Pro Pro Glu Leu His Phe Val Arg Asn His Gly Pro Val Pro His Val
            100                 105                 110

Gln Asp Asp Asp Ile Pro Asp Trp Glu Leu Ser Ile Glu Gly Leu Val
        115                 120                 125

Glu Arg Pro Val Val Trp Thr Phe Gln Gln Ile Leu Asp Glu Phe Glu
    130                 135                 140

Gln Ile Thr Ala Pro Val Thr Leu Val Cys Ala Gly Asn Arg Arg Lys
145                 150                 155                 160

Glu Gln Asn Gln Val Arg Lys Thr Lys Gly Phe Ser Trp Gly Ser Ala
                165                 170                 175

Gly Ile Ser Thr Ser Leu Tyr Thr Gly Pro Leu Met Gly Asp Ile Leu
            180                 185                 190

Arg Arg Ala Lys Pro Leu Arg Arg Ala Lys Tyr Val Cys Met Glu Gly
        195                 200                 205

Ala Asp Val Leu Pro Asn Gly His Tyr Gly Thr Ser Ile Lys Leu Asn
    210                 215                 220

Trp Ala Leu Asp Phe Asn Arg Gly Ile Met Leu Ala His Lys Met Asn
225                 230                 235                 240

Gly Glu Pro Leu Arg Pro Asp His Gly Arg Pro Leu Arg Val Val Val
                245                 250                 255
Pro Gly Gln Ile Gly Gly Arg Ser Val Lys Trp Leu Lys Arg Leu Ile
            260                 265                 270
Leu Thr Asp Ser Pro Ser Thr Asn Trp Tyr His Ile Asn Asp Asn Arg
        275                 280                 285
Leu Leu Pro Thr Met Val Ser Pro Glu Met Ala Ser Glu Asp Pro Lys
    290                 295                 300
Trp Trp Arg Asp Asp Arg Tyr Ala Ile Phe Asp Leu Asn Val Asn Ser
305                 310                 315                 320
Ser Val Val Tyr Pro Glu Asn Asn Glu Glu Leu Val Ile Ala Ser Ala
                325                 330                 335
Pro Ser Thr Tyr Thr Val Lys Gly Tyr Ala Tyr Ser Gly Gly Gly Arg
            340                 345                 350
Arg Ile Thr Arg Val Glu Ile Ser Leu Asp Lys Gly Arg Ser Trp His
        355                 360                 365
Leu Ala His Ile Asp Tyr Ala Glu Asp Lys Tyr Arg Asn Phe Glu Gly
    370                 375                 380
Asp Leu Phe Gly Gly Lys Val Asp Met Tyr Trp Arg Glu Thr Cys Phe

385                 390                 395                 400

Cys Trp Ser Phe Trp Ser Leu Asp Ile Pro Val Ser Asp Leu Gln Ala

                405                 410                 415

Ser Asp Ala Ile Leu Val Arg Ala Met Asp Glu Ser Leu Ala Val Gln

            420                 425                 430

Pro Arg Asp Met Tyr Trp Ser Val Leu Gly Met Met Asn Asn Pro Trp

        435                 440                 445

Phe Arg Val Thr Ile Thr Asn Glu Asn Gly Arg Leu Lys Phe Glu His

    450                 455                 460

Pro Thr His Pro Thr Lys Thr Gly Gly Trp Met Glu Arg Val Lys Lys

465                 470                 475                 480

Ala Gly Gly Asp Leu Ala Asn Gly Tyr Trp Gly Glu Thr Val Gln Gly

                485                 490                 495

Glu Ala Pro Ala Gln Gln Glu Ser Ala Lys Glu Ile Asn Met Arg Arg

            500                 505                 510

Glu Gly Leu Ser Arg Leu Ile Glu Leu Gln Glu Leu Lys Asp His Val

        515                 520                 525

Ser Asn Gly Glu Pro Trp Phe Ile Val Asn Gly Glu Val Tyr Asp Gly

    530                 535                 540

Thr Glu Phe Leu Arg Asp His Pro Gly Gly Ala Gln Ser Ile Ile Ser
545                 550                 555                 560

Ser Ala Gly Met Asp Val Ser Glu Glu Phe Leu Ala Ile His Ser Glu
                565                 570                 575

Thr Ala Arg Ile Met Met Pro Gly Tyr His Ile Gly Thr Leu Ser Thr
            580                 585                 590

Ser Ala Leu Ala Val Leu Gln Asp Asn Gly Leu Glu Glu Gln Asn Asn
        595                 600                 605

Ser Thr Glu Pro Arg Lys Thr Phe Leu Gln Ser Arg Tyr Trp Ser Lys
    610                 615                 620

Thr Thr Leu Val Arg Lys Lys Ile Val Ser Ser Asp Ser Arg Ile Phe
625                 630                 635                 640

Thr Phe Glu Leu Glu His Pro Lys Gln Thr Leu Gly Leu Pro Val Gly
                645                 650                 655

Arg His Leu Met Ile Arg Val Pro Asp Pro Thr Lys Lys Asn Glu Cys
            660                 665                 670

Ile Ile Arg Ser Tyr Thr Pro Ile Ser Gly Ile Thr Gln Glu Gly Thr
        675                 680                 685

Met Asp Ile Leu Val Lys Val Tyr Phe Asp Thr Ala Thr Gln Pro Gly
    690                 695                 700
Gly Lys Met Thr Thr Ala Leu Asp Arg Leu Pro Leu Gly Ser Thr Ile
705                 710                 715                 720
Asp Cys Lys Gly Pro Thr Gly Arg Phe Glu Tyr Leu Gly Asn Gly Asn
                725                 730                 735
Ile Leu Ile Gly Asp Gln Glu Arg His Val Lys Ser Phe Arg Met Ile
            740                 745                 750
Cys Gly Gly Ser Gly Val Thr Pro Ile Phe Gln Val Leu Arg Ala Val
        755                 760                 765
Met Gln Asp Pro Asp Asp Pro Thr Thr Cys Val Val Leu Asn Gly Asn
    770                 775                 780
Arg Arg Glu Glu Asp Ile Leu Cys Arg Ala Glu Leu Asp Ala Leu Val
785                 790                 795                 800
Ala Leu Asn Asn Ala Lys Cys Thr Met Ile His Thr Leu Thr Lys Ala
                805                 810                 815
Pro Glu Thr Trp Ala Gly His Arg Gly Arg Ile Ser Glu Thr Leu Leu
            820                 825                 830
Lys Glu Tyr Ala Met Leu Asn Asp Asp Cys Met Val Leu Val Cys Gly

835                 840                 845

Pro Glu Ser Met Glu Arg Asp Val Gln Lys Ile Leu Leu Gly Leu Gly

850                 855                 860

Trp Glu Glu Ser Asn Leu His Phe Phe

865                 870

<210> 5

<211> 2948

<212> DNA

<213> Monascus purpureus

<220>

<221> exon

<222> (414)..(677)

<220>

<221> exon

<222> (748)..(888)

<220>

<221> exon

<222> (945)..(1215)

<220>

<221> exon

<222> (1275)..(1424)

<220>
<221> exon
<222> (1473)..(1630)

<220>
<221> exon
<222> (1680)..(1829)

<220>
<221> exon
<222> (1886)..(2398)

<400> 5
ggatcccgga tatgtctaag ctgtttctct ctttttttcc ccggctccca agagggctac 60

tcttaattat agatgcatgc atcctggagt atagaccatg caatgcgtat cacagcgcta 120

ccggcgctca tcaacctcaa cgatccgacg ccctgccgag cagcaacagg agataataat 180

tattaatgcg cggacaagaa cagaagaaca tccatgacaa atgtttctga agcttatcat 240

gccgcccgat acgccccgca tggcccctct tctaacattc taccctgcag ctttaactgc 300

ccctgaaacc cgaaaagcat taacataaac gccgtgtaga cggttcaatc gacagcactt 360

ctactacagc gtacaatcat agttcggctt tctcaaacga gaatagcaca gcaatgaccg 420

ccacctggga ggaacgggct gccgataaga gacaccggat cgagcagtcg attccggcgg 480

aatggaaggt caagtccctt cccactgcag actctgtttt cgattttccg gagaagtcgg 540

ggctgttatc cgagaaggaa cgggaaatca cacagtcttc tgcgacggat ctcgtggcga 600

agctggcgaa gggagaactg aagtcggtgg atgttacttt ggcgttttgt aagagggctg 660

ctttggcgca tcaattggta ggtcttccgg ctctccctga cggaatcata tatcagcaga 720

aatggactct gctgatggac aaggcaggtc aactgtgttc ttgaattctt cccggaagcc 780

gccctggcgc aggcgaaagg gctcgatgca tattttgagg agcacaagaa gcctgttggg 840

ccgctccatg ggcttcctat ttccctaaag gaccagttgc gaatcaaggt gcgatcgtgt 900

gattatcgca ggacttggaa tggttacatt ctaacgtggt ccagggcctt gaaacatcca 960

tgggatacgt ctcctggctg ggaaaatatg aaacccgtga ttccatactc acggccctcc 1020

tgcgcaaagc cggcgcggtt ttctacgtga agacaagcgt ccctcaaact ctgatggtat 1080

gcgagacaat caacaacatc accggtcgaa ctcttaaccc gcggaacaag aattggtcgt 1140

gtggtggcag ctctggaggc gagggcgcca tggttggcat ccgtggcagt attattggag 1200

ttggcactga tattggtcag aatgctacca tttcgcaacg tcagccttgc agctgactgt 1260

gatttctgtt ctaggcggct ctattcgggt tccctctgcg ttcaatttcc tctacgggat 1320

tcgaccgagc catggtagga tgccgtatgg ctacatggcg aacagtatgg agggacagga 1380

gaccgtccat agcgttgttg gtcccattgc gcattctgct tcgggtaagc tgggtagcct 1440

tattcgggct tgtattctct aacagattct agatctgaga cttttcttga cgtcggttct 1500

cagcgaggaa ccatggaagt atgactcgaa agtagttccg ctaccgtgga ggtctcatga 1560

agaagaggcc attcgcacta aactgcagtc cgggggtctt acattggggt tctttaattg 1620

cgacggaaac gtaagatagc atgaagtact ggcaaacatc ccttttaaca atcctacagg 1680

ttcttcccca tccaccagtt ctacgaggcc ttgatacggt tgtctcggtg ctgaagaaga 1740

atgggcacag cattgttccc tggacaccat acaaacatga ctttgccgtt gacctgatct 1800

acggaattta ttctgctgat ggaagcacgg tatgttattt ttatcttgtt tagtggaata 1860

ccggagactg ataggtgcta ctcaggacgt actacgagac atcaacgcct ctggagagcc 1920

cgctattcca aacatcaaag atctcctcaa cccgagcgtc aggaaagccg acctaaacga 1980

tgtctggaat gtgcaactcc agaaatggaa gtatcagtgc gagtatctcg acaaatggcg 2040

tgtgctagag gaagagctcg gcaaggagct cgatgccatc atagcgccca tcaccccgac 2100

tgctgctatt agacacaacc agttccggta ctatggctac gcttcggtga tcaacttgct 2160

ggacttcacc agcgtcgtgg ttcctgtgac gtttgcggac aagactatcg atgtgaagaa 2220

tgaggagtat aagccgttga acgagctgga tgcgctggtc aacaaggagt atgatgcgga 2280

ggcgtaccat ggcgcccccg tagcggtgca gattatcggg aggaagttga gtgaggaacg 2340

gacattggcg attgcagagg aaataggaag gctattgggt aactctgtga caccttgaaa 2400

tagcattttg acgtttccgg ggtttggttc atggtggagg tattcctcaa ttatccttgt 2460

ttccatgaaa tgatactttt cacaggaata ttgtatcgct tgatcgtttc tatcagctat 2520

gggccataga ccaaccacac tgtctcgaaa ctagctgtaa aatcagggtt acgatataga 2580

ccacgtataa gaacgcccgg aaggaatggg acatgctgtc aatgttgatg cgtcttaaaa 2640

tgtagagggc cagtggagca cattcccctg cctccagagc ccatggatgc agaaaatcct 2700

aaacacctgg accgctatat ccaggtatat gaagccgata gatacatccg gcgttatcag 2760

ccccatcagc tctacgctgc ggggatggga gatcttattg cagctccatg aatcccgcct 2820

gatacgcccg gtggatcgtc gaagacagaa tcctcttgat ttccgagacg aatgctttgt 2880

ttgccatttg attctataaa agcacctcga gatgcggtat tggcatgcaa gcttggcact 2940

ggccgtcg 2948

<210> 6
<211> 2661
<212> DNA
<213> Aspergillus oryzae

<220>
<221> exon
<222> (360)..(626)

<220>
<221> exon
<222> (678). .(818)

<220>
<221> exon
<222> (871)..(1141)

<220>
<221> exon
<222> (1194)..(1343)

<220>
<221> exon
<222> (1392)..(1549)

<220>

<221> exon

<222> (1603)..(1752)

<220>

<221> exon

<222> (1801)..(2301)

<400> 6

aatcagtctg tagaatgctg gaccagcccg aaagcactcc caggcttggt gaatttataa 60

tggtaaagca atggttatct catgtgggtc cagacaactc ccagaaccac atggccaaga 120

taaacacgag ataaccggtt ttatcgccca attaaaagct aaaaagcctc acttatgctt 180

ccatttctcc ttgcgcggtc cgtatggccg cgatgcaaag ttgaggttgc ggggtaaccc 240

caaatttgag acttggagat tggagatata aagcaacgta ctccatggtc aatgttctgt 300

ggaaacacca gggtgattgc tctaccatcg tctacagctg tatttatcac tttgtcact 359

atg cca tct gcc agc tgg gaa gat ctc gct gcc gac aag agg gca cgt 407
Met Pro Ser Ala Ser Trp Glu Asp Leu Ala Ala Asp Lys Arg Ala Arg
1               5                   10                  15

ttg gag aag tcc atc ccc gac gaa tgg aaa ttc aag tca gtc cca ata 455
Leu Glu Lys Ser Ile Pro Asp Glu Trp Lys Phe Lys Ser Val Pro Ile

            20                  25                  30

gaa ggc tcg gtc atc gat cta cct gag aag tct ggg att ctg tcg cct 503
Glu Gly Ser Val Ile Asp Leu Pro Glu Lys Ser Gly Ile Leu Ser Pro
        35                  40                  45

tct gaa ata aag att aca aac tcg tct gcc aca gaa ctt gtc gct caa 551
Ser Glu Ile Lys Ile Thr Asn Ser Ser Ala Thr Glu Leu Val Ala Gln
    50                  55                  60

tta gcc aat ggc acg ttg aag tcc gtg gat gtg aca ctc gca ttc tgt 599
Leu Ala Asn Gly Thr Leu Lys Ser Val Asp Val Thr Leu Ala Phe Cys
65                  70                  75                  80

aaa aga gct gca ctg gct cat caa ctt gtgggtataa ccttcgcctc 646
Lys Arg Ala Ala Leu Ala His Gln Leu
                85

gatcggagat acatgaaact aatgagaata g gtt aat tgc gca cat gac ttc 698
Val Asn Cys Ala His Asp Phe
90                  95

ttc cca gag cta gca cta gcc cag gcc agg gaa ctt gat cgg tat ttc 746
Phe Pro Glu Leu Ala Leu Ala Gln Ala Arg Glu Leu Asp Arg Tyr Phe
            100                 105                 110

gag acg cac aag aaa ccc gtg gga cca ttg cat gga tta ccg att tct 794
Glu Thr His Lys Lys Pro Val Gly Pro Leu His Gly Leu Pro Ile Ser

        115                 120                 125

ttg aaa gac caa tta cga gtc aag gtaagacgag cttcctacac tactgtgtgc 848
Leu Lys Asp Gln Leu Arg Val Lys
    130                 135

atctcttcta acatagaact ag gga act gaa aca tgc atg gcc tat atc 897
Gly Thr Glu Thr Cys Met Ala Tyr Ile
            140                 145

tct tgg ctg ggt aag cgc gac acc agc gat tcg ata ttg act gcc ctc 945
Ser Trp Leu Gly Lys Arg Asp Thr Ser Asp Ser Ile Leu Thr Ala Leu
                150                 155                 160

ttg aga aaa gcg ggc gca gta ttc ctt gtt aag acg agt gta cca caa 993
Leu Arg Lys Ala Gly Ala Val Phe Leu Val Lys Thr Ser Val Pro Gln
            165                 170                 175

aca ctg atg gta tgt gag acc gtc aat aat att atc ggt cgg aca tcg 1041
Thr Leu Met Val Cys Glu Thr Val Asn Asn Ile Ile Gly Arg Thr Ser
        180                 185                 190

aac cca agg aat ctc aac ctt tct tgc ggt ggt agt tcg gga ggc gaa 1089
Asn Pro Arg Asn Leu Asn Leu Ser Cys Gly Gly Ser Ser Gly Gly Glu
    195                 200                 205

ggt gcc atg att gca atg cgt gga ggc gcc atc ggt ata gga act gat 1137
Gly Ala Met Ile Ala Met Arg Gly Gly Ala Ile Gly Ile Gly Thr Asp

210                    215                    220                    225

atc g gtagctatcc atacttggtt catcagttat tctggcgact aatgatatcc     1191
Ile

ag gt gga tct att cgt gtc cca gcc gca ttc aac tcc ttg tat ggg     1237
   Gly Gly Ser Ile Arg Val Pro Ala Ala Phe Asn Ser Leu Tyr Gly
               230                    235                    240

att cgt cca agt cac gat cgt ctg cct tac ggt ggt atg acg aac agc     1285
Ile Arg Pro Ser His Asp Arg Leu Pro Tyr Gly Gly Met Thr Asn Ser
            245                    250                    255

atg gaa ggt cag gaa acg ata cac agc gtc gtt gga cca att gcg cat     1333
Met Glu Gly Gln Glu Thr Ile His Ser Val Val Gly Pro Ile Ala His
        260                    265                    270

tct gct caa g gtagggatct gggatatttc ttcgcgtcga gatactgatg     1383
Ser Ala Gln
    275

ctttctag at gtc aga ctc ttc ctt cag tct gtc ctt aag gag gaa cct     1432
         Asp Val Arg Leu Phe Leu Gln Ser Val Leu Lys Glu Glu Pro
                     280                    285                    290

tgg aag tat gat tcg aaa gtc ata ccg ctt cct tgg agg gag gcc gag     1480
Trp Lys Tyr Asp Ser Lys Val Ile Pro Leu Pro Trp Arg Glu Ala Glu

                295                 300                 305

gag aac gcc gcc caa gca aaa att gct gag aag agt cta aat ttc gca 1528
Glu Asn Ala Ala Gln Ala Lys Ile Ala Glu Lys Ser Leu Asn Phe Ala
            310                 315                 320

ttt tac gat ttt gat ggc gtt gtaagtatta gtcgctcctc ctccttcgca 1579
Phe Tyr Asp Phe Asp Gly Val
        325

atcatgcctg acagttggat aag gta cgt cct cac cct ccg att act cgt 1629
Val Arg Pro His Pro Pro Ile Thr Arg
330                 335

ggc gtt gag atc gtc cgg tct acg ctc gag aag gac gga cat acc gtg 1677
Gly Val Glu Ile Val Arg Ser Thr Leu Glu Lys Asp Gly His Thr Val
    340                 345                 350

gca ccc tgg aca ccc tac aag cat gca ttt gcc gta gat tta gcc aac 1725
Ala Pro Trp Thr Pro Tyr Lys His Ala Phe Ala Val Asp Leu Ala Asn
355                 360                 365                 370

aaa atc tac gct gca gat gga agc acg gtaagtagcc cccctaagaa 1772
Lys Ile Tyr Ala Ala Asp Gly Ser Thr
                375

aattagtata cgtgctaaca tattgtag gat gtt tac aag cac atc aac gcc 1824
Asp Val Tyr Lys His Ile Asn Ala

380                 385

tca gga gaa ccc gct att ccg aac atc aag gac ctc atg aat ccc aac 1872
Ser Gly Glu Pro Ala Ile Pro Asn Ile Lys Asp Leu Met Asn Pro Asn
        390                 395                 400

cta ccc aag gca gat ttg aat gag gta tgg gac gcg cag ctg caa aaa 1920
Leu Pro Lys Ala Asp Leu Asn Glu Val Trp Asp Ala Gln Leu Gln Lys
    405                 410                 415

tgg cgt tat cag tgt gaa tac ctt gac aag tgg cgc gaa tgg gag gaa 1968
Trp Arg Tyr Gln Cys Glu Tyr Leu Asp Lys Trp Arg Glu Trp Glu Glu
420                 425                 430                 435

cgg acg ggc aag gag ctt gac gct atc atc gcc ccg gtg gcg gcg aca 2016
Arg Thr Gly Lys Glu Leu Asp Ala Ile Ile Ala Pro Val Ala Ala Thr
                440                 445                 450

gct gca gtc cgc cac aac caa ttc cgg tac tat ggg tat gct act gtc 2064
Ala Ala Val Arg His Asn Gln Phe Arg Tyr Tyr Gly Tyr Ala Thr Val
            455                 460                 465

ttt aac gtg tta gat tac acc agt gtt gtt gtc ccg gtt acc tat gca 2112
Phe Asn Val Leu Asp Tyr Thr Ser Val Val Val Pro Val Thr Tyr Ala
        470                 475                 480

gac aag gcg gtg gat cac aga ttg gcg gat tat cag ccg gtt agt gat 2160
Asp Lys Ala Val Asp His Arg Leu Ala Asp Tyr Gln Pro Val Ser Asp

485                 490                 495

atg gat aag gcg gtt tat gcg gag tat gat ccc gag gtt tat cat ggc    2208
Met Asp Lys Ala Val Tyr Ala Glu Tyr Asp Pro Glu Val Tyr His Gly
500                 505                 510                 515

gca ccc gtt gcc gtg cag att atc ggc aga cgt ctt agt gag gag cgg    2256
Ala Pro Val Ala Val Gln Ile Ile Gly Arg Arg Leu Ser Glu Glu Arg
                520                 525                 530

acc ctg gct att gcg gag tat gtt ggg aag ttg tta ggt cac            2298
Thr Leu Ala Ile Ala Glu Tyr Val Gly Lys Leu Leu Gly His
            535                 540                 545

tagctttcaa gctcagtttt agccactact tgaagcgtct ttgtcgagtg agactactgt 2358

aattagcata ccatttacga ggccacgccg agtatttcgc cattcaaagc agcttatacg 2418

tacctatggg ttgttctgat atatgggtag cttataatct ccactcctcg tattccgaag 2478

atacccaaaa atcctttaca atctgtctaa tgccttgctg aatggcgcca catccacaag 2538

gtcggtgtca tctttcgagc cgtaatgttt ctaccaagga tgtactaatg acactccttg 2598

acagcttaga cctactacgc acgcctcgtt atgtgtgtca gagacaaata agcactatct 2658

aga                                                                2661

<210> 7

<211> 1647

<212> DNA

<213> Monascus purpureus

<400> 7

atgaccgcca cctgggagga acgggctgcc gataagagac accggatcga gcagtcgatt 60

ccggcggaat ggaaggtcaa gtcccttccc actgcagact ctgttttcga ttttccggag 120

aagtcggggc tgttatccga gaaggaacgg gaaatcacac agtcttctgc gacggatctc 180

gtggcgaagc tggcgaaggg agaactgaag tcggtggatg ttactttggc gttttgtaag 240

agggctgctt tggcgcatca attggtcaac tgtgttcttg aattcttccc ggaagccgcc 300

ctggcgcagg cgaaagggct cgatgcatat tttgaggagc acaagaagcc tgttgggccg 360

ctccatgggc ttcctatttc cctaaaggac cagttgcgaa tcaagggcct tgaaacatcc 420

atgggatacg tctcctggct gggaaaatat gaaacccgtg attccatact cacggccctc 480

ctgcgcaaag ccggcgcggt tttctacgtg aagacaagcg tccctcaaac tctgatggta 540

tgcgagacaa tcaacaacat caccggtcga actcttaacc cgcggaacaa gaattggtcg 600

tgtggtggca gctctggagg cgagggcgcc atggttggca tccgtggcag tattattgga 660

gttggcactg atattggcgg ctctattcgg gttccctctg cgttcaattt cctctacggg 720

attcgaccga gccatggtag gatgccgtat ggctacatgg cgaacagtat ggagggacag 780

gagaccgtcc atagcgttgt tggtcccatt gcgcattctg cttcggatct gagacttttc 840

ttgacgtcgg ttctcagcga ggaaccatgg aagtatgact cgaaagtagt tccgctaccg 900

tggaggtctc atgaagaaga ggccattcgc actaaactgc agtccggggg tcttacattg 960

gggttcttta attgcgacgg aaacgttctt ccccatccac cagttctacg aggccttgat 1020

acggttgtct cggtgctgaa gaagaatggg cacagcattg ttccctggac accatacaaa 1080

catgactttg ccgttgacct gatctacgga atttattctg ctgatggaag cacggacgta 1140

ctacgagaca tcaacgcctc tggagagccc gctattccaa acatcaaaga tctcctcaac 1200

ccgagcgtca ggaaagccga cctaaacgat gtctggaatg tgcaactcca gaaatggaag 1260

tatcagtgcg agtatctcga caaatggcgt gtgctagagg aagagctcgg caaggagctc 1320

gatgccatca tagcgcccat caccccgact gctgctatta gacacaacca gttccggtac 1380

tatggctacg cttcggtgat caacttgctg gacttcacca gcgtcgtggt tcctgtgacg 1440

tttgcggaca agactatcga tgtgaagaat gaggagtata agccgttgaa cgagctggat 1500

gcgctggtca acaaggagta tgatgcggag gcgtaccatg gcgcccccgt agcggtgcag 1560

attatcggga ggaagttgag tgaggaacgg acattggcga ttgcagagga aataggaagg 1620

ctattgggta actctgtgac accttga 1647

<210> 8

<211> 548

<212> PRT

<213> Monascus purpureus

<400> 8

Met Thr Ala Thr Trp Glu Glu Arg Ala Ala Asp Lys Arg His Arg Ile
1               5                   10                  15

Glu Gln Ser Ile Pro Ala Glu Trp Lys Val Lys Ser Leu Pro Thr Ala
            20                  25                  30

Asp Ser Val Phe Asp Phe Pro Glu Lys Ser Gly Leu Leu Ser Glu Lys
        35                  40                  45

Glu Arg Glu Ile Thr Gln Ser Ser Ala Thr Asp Leu Val Ala Lys Leu
    50                  55                  60

Ala Lys Gly Glu Leu Lys Ser Val Asp Val Thr Leu Ala Phe Cys Lys
65                  70                  75                  80

Arg Ala Ala Leu Ala His Gln Leu Val Asn Cys Val Leu Glu Phe Phe
                85                  90                  95

Pro Glu Ala Ala Leu Ala Gln Ala Lys Gly Leu Asp Ala Tyr Phe Glu
            100                 105                 110

Glu His Lys Lys Pro Val Gly Pro Leu His Gly Leu Pro Ile Ser Leu
        115                 120                 125

Lys Asp Gln Leu Arg Ile Lys Gly Leu Glu Thr Ser Met Gly Tyr Val
    130                 135                 140

Ser Trp Leu Gly Lys Tyr Glu Thr Arg Asp Ser Ile Leu Thr Ala Leu
145                 150                 155                 160

Leu Arg Lys Ala Gly Ala Val Phe Tyr Val Lys Thr Ser Val Pro Gln
                165                 170                 175

Thr Leu Met Val Cys Glu Thr Ile Asn Asn Ile Thr Gly Arg Thr Leu
            180                 185                 190

Asn Pro Arg Asn Lys Asn Trp Ser Cys Gly Gly Ser Ser Gly Gly Glu
        195                 200                 205

Gly Ala Met Val Gly Ile Arg Gly Ser Ile Ile Gly Val Gly Thr Asp
    210                 215                 220

Ile Gly Gly Ser Ile Arg Val Pro Ser Ala Phe Asn Phe Leu Tyr Gly
225                 230                 235                 240

Ile Arg Pro Ser His Gly Arg Met Pro Tyr Gly Tyr Met Ala Asn Ser
                245                 250                 255

Met Glu Gly Gln Glu Thr Val His Ser Val Val Gly Pro Ile Ala His
            260                 265                 270

Ser Ala Ser Asp Leu Arg Leu Phe Leu Thr Ser Val Leu Ser Glu Glu
        275                 280                 285

Pro Trp Lys Tyr Asp Ser Lys Val Val Pro Leu Pro Trp Arg Ser His
    290                 295                 300

Glu Glu Glu Ala Ile Arg Thr Lys Leu Gln Ser Gly Gly Leu Thr Leu
305                 310                 315                 320

Gly Phe Phe Asn Cys Asp Gly Asn Val Leu Pro His Pro Pro Val Leu
                325                 330                 335

Arg Gly Leu Asp Thr Val Val Ser Val Leu Lys Lys Asn Gly His Ser
            340                 345                 350

Ile Val Pro Trp Thr Pro Tyr Lys His Asp Phe Ala Val Asp Leu Ile
        355                 360                 365

Tyr Gly Ile Tyr Ser Ala Asp Gly Ser Thr Asp Val Leu Arg Asp Ile

    370                 375                 380

Asn Ala Ser Gly Glu Pro Ala Ile Pro Asn Ile Lys Asp Leu Leu Asn
385                 390                 395                 400

Pro Ser Val Arg Lys Ala Asp Leu Asn Asp Val Trp Asn Val Gln Leu
                405                 410                 415

Gln Lys Trp Lys Tyr Gln Cys Glu Tyr Leu Asp Lys Trp Arg Val Leu
            420                 425                 430

Glu Glu Glu Leu Gly Lys Glu Leu Asp Ala Ile Ile Ala Pro Ile Thr
        435                 440                 445

Pro Thr Ala Ala Ile Arg His Asn Gln Phe Arg Tyr Tyr Gly Tyr Ala
    450                 455                 460

Ser Val Ile Asn Leu Leu Asp Phe Thr Ser Val Val Val Pro Val Thr
465                 470                 475                 480

Phe Ala Asp Lys Thr Ile Asp Val Lys Asn Glu Glu Tyr Lys Pro Leu
                485                 490                 495

Asn Glu Leu Asp Ala Leu Val Asn Lys Glu Tyr Asp Ala Glu Ala Tyr
            500                 505                 510

His Gly Ala Pro Val Ala Val Gln Ile Ile Gly Arg Lys Leu Ser Glu
        515                 520                 525

Glu Arg Thr Leu Ala Ile Ala Glu Glu Ile Gly Arg Leu Leu Gly Asn
    530                 535                 540

Ser Val Thr Pro
545

<210> 9
<211> 4142
<212> DNA
<213> Monascus purpureus

<220>
<221> exon
<222> (616)..(740)

<220>
<221> exon
<222> (827)..(875)

<220>
<221> exon
<222> (936)..(1092)

<220>
<221> exon
<222> (1177)..(1949)

<400> 9
   aagcttacac tttcacttct aggacatgga aaagccagcc cctggagtaa cgctcgcgac 60
   aacatgcata gatacatcac accatgcaca tgcacagttg aaacacatac cttacgtatt 120
   tcctgagctt ccgcatctgc ttcccacggt accgcacgaa gatctgccaa cccaccacgt 180
   aaccgaggtg ctgcttgcag tcacaaactg ccgacagctc gggacaaccc ctttccccgc 240
   agtactacct ccgcattccc cagcaagacc gactttgacc agtggcatga tatcattcat 300
   gtgcatgcag aggagtgtag gagtacagct gaagggctgg tgacccctcc cagcccccga 360
   caggaagatg caggagagct gagtcgatcg agaacactgg gaggggccgg tacaaagtac 420
   ggaatactgg ctctgtctgg agatataaaa tgaactataa aatgccggtc atttccccca 480
   attgactcag aactgggcta tcagaagttt tactaccttt atcgaactgc ctgcatttct 540
   ctaactgtac ttctgtttgc aggctttcat atcatctgcg tctttatacc ttcttctcat 600
   tgttcactct tcaacatggc tgaacctcaa atcccaacca agcagaaagc tgcgatctat 660
   gataaaccgg gtacggtctc caccaaagtt gtggagatag atgtgcctga acccggaccg 720
   ggagaagttc ttgtcaattt gtatgtcttg tacacccagt caatgccctc atgccttgcg 780
   aattattatt gagagtatac atatagctga ctacaggatg ctgcaggact cactcgggta 840
   tatgccactc ggattatggt gtcatgacaa actctgttcg tcttcatcta tactgcgcat 900
   acactgtagt ccaattctaa caatgatatc accagtggaa actgctcccc tacccaaccc 960
   agcccggaca gattggcggc catgaaggag tcggaaaggt agtcaagttc ggccctggag 1020
   ccaatggaac cgggttgaag attggcgata gggtaggaat caaatgggtg tccagtgcat 1080
   gcgggaactg tcgtacgtcc ggccttcatt tcttatgaaa aggaaaagga aaatcctttt 1140
   ctcttggacc aattcattaa atacaataat acacagaccc atgccacgca ggcgcagacg 1200
   gaatctgcct caaccagaaa atctccggct actacacccc gggcacattc caacaatacg 1260
   cgacaggccc agctaactac gtaaccccga tccccgaaaa cctctcttcg gccgaggccg 1320
   cgccccttct ctgcgccggc gtcaccgtct acgcagccct gaaacgcagc aaagcccaac 1380
   caggccagtg gattgtgatt tccggcgcag acggcggcct gggccaccta gccgtccaaa 1440
   tagccagtcg aggcatgggc ctgcgcgtga tcggcatcga ccacggcagt aaagcagccc 1500
   tcgttaagga atcaggtgcg gagcatttcg tcgatatcac cgcattcccg aaagacgaca 1560
   atggcgctgc catcgcggca cacgtgaagt ccctcacgac agagaagttg ggggcccacg 1620
   ccgttatcgt ctgcacggcg tcaaacgcgg cgtacgcgca ggcatttctc ttcctgcgct 1680
   tcaatggtac gctggtctgt gtggggatgc cggaacatga gtcccaggct attgctacgg 1740
   catatccggc tgccatcgtt ttcaaccagg ctactattac cggttcggca gtggggaatc 1800
   ggatagaggc gattgaggtg ctagattttg ccgctagggg tattatcaaa tctcatgtta 1860
   ggatggctaa gttggaggat ttgacagatt tgtttaatga gatggcagag ggaaagttgc 1920
   aggggagagt ggtcttggat ctttcttagt tgggttggat ggttgacgag atgggaaaca 1980
   gtcaatttta tgattgaaat tgattaattg atttgtacct gaatgtcttg gggtttgcct 2040
   ataaggagta gtttattaag gaaaccatat aactaactag cagcccagtc aaggtaaaag 2100
   caagataaca ggcgacagca acacaaagaa aaagaaggca aggtttcagc gcgaaaagca 2160
   ctaacgcatg ccatatcaat cttcatcaaa tccacgagcc cgtttactgt cctctcgaaa 2220
   cgaagctaga gctgggtgtt tctgcgcaga actcgaggaa gcctctgcgc ccgactcagc 2280
   aacatggatc agggtagcca gcttgcgatg tacagctcag gagcctgatt gacctcaatc 2340
   ctgcgcagga tatcctttaa cttggacgtg tactgacgga gatccgacgt agggagcaat 2400
   tcaaggacat cgagaatgaa gaacaggagc ctcatctggc cttttctcag atccgataag 2460
   ctgatggtac aagttgcgat ggtcttcctc ttcgccgtga aatgtggctc ggccccacat 2520
   gttaccgtcc agagcgtcct tgttgcgatt gctgatatcc tccagcaagg tgacaagggc 2580
   tgttgtggct cccccggtgc catatgggaa tcgggctaga atgttttctc ggatacgatg 2640
   gacgttgtac cggatttgtt tgatgacgtt gctgacgtcg acgacaacga cacttgttgc 2700
   gccgtccgtg ggtaactggc ctgtctggca gtatcagtcg aaatcggaga gtaatttctg 2760
   tgatttctgc agaatcttgt cgaagtagat agctgcgcag gctccagcgg gcattgcctt 2820
   ggagaggctg aagtagacgt cgtcgtgcac ggctagtcga aacatggtgg cttcgaagtc 2880
   tccttgaacg acgcactgct ctggcttacg aacttgtctc gctatcgcta ccaagtcagg 2940
   acggaaatct tcagggaggg tgattttgtt gaacgcagac atgatgtctc gcgctctgtc 3000
   cgatctgctt aaacacgaga aaccctccgt ttcactgtca acatattgtc aatttaattg 3060
   atctgtgact actcccagct tgccatctag gagttgttca atgtgtgaga accctggagt 3120
   atatccattg ctcgacaagg gaacggcagt ggtagggggt tgggccagga gacagccatg 3180
   gggttgatcg agaagccatt atcaacggga agaaatcagt tcttcatttc tcttcccctt 3240
   gccaacatgc acggagaggg actgtacttc caaaaatgtg ctgaaaaggc acgctgggcc 3300
   gattcaagaa gaccgcacaa gtgcatttat acccatccgg ctgatagtaa ggacctgcgg 3360
   ccgctccgac agtcgaaagc cataccctat atgcatggcg cgacagccca caacgtcgaa 3420
   ttgggaagta aggccgacga gagctttgga cattataagt aatgccggat cttgctagaa 3480
   ctctcgacaa tttagtttct tcgctggtat tatcatttct cgggccggaa gtctctacac 3540
   actgcatgtt gatctggatt aaaaggtctc ggaactgtct tgttggagta gacgtagcga 3600
   gaaatgatta ggagggtttg ctattttcgt agaccccgag aaggtcctat cccggtatgt 3660
   ataagggcct catgggattc atggtcccta acgtgagcag cgtatgaacc aaggtatagc 3720
   tggtaatgag tactcacaag ctattgaata gatttagagt aaattcggga ttctcagtca 3780
   aatggcatga tcaggatagg gtgcatttcc cccgaggcgt aggacatgaa gccaaattta 3840
   cagacccaga ccctgcctca ttaggtttct tgtgcagtga agggccaatg aacactgaga 3900
   aatagcaatg acggcattat catgcccggg acctattccg acaaacagga tgaaaattag 3960
   ccgatgcccc ttgggaactg tgaacaatcc aacttggcca tattgcctac atttcatcat 4020
   tggttgcacg gcaaaaagtc atagacaaga gcgcttaaga agcgtagaac taaatcaacg 4080
   aataccagtc cttccagata tgcaaaggaa aagtatcgta tccatataga tgacccaagc 4140
   tt 4142

<210> 10

<211> 2190

<212> DNA

<213> Aspergillus nidulans

<220>

<221> exon

<222> (776)..(900)

<220>

<221> exon

<222> (999)..(1047)

<220>

<221> exon

<222> (1114)..(1270)

<220>

<221> exon

<222> (1325)..(2097)

<400> 10

tggccatagg cattacggct tgacatcatt ggcatagact tgttcaagaa ggttcattat 60

atagtgagtg acgcttggag tccagggtat ctcacttcga cgcgatataa agtcagacct 120

tgtcctagac atcgtctgcg gtcctgctat ggttcctcct cctggacccc cgcgttttcc 180

ccgccatcac atcgagcaat tcgacaccaa gttccgcagc gtttccgtcc caaccttgaa 240

cctgtcggat cccattggcg aagaccacat tacgaacact ctcaagtcag ctagtttact 300

gttacggact caaaagacta tgaaacgcgt cttatttgtc taccgtaacc gaggctgttc 360

caccacggca cctcctcggc cgccggcgcc cctcacgctg cctcgagact gattggatga 420

atcatgatac cacgttccat gaatgcgata ttctagctta gctgcgtcat gctgaaactt 480

cgctctctca gactcctgct ttgggctggc ctaaggccca ctgctctctc ctccttggat 540

cagccactct cccctccctg cggagatgcc ctttcccctc attttcatat cagcaatcag 600

actacctgac tgcttctaag gttatggtca ggctagagat gatagtcgac ttggcgttac 660

gttgctctgg tctgggatag ccatataaag agaggcttgt ccttcctcta cttctttcac 720

cttcccacat ttatcttatc aacgatacaa gagaagcccc gcgaaatctg tcaga atg 778
Met
1

gct gct cct gaa atc ccc aag aag caa aag gct gtc atc tac gac aac 826
Ala Ala Pro Glu Ile Pro Lys Lys Gln Lys Ala Val Ile Tyr Asp Asn
            5                   10                  15

ccc ggt acc gtc tct acc aag gtc gtc gag ctg gat gta cct gag ccc 874
Pro Gly Thr Val Ser Thr Lys Val Val Glu Leu Asp Val Pro Glu Pro
        20                  25                  30

ggc gac aat gaa gtc ctg atc aat ct gtgcgtgccc tggatatacc 920
Gly Asp Asn Glu Val Leu Ile Asn Leu
    35                  40

tgttaccttg gaggaatggg atatccaagc cgagtcgagt actggcatgt accaattact 980

aacgttataa cattgtag c act cat tcc ggc gtt tgc cac tca gat ttt 1029
Thr His Ser Gly Val Cys His Ser Asp Phe
        45                  50

ggt att atg acc aac acg gtaggcactt agttcctgtc ctggagtctc 1077
Gly Ile Met Thr Asn Thr
        55

tatcaagggc gaacgcatgc taatggtatg atacag tgg aag ata cta ccc ttc 1131
Trp Lys Ile Leu Pro Phe
    60

cct act cag ccc gga caa gtc ggt ggc cat gaa ggc gtt ggc aaa gtg 1179
Pro Thr Gln Pro Gly Gln Val Gly Gly His Glu Gly Val Gly Lys Val
65                  70                  75                  80

gtg aag ctc ggc gcg ggc gct gaa gca tca gga ttg aag atc ggg gac 1227

Val Lys Leu Gly Ala Gly Ala Glu Ala Ser Gly Leu Lys Ile Gly Asp
                85                  90                  95

aga gtc ggt gtt aag tgg att tcc agc gcc tgt ggg cag tgc c 1270
Arg Val Gly Val Lys Trp Ile Ser Ser Ala Cys Gly Gln Cys
            100                 105                 110

gtacgtacat acaccttaga cgcattttaa ccaaaacttg ctaacaacgc aaag ct 1326
Pro

cca tgc cag gac ggc gcc gac ggc ctc tgc ttc aac caa aag gta tca 1374
Pro Cys Gln Asp Gly Ala Asp Gly Leu Cys Phe Asn Gln Lys Val Ser
            115                 120                 125

ggt tac tac acc cct ggc aca ttc cag caa tac gtg ctc ggt cct gcg 1422
Gly Tyr Tyr Thr Pro Gly Thr Phe Gln Gln Tyr Val Leu Gly Pro Ala
        130                 135                 140

caa tac gtt acc cca att ccc gat ggc ctc cca tca gcc gaa gcg gcg 1470
Gln Tyr Val Thr Pro Ile Pro Asp Gly Leu Pro Ser Ala Glu Ala Ala
    145                 150                 155

ccc ctt ctc tgt gcc ggt gtc aca gtc tac gct tct ctt aag cgc agt 1518
Pro Leu Leu Cys Ala Gly Val Thr Val Tyr Ala Ser Leu Lys Arg Ser
160                 165                 170                 175

aaa gcc caa cca ggt caa tgg atc gtc atc tcc ggc gct ggc ggc ggc 1566
Lys Ala Gln Pro Gly Gln Trp Ile Val Ile Ser Gly Ala Gly Gly Gly

                180                 185                 190

ctt ggc cac tta gcc gtc cag atc gca gcc aag ggc atg ggc ctg cgt 1614
Leu Gly His Leu Ala Val Gln Ile Ala Ala Lys Gly Met Gly Leu Arg
            195                 200                 205

gtg att ggc gtt gac cac ggg agt aaa gaa gag ctc gtc aag gcg tca 1662
Val Ile Gly Val Asp His Gly Ser Lys Glu Glu Leu Val Lys Ala Ser
        210                 215                 220

ggc gcc gag cac ttc gtg gat atc acc aag ttc cca acg ggc gat aaa 1710
Gly Ala Glu His Phe Val Asp Ile Thr Lys Phe Pro Thr Gly Asp Lys
    225                 230                 235

ttc gag gcc atc tcc tcg cac gtc aaa tcg ctt aca acg aag ggt ctt 1758
Phe Glu Ala Ile Ser Ser His Val Lys Ser Leu Thr Thr Lys Gly Leu
240                 245                 250                 255

ggt gcg cat gct gtc ata gtt tgc acg gcg tcc aat att gct tac gct 1806
Gly Ala His Ala Val Ile Val Cys Thr Ala Ser Asn Ile Ala Tyr Ala
                260                 265                 270

cag tct ttg ctc ttc ctc cgg tac aac gga acg atg gtc tgc gtg ggt 1854
Gln Ser Leu Leu Phe Leu Arg Tyr Asn Gly Thr Met Val Cys Val Gly
            275                 280                 285

atc ccc gag aac gag ccg cag cgt atc gca agt gcg tac cca ggc ctg 1902
Ile Pro Glu Asn Glu Pro Gln Arg Ile Ala Ser Ala Tyr Pro Gly Leu

290                 295                 300

ttt atc cag aag cat gtg cat gtc act ggg tcg gct gtc gga aat agg    1950
Phe Ile Gln Lys His Val His Val Thr Gly Ser Ala Val Gly Asn Arg
    305                 310                 315

aac gag gcg att gag act atg gag ttt gcg gcg agg ggt gtc att aag    1998
Asn Glu Ala Ile Glu Thr Met Glu Phe Ala Ala Arg Gly Val Ile Lys
320                 325                 330                 335

gcg cac ttc cgg gag gag aag atg gag gcc ttg act gaa att ttc aaa    2046
Ala His Phe Arg Glu Glu Lys Met Glu Ala Leu Thr Glu Ile Phe Lys
                340                 345                 350

gag atg gag gag ggg aag ttg cag ggg cgg gtg gtg ctt gat ctt tct    2094
Glu Met Glu Glu Gly Lys Leu Gln Gly Arg Val Val Leu Asp Leu Ser
            355                 360                 365

tagtagctct tagcttggat gccctgcatg gcctgacttg ttatgtttat ttcaatgtta 2154

acttctcttg aaatatgctc ttgttagtca accctt                           2190

<210> 11
<211> 1104
<212> DNA
<213> Monascus purpureus

<400> 11

atggctgaac ctcaaatccc aaccaagcag aaagctgcga tctatgataa accgggtacg 60

gtctccacca aagttgtgga gatagatgtg cctgaacccg gaccgggaga agttcttgtc 120

aatttgactc actcgggtat atgccactcg gattatggtg tcatgacaaa ctcttggaaa 180

ctgctcccct acccaaccca gcccggacag attggcggcc atgaaggagt cggaaaggta 240

gtcaagttcg gccctggagc caatggaacc gggttgaaga ttggcgatag ggtaggaatc 300

aaatgggtgt ccagtgcatg cgggaactgt cacccatgcc acgcaggcgc agacggaatc 360

tgcctcaacc agaaaatctc cggctactac accccgggca cattccaaca atacgcgaca 420

ggcccagcta actacgtaac cccgatcccc gaaaacctct cttcggccga ggccgcgccc 480

cttctctgcg ccggcgtcac cgtctacgca gccctgaaac gcagcaaagc ccaaccaggc 540

cagtggattg tgatttccgg cgcagacggc ggcctgggcc acctagccgt ccaaatagcc 600

agtcgaggca tgggcctgcg cgtgatcggc atcgaccacg gcagtaaagc agccctcgtt 660

aaggaatcag gtgcggagca tttcgtcgat atcaccgcat tcccgaaaga cgacaatggc 720

gctgccatcg cggcacacgt gaagtccctc acgacagaga agttgggggc ccacgccgtt 780

atcgtctgca cggcgtcaaa cgcggcgtac gcgcaggcat ttctcttcct gcgcttcaat 840

ggtacgctgg tctgtgtggg gatgccggaa catgagtccc aggctattgc tacggcatat 900

ccggctgcca tcgttttcaa ccaggctact attaccggtt cggcagtggg gaatcggata 960

gaggcgattg aggtgctaga ttttgccgct aggggtatta tcaaatctca tgttaggatg 1020

gctaagttgg aggatttgac agatttgttt aatgagatgg cagagggaaa gttgcagggg 1080

agagtggtct tggatctttc ttag 1104

<210> 12
<211> 367
<212> PRT
<213> Monascus purpureus

<400> 12
Met Ala Glu Pro Gln Ile Pro Thr Lys Gln Lys Ala Ala Ile Tyr Asp
1               5                   10                  15

Lys Pro Gly Thr Val Ser Thr Lys Val Val Glu Ile Asp Val Pro Glu
            20                  25                  30

Pro Gly Pro Gly Glu Val Leu Val Asn Leu Thr His Ser Gly Ile Cys
        35                  40                  45

His Ser Asp Tyr Gly Val Met Thr Asn Ser Trp Lys Leu Leu Pro Tyr

    50                  55                  60

Pro Thr Gln Pro Gly Gln Ile Gly Gly His Glu Gly Val Gly Lys Val
65                  70                  75                  80

Val Lys Phe Gly Pro Gly Ala Asn Gly Thr Gly Leu Lys Ile Gly Asp
                85                  90                  95

Arg Val Gly Ile Lys Trp Val Ser Ser Ala Cys Gly Asn Cys His Pro
            100                 105                 110

Cys His Ala Gly Ala Asp Gly Ile Cys Leu Asn Gln Lys Ile Ser Gly
        115                 120                 125

Tyr Tyr Thr Pro Gly Thr Phe Gln Gln Tyr Ala Thr Gly Pro Ala Asn
    130                 135                 140

Tyr Val Thr Pro Ile Pro Glu Asn Leu Ser Ser Ala Glu Ala Ala Pro
145                 150                 155                 160

Leu Leu Cys Ala Gly Val Thr Val Tyr Ala Ala Leu Lys Arg Ser Lys
                165                 170                 175

Ala Gln Pro Gly Gln Trp Ile Val Ile Ser Gly Ala Asp Gly Gly Leu
            180                 185                 190

Gly His Leu Ala Val Gln Ile Ala Ser Arg Gly Met Gly Leu Arg Val
        195                 200                 205

Ile Gly Ile Asp His Gly Ser Lys Ala Ala Leu Val Lys Glu Ser Gly
    210                 215                 220

Ala Glu His Phe Val Asp Ile Thr Ala Phe Pro Lys Asp Asp Asn Gly
225                 230                 235                 240

Ala Ala Ile Ala Ala His Val Lys Ser Leu Thr Thr Glu Lys Leu Gly
                245                 250                 255

Ala His Ala Val Ile Val Cys Thr Ala Ser Asn Ala Ala Tyr Ala Gln
            260                 265                 270

Ala Phe Leu Phe Leu Arg Phe Asn Gly Thr Leu Val Cys Val Gly Met
        275                 280                 285

Pro Glu His Glu Ser Gln Ala Ile Ala Thr Ala Tyr Pro Ala Ala Ile
    290                 295                 300

Val Phe Asn Gln Ala Thr Ile Thr Gly Ser Ala Val Gly Asn Arg Ile
305                 310                 315                 320

Glu Ala Ile Glu Val Leu Asp Phe Ala Ala Arg Gly Ile Ile Lys Ser
                325                 330                 335

His Val Arg Met Ala Lys Leu Glu Asp Leu Thr Asp Leu Phe Asn Glu
            340                 345                 350

Met Ala Glu Gly Lys Leu Gln Gly Arg Val Val Leu Asp Leu Ser
        355                 360                 365

<210> 13
<211> 3831
<212> DNA
<213> Monascus purpureus

<220>
<221> exon
<222> (1014)..(1806)

<220>
<221> exon
<222> (1866)..(1983)

<220>
<221> exon
<222> (2039)..(2140)

<220>
<221> exon
<222> (2197)..(2632)

<400> 13
ttagtcctgg gggatcattg gaatgccgca gtaatcacag agtgagtaca gagtaacact 60

gtcaagcatt gagaataata tgctctccgg ttgagagggg agcgatagtt cgttagttac 120

atcgctgtgg cttatgtaat cacaccgggt gcgatgccac ggttcaatcc ctcccaagtt 180

ccaactgtgg attcgacaat ttcagctttc cacagttcag ctctacagga tcgatccatc 240

gccaaggatt ccccggatcg atcggtgcgg tggttcgtga gaacaaactc aatcgtatct 300

aacaagtcca gccagctaat tcccgcggta gcaggattac tcaataaatc ccaacgaaaa 360

acgtgaatat ttattgtcct tttcgaagag aaaaacagca gtattgtcaa tacaaaccga 420

ctgaaatacc ttcgcgagga cacggtggac tggcgcaggt attctggagc aagtgaggcc 480

ctacgcccga gatacatcga tcatcccact gacagcggtt tctagaagtg tcataaacac 540

ctcgcccatt acgcacgtgg gcacaagagg cacggtgaaa ggccttgtgg accgtctgct 600

gctgcaaggg gccgtcgaag gatgaacctc cggagatgag ctggtggctt ctcgtccgga 660

tcgcgtcggc tttgccgtgc aaagcttgtt ccgtggggct gttatcaatc aatcgagtcc 720

cgtctgacca gcgccctatt catctacgaa taatggtctg atccatgtct agcatcggac 780

aacaacgcga gcaatgcgac gttccatcgc cgacttgaac gtgtcctgca cgtgggtgcc 840

tgcagttgct cccattcctc ctccggcgaa ttctcctcag tcatcatgat cggcacacta 900

ctatcctggt atacagctgc tgctatgcaa gattgctacc ccgaaccact ataagaacct 960

gctacgcccg gtgtgaatga tgtgaaaaac agcagagcgg ccttcatctc tatatgcctc 1020

tcttctcttt tctctccgcg acaccaacag tcatgcagct tatcttcacg gttgcagcca 1080

tcgcgagtgt ggcagccggg tttcagtcgg tgattagcga aaagcaattt tctcaggaat 1140

ttctcgacaa ctacagcatc ctgaagcact atggcggtaa tggcccttac tccagccgcc 1200

ggtcctacgg gatctcgcgc gaacctcccg actcgtgctc cgtcgaccag gtcatcatga 1260

tcatgcgtca tggcgagcga tatccgtctc cagacctcgg agcgagcatc gaagcagctc 1320

tcgccaagat caagtcctcg aacgtatcca cataccaggg cgacctggat ttcctaaatt 1380

cctggaccta ctatgtcccc aatcactgtg cctacaatgc cgagacctcc accggaccgt 1440

atgctggtct cctcgaggga ttcaagcgag gtagcgacta ccgcgctcgt tacggtcatt 1500

tgtgggacgg ggagtcgatt gtcccaatct tcgctgctgg ttaccagcgt atcattgcta 1560

cttctcgaaa attcggcgag ggtttcttcg gcgccaacta ctccaccaat gcggccatca 1620

atattatctc agaggcaaag gagatgggtg caaatagcct cacacccact tgcgaccacg 1680

acaatgacac cagcacttgc aactccctga caacggtgtg gccacagttc aaagtcgctg 1740

cagcccgttt gaattctcag aaccccggtt tggatctgaa tgccactgac atctactatc 1800

tgatgtgtat gtatagtctt tcccatcctg cccctattgc cgtatggacg acttactggt 1860

tgcagccatg gcttcctttg aattgaacgc tcggccgtac tccgactgga tcaatgtttt 1920

tacccttgat gagtgggtga cgtttggtta cgttcaggac ctgaattatt attactgcgc 1980

cgggtatggg tctctggttg tcccaggtct gggtcacagt cgctgatctt tcacgcagcc 2040

caggagacaa gaacatggcc gctgtggggg ccgtatatgt aaatgcatct ctgactctcc 2100

tcaaccaagg cccgtcagct ggcacattat ggtttaactt gttagtaccc tcttcccagc 2160

aatggagccc ctattcccgg ctgacccatg ttgcagtgcc catgatacaa acatcacccc 2220

cattcttgcg gccctcggcg tcctcacccc ggagcgtgat cttcccaccg accgtgttgt 2280

cttcgatagc aagtggtcct ccggggacat cgtcccccag gccggccacc tgacaatcga 2340

gcgactgaac tgcacgagca ctgccgcatc acctgccggc gtctatgtcc gcttggtcca 2400

aaatgaggcc gtcatcccgg ttgaagcgtg ccaatcaggt cctgggtact cttgctccct 2460

ggccgacttc accgaaatta tgtccaagca gcttcccgat ttcgtgtcga cttgcagtat 2520

tcgctcgtcg taccctcagt acctagactt ctggtggaac tacaatacga ccaccgatct 2580

gaactatccg aagggcccgg ttccctgtgc cgagggagtg gcgacaagct agtaggctct 2640

atgctgtcct cacggacatg tattccacac cactcctccc atcacaacca ggacgatgct 2700

tactgaacag tccgtttcat catatggggg tgtctctgta gtatataata tgtcagtctt 2760

ttccgcaatc ttaggaatag aaggaattca ccgtcctggt ccacagtcct ctagtgggca 2820

ggaaaaatgg caatgctgac gtgtgtggaa aaataatatc cagcggcgag cggccgctgg 2880

gccgcccagc acctgtgtag catcgctatt ctgcacctgc caagaacttt ttagtcacac 2940

gtagcttgat ctactatatg aagtggagaa caaggctact ttccctgggt tgattggaaa 3000

tgttgagata gtcacgagct gctatgaatg ttttgttttg actacattaa agtacatagg 3060

tgtacatggc tgactgcttg ctgctcctgg gcaggatctt actgaagtag gtaaggaaga 3120

gcaacatgag gccgtatggg cagataacct gaacgtgtgc tacgtgagcc ccgacgggat 3180

tatctttgat gatgcatgag tcttgccagg gaatacaaca tcataatacc cagctccaga 3240

cttgaaatcc agtgttgaac tcattatgca ggagttttga cattgtagtt ggccatgcaa 3300

tctgtgtcag ctaccctggc aatgcaacct ctgaggtcaa ttcctatgcc accagtgcaa 3360

tgggggcgac ggctacctct gctgccccaa taccaagcaa tgtaattgcc tgggaccaac 3420

gtgtactgcg ggagatatta ttcaacccaa ggtaatccaa ggctttctct gtgggtttgg 3480

gacctcaccg ctgactgaac caagaaaatg attactgcca ggccatcgca atgaaggagg 3540

gaatcagcct tgacgcagca acttgtactt gaattatagc tattgtactg gcccgtcatt 3600

ccagattata gacgttccgg ggctaattca agtcaatctc tgggtgtgca gccggttgga 3660

aacattgaga tgtatcctgg ctcgcgcctg gcaggcgatg ctccagtatc ggcttgtgcg 3720

gacccactgg gtgttaccgt atccaaacga ctgacgcttc tggagtcatg gcacggcact 3780

ccatggacta gggtggagac ggctactcac ttgggtcagg gatcctctag a 3831

<210> 14

<211> 2071

<212> DNA

<213> Aspergillus niger

<220>

<221> exon

<222> (136)..(916)

<220>

<221> exon

<222> (971)..(1088)

<220>

<221> exon

<222> (1141)..(1245)

<220>

<221> exon

<222> (1305)..(1740)

<400> 14

gcatgctgga ccgcaatctc cgatcgccgg gtataaaagg tcctccaaac ccctctcggt 60

cgatatgtac cccgctcgtc atctccaatc ctctcgagag caccttctcc agcttttgtc 120

aattgtacct tcgca atg cct cgc acc tct ctc ctc acc ctg gcc tgt gct 171
Met Pro Arg Thr Ser Leu Leu Thr Leu Ala Cys Ala
1               5                   10

ctg gcc acg ggc gca tcc gct ttc tcc tac ggc gct gcc att cct cag 219
Leu Ala Thr Gly Ala Ser Ala Phe Ser Tyr Gly Ala Ala Ile Pro Gln
        15                  20                  25

tca acc cag gag aag cag ttc tct cag gag ttc cgc gat ggc tac agc 267
Ser Thr Gln Glu Lys Gln Phe Ser Gln Glu Phe Arg Asp Gly Tyr Ser
    30                  35                  40

atc ctc aag cac tac ggt ggt aac gga ccc tac tcc gag cgt gtg tcc 315
Ile Leu Lys His Tyr Gly Gly Asn Gly Pro Tyr Ser Glu Arg Val Ser
45                  50                  55                  60

tac ggt atc gct cgc gat ccc ccg acc agc tgc gag gtc gat cag gtc 363
Tyr Gly Ile Ala Arg Asp Pro Pro Thr Ser Cys Glu Val Asp Gln Val
                65                  70                  75

atc atg gtc aag cgt cac gga gag cgc tac ccg tcc cct tca gcc ggc 411
Ile Met Val Lys Arg His Gly Glu Arg Tyr Pro Ser Pro Ser Ala Gly
            80                  85                  90

aag gac atc gaa gag gcc ctg gcc aag gtc tac agc atc aac act act 459
Lys Asp Ile Glu Glu Ala Leu Ala Lys Val Tyr Ser Ile Asn Thr Thr
        95                  100                 105

gaa tac aag ggc gac ctg gcc ttc ctg aac gac tgg acc tac tac gtc 507
Glu Tyr Lys Gly Asp Leu Ala Phe Leu Asn Asp Trp Thr Tyr Tyr Val
    110                 115                 120

cct aat gag tgc tac tac aac gcc gag acc acc agc ggc ccc tac gcc 555
Pro Asn Glu Cys Tyr Tyr Asn Ala Glu Thr Thr Ser Gly Pro Tyr Ala
125                 130                 135                 140

ggt ttg ctg gac gcg tac aac cat ggc aac gat tac aag gct cgc tac 603
Gly Leu Leu Asp Ala Tyr Asn His Gly Asn Asp Tyr Lys Ala Arg Tyr
                145                 150                 155

ggc cac ctc tgg aac ggt gag acg gtc gtg ccc ttc ttt tct agt ggc 651
Gly His Leu Trp Asn Gly Glu Thr Val Val Pro Phe Phe Ser Ser Gly
            160                 165                 170

tac gga cgt gtc atc gag acg gcc cgc aag ttc ggt gag ggt ttc ttt 699
Tyr Gly Arg Val Ile Glu Thr Ala Arg Lys Phe Gly Glu Gly Phe Phe
        175                 180                 185

ggc tac aac tac tcc acc aac gct gcc ctc aac atc atc tcc gag tcc 747
Gly Tyr Asn Tyr Ser Thr Asn Ala Ala Leu Asn Ile Ile Ser Glu Ser
    190                 195                 200

gag gtc atg ggc gcg gac agc ctc acg ccc acc tgt gac acc gac aac 795
Glu Val Met Gly Ala Asp Ser Leu Thr Pro Thr Cys Asp Thr Asp Asn
205                 210                 215                 220

gac cag acc acc tgc gac aac ctg act tac cag ctg ccc cag ttc aag 843
Asp Gln Thr Thr Cys Asp Asn Leu Thr Tyr Gln Leu Pro Gln Phe Lys
                225                 230                 235

gtc gct gct gcc cgc cta aac tcc cag aac ccc ggc atg aac ctc acc 891
Val Ala Ala Ala Arg Leu Asn Ser Gln Asn Pro Gly Met Asn Leu Thr
            240                 245                 250

gca tct gat gtc tac aac ctg atg g gtatgtgatt acggtacaat 936
Ala Ser Asp Val Tyr Asn Leu Met
        255                 260

cattggctca aacctccagc tgacagcatc ctag tt atg gcc tcc ttt gag ctc 990
Val Met Ala Ser Phe Glu Leu
                265

aat gct cgt ccc ttc tcc aac tgg atc aac gcc ttt acc cag gac gaa 1038
Asn Ala Arg Pro Phe Ser Asn Trp Ile Asn Ala Phe Thr Gln Asp Glu
        270                 275                 280

tgg gtc agc ttc ggt tac gtt gag gat ttg aac tac tac tac tgc gct 1086
Trp Val Ser Phe Gly Tyr Val Glu Asp Leu Asn Tyr Tyr Tyr Cys Ala
    285                 290                 295

gg gtgagtttac catttgatcc attattgtct tggatcagct aacgatcgat ag t 1141
Gly
300

ccc ggt gac aag aac atg gct gct gtg ggt gcc gtc tac gcc aac gcc 1189
Pro Gly Asp Lys Asn Met Ala Ala Val Gly Ala Val Tyr Ala Asn Ala
                305                 310                 315

agt ctc acc ctc ctg aac cag gga ccc aag gaa gcc ggc tcc ttg ttc 1237
Ser Leu Thr Leu Leu Asn Gln Gly Pro Lys Glu Ala Gly Ser Leu Phe
            320                 325                 330

ttc aac tt gtacgttctc ggcagaatca gagtctcaca aaaagaaact cttcactaac 1295
Phe Asn Phe
        335

atatagtag t gcc cac gac acc aac atc acc ccc atc ctc gcc gcc cta 1344
Ala His Asp Thr Asn Ile Thr Pro Ile Leu Ala Ala Leu
                340                 345

ggc gtc ctc atc ccc aac gag gac ctt cct ctt gac cgg gtc gcc ttc 1392
Gly Val Leu Ile Pro Asn Glu Asp Leu Pro Leu Asp Arg Val Ala Phe
    350                 355                 360

ggc aac ccc tac tcg atc ggc aac atc gtg ccc atg ggt ggc cat ctg 1440
Gly Asn Pro Tyr Ser Ile Gly Asn Ile Val Pro Met Gly Gly His Leu
365                 370                 375                 380

acc atc gag cgt ctc agc tgc cag gcc acc gcc ctc tcg gac gag ggt 1488
Thr Ile Glu Arg Leu Ser Cys Gln Ala Thr Ala Leu Ser Asp Glu Gly
                385                 390                 395

acc tac gtg cgt ctg gtg ctg aac gag gct gta ctc ccc ttc aac gac 1536
Thr Tyr Val Arg Leu Val Leu Asn Glu Ala Val Leu Pro Phe Asn Asp
            400                 405                 410

tgc acc tcc gga ccg ggc tac tcc tgc cct ctg gcc aac tac acc tcc 1584
Cys Thr Ser Gly Pro Gly Tyr Ser Cys Pro Leu Ala Asn Tyr Thr Ser
        415                 420                 425

atc ctg aac aag aat ctg cca gac tac acg acc acc tgc aat gtc tct 1632
Ile Leu Asn Lys Asn Leu Pro Asp Tyr Thr Thr Thr Cys Asn Val Ser
    430                 435                 440

gcg tcc tac ccg cag tat ctg agc ttc tgg tgg aac tac aac acc acg 1680
Ala Ser Tyr Pro Gln Tyr Leu Ser Phe Trp Trp Asn Tyr Asn Thr Thr
445                 450                 455                 460

acg gag ctg aac tac cgc tct agc cct att gcc tgc cag gag ggt gat 1728
Thr Glu Leu Asn Tyr Arg Ser Ser Pro Ile Ala Cys Gln Glu Gly Asp
                465                 470                 475

gct atg gac tagatgcaga ggggtaggtc ccgggatact ttagtgatga 1777
Ala Met Asp

ttgatattca agtttggtgg tgacgatcac cttgttaata gtcttgtaca gtcatacggt 1837

gaatgtaaat aatgataata gcaatgatac atgttggaat ctcgttttgt tctttgtgtg 1897

cataggcgct ttgggggtgt atttttaggc gttagactta ttttcaattc gtgtataatg 1957

cggtcagtaa atgaatcatc aattattcaa atgcaatgct gtatacgtga aactattggg 2017

ttaagacgca gctactagct gactgcttgg ttactttctg tgtacaccgc atgc 2071

<210> 15
<211> 1449
<212> DNA
<213> Monascus purpureus

<400> 15
atgcctctct tctcttttct ctccgcgaca ccaacagtca tgcagcttat cttcacggtt 60

gcagccatcg cgagtgtggc agccgggttt cagtcggtga ttagcgaaaa gcaattttct 120

caggaatttc tcgacaacta cagcatcctg aagcactatg gcggtaatgg cccttactcc 180

agccgccggt cctacgggat ctcgcgcgaa cctcccgact cgtgctccgt cgaccaggtc 240

atcatgatca tgcgtcatgg cgagcgatat ccgtctccag acctcggagc gagcatcgaa 300

gcagctctcg ccaagatcaa gtcctcgaac gtatccacat accagggcga cctggatttc 360

ctaaattcct ggacctacta tgtccccaat cactgtgcct acaatgccga gacctccacc 420

ggaccgtatg ctggtctcct cgagggattc aagcgaggta gcgactaccg cgctcgttac 480

ggtcatttgt gggacgggga gtcgattgtc ccaatcttcg ctgctggtta ccagcgtatc 540

attgctactt ctcgaaaatt cggcgagggt ttcttcggcg ccaactactc caccaatgcg 600

gccatcaata ttatctcaga ggcaaaggag atgggtgcaa atagcctcac acccacttgc 660

gaccacgaca atgacaccag cacttgcaac tccctgacaa cggtgtggcc acagttcaaa 720

gtcgctgcag cccgtttgaa ttctcagaac cccggtttgg atctgaatgc cactgacatc 780

tactatctga tgtccatggc ttcctttgaa ttgaacgctc ggccgtactc cgactggatc 840

aatgttttta cccttgatga gtgggtgacg tttggttacg ttcaggacct gaattattat 900

tactgcgccg gcccaggaga caagaacatg gccgctgtgg gggccgtata tgtaaatgca 960

tctctgactc tcctcaacca aggcccgtca gctggcacat tatggtttaa ctttgcccat 1020

gatacaaaca tcacccccat tcttgcggcc ctcggcgtcc tcaccccgga gcgtgatctt 1080

cccaccgacc gtgttgtctt cgatagcaag tggtcctccg gggacatcgt cccccaggcc 1140

ggccacctga caatcgagcg actgaactgc acgagcactg ccgcatcacc tgccggcgtc 1200

tatgtccgct tggtccaaaa tgaggccgtc atcccggttg aagcgtgcca atcaggtcct 1260

gggtactctt gctccctggc cgacttcacc gaaattatgt ccaagcagct tcccgatttc 1320

gtgtcgactt gcagtattcg ctcgtcgtac cctcagtacc tagacttctg gtggaactac 1380

aatacgacca ccgatctgaa ctatccgaag ggcccggttc cctgtgccga gggagtggcg 1440

acaagctag 1449

<210> 16

<211> 482

<212> PRT

<213> Monascus purpureus

<400> 16

Met Pro Leu Phe Ser Phe Leu Ser Ala Thr Pro Thr Val Met Gln Leu

1               5                   10                  15

Ile Phe Thr Val Ala Ala Ile Ala Ser Val Ala Ala Gly Phe Gln Ser

            20                  25                  30

Val Ile Ser Glu Lys Gln Phe Ser Gln Glu Phe Leu Asp Asn Tyr Ser

        35                  40                  45

Ile Leu Lys His Tyr Gly Gly Asn Gly Pro Tyr Ser Ser Arg Arg Ser

    50                  55                  60

Tyr Gly Ile Ser Arg Glu Pro Pro Asp Ser Cys Ser Val Asp Gln Val

65                  70                  75                  80

Ile Met Ile Met Arg His Gly Glu Arg Tyr Pro Ser Pro Asp Leu Gly

                85                  90                  95

Ala Ser Ile Glu Ala Ala Leu Ala Lys Ile Lys Ser Ser Asn Val Ser

            100                 105                 110

Thr Tyr Gln Gly Asp Leu Asp Phe Leu Asn Ser Trp Thr Tyr Tyr Val

        115                 120                 125

Pro Asn His Cys Ala Tyr Asn Ala Glu Thr Ser Thr Gly Pro Tyr Ala

    130                 135                 140

Gly Leu Leu Glu Gly Phe Lys Arg Gly Ser Asp Tyr Arg Ala Arg Tyr
145                 150                 155                 160

Gly His Leu Trp Asp Gly Glu Ser Ile Val Pro Ile Phe Ala Ala Gly
                165                 170                 175
Tyr Gln Arg Ile Ile Ala Thr Ser Arg Lys Phe Gly Glu Gly Phe Phe
            180                 185                 190
Gly Ala Asn Tyr Ser Thr Asn Ala Ala Ile Asn Ile Ile Ser Glu Ala
        195                 200                 205
Lys Glu Met Gly Ala Asn Ser Leu Thr Pro Thr Cys Asp His Asp Asn
    210                 215                 220
Asp Thr Ser Thr Cys Asn Ser Leu Thr Thr Val Trp Pro Gln Phe Lys
225                 230                 235                 240
Val Ala Ala Ala Arg Leu Asn Ser Gln Asn Pro Gly Leu Asp Leu Asn
                245                 250                 255
Ala Thr Asp Ile Tyr Tyr Leu Met Ser Met Ala Ser Phe Glu Leu Asn
            260                 265                 270
Ala Arg Pro Tyr Ser Asp Trp Ile Asn Val Phe Thr Leu Asp Glu Trp
        275                 280                 285
Val Thr Phe Gly Tyr Val Gln Asp Leu Asn Tyr Tyr Tyr Cys Ala Gly
    290                 295                 300

Pro Gly Asp Lys Asn Met Ala Ala Val Gly Ala Val Tyr Val Asn Ala
305                 310                 315                 320

Ser Leu Thr Leu Leu Asn Gln Gly Pro Ser Ala Gly Thr Leu Trp Phe
                325                 330                 335

Asn Phe Ala His Asp Thr Asn Ile Thr Pro Ile Leu Ala Ala Leu Gly
            340                 345                 350

Val Leu Thr Pro Glu Arg Asp Leu Pro Thr Asp Arg Val Val Phe Asp
        355                 360                 365

Ser Lys Trp Ser Ser Gly Asp Ile Val Pro Gln Ala Gly His Leu Thr
    370                 375                 380

Ile Glu Arg Leu Asn Cys Thr Ser Thr Ala Ala Ser Pro Ala Gly Val
385                 390                 395                 400

Tyr Val Arg Leu Val Gln Asn Glu Ala Val Ile Pro Val Glu Ala Cys
                405                 410                 415

Gln Ser Gly Pro Gly Tyr Ser Cys Ser Leu Ala Asp Phe Thr Glu Ile
            420                 425                 430

Met Ser Lys Gln Leu Pro Asp Phe Val Ser Thr Cys Ser Ile Arg Ser
        435                 440                 445

Ser Tyr Pro Gln Tyr Leu Asp Phe Trp Trp Asn Tyr Asn Thr Thr Thr

    450                 455                 460

Asp Leu Asn Tyr Pro Lys Gly Pro Val Pro Cys Ala Glu Gly Val Ala
465                 470                 475                 480

Thr Ser

<210> 17
<211> 1181
<212> DNA
<213> Monascus purpureus

<400> 17
gatatcaatg agtacaacta tatcaggctg gttctgagac atgaatcgag caatcagttg 60

ataatgcaac tagtgtcgtg cagagcagac atgctcgagg aatgatgcaa gtatggtctg 120

ataatcgagt gcaatcagcc cagaagaata tctacatgga ctttttgaga cgtatggaac 180

atcaggtcat ggccagtgcc agataatcag acaatgacga gagggcaaag acatgaggag 240

aggatgcatt gactcttgag atagtagcat gatggggata acgttgtatg gcttaattca 300

tttctgacat ctgatagtaa tgccatgaat catggaacaa gaaaaatagc accagaaagc 360

acttcggtgt ggcagatatg tagtgcagcc ggtgagtgct ttgaggaatc ccatcagcag 420

acgcgatgga agcatctgga agcattatat aagccattgg tgcctgaggg gaccagggcc 480

cgggccaact gttgttccgt gggtagatca ggtgacctgc catagcccct tctagcgggg 540

ataatcgtac attttatagt tcattaggta tataccgtgt tacatcacca ctggggccgc 600

gacgaagatc cccttgtccc tacggagcga cggaggacgt tggcggggga ttatcgcgta 660

gagaatagcc taggcagagc gctgcaggga atacccgaga aatcaggcaa gaagagcagc 720

ggcgatggat ctatgatgcg tggcagtcac ctgattggcg ggcgtggagt ctgcagcgcg 780

caaaatatcg tgattcttcc tgctctgccc ggtgtctgaa accggaaaac cagctccagg 840

caggccattg ccgcgtccgc ggcacaactc ggggcacggc agtgtcaagg acaacgaggc 900

gaatcactgg ctgcgtttgg ccacccggtg tgtgcggtgt gcgccggtgg aagacaacca 960

gcagacgcgc gctgtttgat gacccctccg ttgcgtcagt ccattcctcc ccctccctgc 1020

cccgccccgc gctgcctggc gacgtcccat actatattac tcccgtcctc ctctcttcct 1080

ggctctctct ttttcagatt catcaatcca tcacatctca tcaacccatc tcgatccatc 1140

tcgatacatc tcgatttcgt ctaatatcta tcactctatc a 1181

<210> 18

<211> 2456

<212> DNA

<213> Monascus purpureus

<220>

<221> exon

<222> (1079)..(1129)

<220>

<221> intron

<222> (1130)..(1194)

<220>

<221> exon

<222> (1195)..(1208)

<220>

<221> intron

<222> (1209)..(1272)

<220>

<221> exon

<222> (1273)..(1324)

<220>

<221> intron

<222> (1325)..(1408)

<220>

<221> exon
<222> (1409)..(2090)

<220>
<221> intron
<222> (2091)..(2149)

<220>
<221> exon
<222> (2150)..(2346)

<220>
<221> CDS
<222> join(1079..1129, 1195..1208, 1273..1324, 1409..2090, 2150..2346)

<400> 18
gatatcaatg agtacaacta tatcaggctg gttctgagac atgaatcgag caatcagttg 60

ataatgcaac tagtgacgtg cagagcagac atgctcgagg aatgatgcaa gtatggtctg 120

ataatcgagt gcaatcagcc cagaagaata tctacatgga ctttttgaga cgtatggaac 180

atcaggtcat ggccagtgcc agataatcag acaatgacga gagggcaaag acatgaggag 240

aggatgcatt gactcttgag atagtagcat gatggggata acgttgtatg gcttaattca 300

tttctgacat ctgatagtaa tgccatgaat catggaacaa gaaaaatagc accagaaagc 360

acttcggtgt ggcagatatg tagtgcagcc ggtgagtgct ttgagaatcc catcacgaga 420

cgcgatggaa gcatctggaa gcattatata agccattggt gcctgagggg accaggcccg 480

ggccaactgt tgttccgtgg gtagatcagg tgacctgcca tagccccttc tagcggggat 540

aatcgtacat tttatagttc attaggtata taccgtgtta catcaccact ggggccgcga 600

cgaagatccc cttgtcccta cggagcgacg gaggacgttg gcgggggatt atcgcgtaga 660

gaatagccta ggcagagctg cagcggcaaa atatcgtgat tcttcctgct ttgcccggtg 720

tctgaaaccg gaaaaccagc tccaggccat tgcctccgcg gcacaactcg gggcacggca 780

gtgtcaagga caacgaggcg aatcactggc tgcgtttggc cacccggtgt gtgcggtgtg 840

cgcggtggaa gacaaccagc agagcgcgct gtttgatgac ccctccgttg cgtcagtcca 900

ttcctccccc tccctgcccc gccccggctg cctggcgacg tccatactat attactcccg 960

tcctcctctc ttcctggctc tctcttttc agattcatca atccatcaca tctcatcaac 1020

ccatctcgat ccatctcgat acatctcgat ttcgtctaat atctatcact ctatcaaa 1078

atg gtt gtc ccc aag gtt gga atc aac ggc ttc ggt cgt atc ggc cgt 1126
Met Val Val Pro Lys Val Gly Ile Asn Gly Phe Gly Arg Ile Gly Arg
1               5                   10                  15

att gtaagttccc tctccctatc ctcttgctcg tctcgtgcca ataaatccct 1179
Ile

aacaatgttt ccaag gtc ttc cgt aac gc gtaagtagct ttctcgggcg 1228
Val Phe Arg Asn Ala
        20

ttttccctgg atccccagag agtatcctaa cccttcgcct acag t atc gag cac 1282
Ile Glu His
        25

gag ggt gtt gac atc gtt gcc gtc aac gac ccc ttc att gag 1324
Glu Gly Val Asp Ile Val Ala Val Asn Asp Pro Phe Ile Glu
                30                  35

gtccactatg ctgtacgttc cgttccattc ctgcccagca tcgtcacctc gctcgagaag 1384

ctaaccagac acgatatcga ttag gcc tac atg ctc aag tat gac agc acc 1435
Ala Tyr Met Leu Lys Tyr Asp Ser Thr
40                  45

cac ggc cgc ttc aac gga gcc gtc gag ttc gac ggc aac acg ctc atc 1483
His Gly Arg Phe Asn Gly Ala Val Glu Phe Asp Gly Asn Thr Leu Ile
    50                  55                  60

gtc aac ggc aag aag atc aag ttc tac gca gag agg gac ccc gct cag 1531
Val Asn Gly Lys Lys Ile Lys Phe Tyr Ala Glu Arg Asp Pro Ala Gln
65                  70                  75                  80

atc ccc tgg agc gag act ggc cag tac gtc gtt gag tcc act ggt gtc 1579
Ile Pro Trp Ser Glu Thr Gly Gln Tyr Val Val Glu Ser Thr Gly Val
                85                  90                  95

ttc acc aag cag gag aag gcc tcc ctt cac ctg aga ggg tgt gcc aag 1627
Phe Thr Lys Gln Glu Lys Ala Ser Leu His Leu Arg Gly Cys Ala Lys
            100                 105                 110

aag gtc atc atc tcc gct ccc tct tcc gac tcc ccc atg ttt gtc atg 1675
Lys Val Ile Ile Ser Ala Pro Ser Ser Asp Ser Pro Met Phe Val Met
        115                 120                 125

ggt gtc aac aac gac caa tac acc aag gac atc acc gtc ctt tcc aac 1723
Gly Val Asn Asn Asp Gln Tyr Thr Lys Asp Ile Thr Val Leu Ser Asn
    130                 135                 140

gcc tct tgc acc acc aac tgc ttg gct ccc ctt gcc aag gtc atc aat 1771
Ala Ser Cys Thr Thr Asn Cys Leu Ala Pro Leu Ala Lys Val Ile Asn
145                 150                 155                 160

gac aag ttc ggc atc gtc gag ggt ctg atg acc aca gtc cac tcc tac 1819
Asp Lys Phe Gly Ile Val Glu Gly Leu Met Thr Thr Val His Ser Tyr
                165                 170                 175

act gct acc cag aag gtc gtc gat ggc ccc tcc aac aag gac tgg aga 1867
Thr Ala Thr Gln Lys Val Val Asp Gly Pro Ser Asn Lys Asp Trp Arg
            180                 185                 190

ggt ggc cgt acc gct gcc cag aac atc atc ccc agc tcc acc ggt gtg 1915
Gly Gly Arg Thr Ala Ala Gln Asn Ile Ile Pro Ser Ser Thr Gly Val
        195                 200                 205

cct aag gca gtc ggc aag gtc att cct tcc ttg aac ggc aag ctc act 1963
Pro Lys Ala Val Gly Lys Val Ile Pro Ser Leu Asn Gly Lys Leu Thr
    210                 215                 220

ggc atg tct atg cgt gtg cct act tcc aac gcc tcc gtt gtc gac ctt 2011
Gly Met Ser Met Arg Val Pro Thr Ser Asn Ala Ser Val Val Asp Leu
225                 230                 235                 240

act gcc cgt ctc gag aag gcc gcc acc tac gac gag atc aag cag gcc 2059
Thr Ala Arg Leu Glu Lys Ala Ala Thr Tyr Asp Glu Ile Lys Gln Ala
                245                 250                 255

gtc aag aag gcc tct gag cgc cct ctg aag g gtgagtttaa aatgaccctc 2110
Val Lys Lys Ala Ser Glu Arg Pro Leu Lys
            260                 265

gatatgttgc acacggactc gattactgac taggactag gc atc ctc ggc tac act 2166
Gly Ile Leu Gly Tyr Thr
            270

gag gat gac gtt gtc tcc tcc gat ctc aac gga gac ccc cac tcc tcc 2214
Glu Asp Asp Val Val Ser Ser Asp Leu Asn Gly Asp Pro His Ser Ser
        275                 280                 285

atc ttc gat gcc aag gct ggt atc gcc ctc aac tcg aac ttc gtc aag 2262
Ile Phe Asp Ala Lys Ala Gly Ile Ala Leu Asn Ser Asn Phe Val Lys
    290                 295                 300

ctg ttt tcc tgg tac gac aac gag tgg ggt tac tcc cgc cgt gtt atc 2310
Leu Phe Ser Trp Tyr Asp Asn Glu Trp Gly Tyr Ser Arg Arg Val Ile
305                 310                 315                 320

gac ctc att gcc tat gcc cag gtc gat gcc cag taattattag acgggctcct 2363
Asp Leu Ile Ala Tyr Ala Gln Val Asp Ala Gln
                325                 330

gagacgaaaa gtctcctatg aaatcagaat gagcaatccc tcaacgtact atccccactt 2423

cagctgaagt ccctgcgcga ccggcagagg cct 2456

<210> 19
<211> 21
<212> DNA
<213> Artificial Sequence

<220>
<223> sense primer for amplification of Aspergillus oryzae
amdS gene

<400> 19

aatcagtctg tagaatgctg g                                                21

<210> 20
<211> 21
<212> DNA
<213> Artificial Sequence

<220>
<223> antisense primer for amplification of Aspergillus oryzae
amdS gene

<400> 20
tctagatagt gcttatttgt c                                                21

<210> 21
<211> 29
<212> DNA
<213> Artificial Sequence

<220>
<223> sense primer for amplification of Aspergillus nidulans
alcB gene

<400> 21
gggaattcgg cgtytgccay acagatctt                                        29

<210> 22
<211> 29
<212> DNA
<213> Artificial Sequence

<220>
<223> antisense primer for amplification of Aspergillus nidulans
alcB gene

<400> 22
ccgaattcga gkatgacrgc rtgcgcacc                                    29

<210> 23
<211> 26
<212> DNA
<213> Artificial Sequence

<220>
<223> sense primer for amplification of Aspergillus niger aph
gene

<400> 23
gggaattcat gcctcgcacc tctctc                                       26

<210> 24

<211> 26

<212> DNA

<213> Artificial Sequence

<220>

<223> antisense primer for amplification of Aspergillus niger aph

gene

<400> 24

ccgaattcct agtccatagc atcacc                                          26

<210> 25

<211> 28

<212> DNA

<213> Artificial Sequence

<220>

<223> sense primer for amplification of promoter region of

Monascus purpureus GAPDH gene

<400> 25

ggtctagaga tatcaatgag tacaacta                                        28

<210> 26

<211> 28

<212> DNA

<213> Artificial Sequence

<220>
<223> antisense primer for amplification of promoter region of
Monascus purpureus GAPDH gene

<400> 26
acaaccgaat tctgatagag tgatagat                                        28

<210> 27
<211> 28
<212> DNA
<213> Artificial Sequence

<220>
<223> sense primer for amplification of terminator region of
Monascus purpureus GAPDH gene

<400> 27
caggaattct tagacgggct cctgagac                                        28

<210> 28
<211> 30
<212> DNA
<213> Artificial Sequence

<220>

<223> antisense primer for amplification of terminator region of

Monascus purpureus GAPDH gene

<400> 28

ccaagcttag gcctctgccg gtcgcgcgca                                        30

<210> 29

<211> 27

<212> DNA

<213> Artificial Sequence

<220>

<223> sense primer for amplification of promoter region of

Monascus purpureus alcB gene

<400> 29

ggctcaggat ccttcctcgt gagaagg                                           27

<210> 30

<211> 27

<212> DNA

<213> Artificial Sequence

<220>

<223> antisense primer for amplification of promoter region of

Monascus purpureus alcB gene

<400> 30
aggttcagcc atgaattcaa gagtgaa                                    27

<210> 31
<211> 30
<212> DNA
<213> Artificial Sequence

<220>
<223> sense primer for amplification of terminator region of
Monascus purpureus alcB gene

<400> 31
ctgaattcgg ttggatggtt gacgagatgg                                 30

<210> 32
<211> 30
<212> DNA
<213> Artificial Sequence

<220>
<223> antisense primer for amplification of terminator region of
Monascus purpureus alcB gene

<400> 32
tcctcgagtt ctgcgcagaa acacccagct                                        30

<210> 33
<211> 27
<212> DNA
<213> Artificial Sequence

<220>
<223> sense primer for amplification of promoter region of
Monascus purpureus aph gene

<400> 33
ttagtcctga gctcatcatt ggaatgc                                           27

<210> 34
<211> 27
<212> DNA
<213> Artificial Sequence

<220>
<223> antisense primer for amplification of promoter region of
Monascus purpureus aph gene

<400> 34
gagaagagag gatcctagag atgaagg                                           27

<210> 35

<211> 27

<212> DNA

<213> Artificial Sequence

<220>

<223> sense primer for amplification of terminator region of

Monascus purpureus aph gene

<400> 35

acaagctagt aggatccatg ctgtcct 27

<210> 36

<211> 27

<212> DNA

<213> Artificial Sequence

<220>

<223> antisense primer for amplification of terminator region of

Monascus purpureus aph gene

<400> 36

gttcttggca ggtaccgaat agcgatg 27

<210> 37

<211> 27

<212> DNA

<213> Artificial Sequence

<220>

<223> sense primer for amplification of promoter region and

signal sequence of Monascus purpureus aph gene

<400> 37

ttagtcctga gctcatcatt ggaatgc 27

ATGATGGTCGCGTGGTGGTCTCTATTTCTGTACGGCCTTCAGGTCGCGGCACCTGCTTTGGCT

<210> 38

<211> 48

<212> DNA

<213> Artificial Sequence

<220>

<223> antisense primer for amplification of promoter region and

signal sequence of Monascus purpureus aph gene

<400> 38

atcgcaagtg gattgatttc tcgacccggc tgccacactc gcgatggc 48

<210> 39

<211> 49

<212> DNA
<213> Artificial Sequence

<220>
<223> sense primer for amplification of DNA encoding mature
polypeptide of Aspergillus niger phytase

<400> 39
gccatcgcga gtgtggcagc cgggtcgaga aatcaatcca cttgcgata 49

<210> 40
<211> 27
<212> DNA
<213> Artificial Sequence

<220>
<223> antisense primer for amplification of DNA encoding mature
polypeptide of Aspergillus niger phytase

<400> 40
gttcttggca ggtaccgaat agcgatg 27

<210> 41
<211> 28
<212> DNA
<213> Artificial Sequence

<220>

<223> sense primer for amplification of DNA encoding signal

peptide of Aspergillus oryzae Taka-amylase A

<400> 41

ggtctagaga tatcaatgag tacaacta 28

<210> 42

<211> 48

<212> DNA

<213> Artificial Sequence

<220>

<223> antisense primer for amplification of DNA encoding signal

peptide of Aspergillus oryzae Taka-amylase A

<400> 42

atcgcaagtg gattgatttc tcgaagccaa agcaggtgcc gcgacctg 48

<210> 43

<211> 48

<212> DNA

<213> Artificial Sequence

<220>

<223> sense primer for amplification of DNA encoding mature
polypeptide of Aspergillus niger phytase

<400> 43
caggtcgcgg cacctgcttt ggcttcgaga aatcaatcca cttgcgat 48

<210> 44
<211> 30
<212> DNA
<213> Artificial Sequence

<220>
<223> antisense primer for amplification of DNA encoding mature
polypeptide of Aspergillus niger phytase

<400> 44
ccaagcttag gcctctgccg gtcgcgcgca 30